(19) Europäisches Patentamt
European Patent Office
Office européen des brevets

(11) **EP 3 702 473 A1**

(12) **EUROPEAN PATENT APPLICATION**
published in accordance with Art. 153(4) EPC

(43) Date of publication:
**02.09.2020 Bulletin 2020/36**

(51) Int Cl.:
*C12Q 1/6869* (2018.01)   *C12M 1/00* (2006.01)
*C12N 15/09* (2006.01)   *C12Q 1/68* (2018.01)
*G16B 99/00* (2019.01)

(21) Application number: **18869832.8**

(22) Date of filing: **26.10.2018**

(86) International application number:
**PCT/JP2018/039963**

(87) International publication number:
**WO 2019/083024 (02.05.2019 Gazette 2019/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.10.2017   JP 2017208651**
**25.10.2018   JP 2018201317**

(71) Applicant: **Sysmex Corporation**
**Kobe-shi, Hyogo 651-0073 (JP)**

(72) Inventors:
• **INOUE Fumio**
**Kobe-shi**
**Hyogo 651-0073 (JP)**
• **SUZUKI Seigo**
**Kobe-shi**
**Hyogo 651-0073 (JP)**
• **SUZUKI Kenichiro**
**Kobe-shi**
**Hyogo 651-0073 (JP)**

(74) Representative: **Hoffmann Eitle**
**Patent- und Rechtsanwälte PartmbB**
**Arabellastraße 30**
**81925 München (DE)**

(54) **GENE ANALYSIS METHOD, GENE ANALYZER, MANAGEMENT SERVER, GENE ANALYSIS SYSTEM, PROGRAM AND RECORDING MEDIUM**

(57)     For analyzing gene sequences by use of various gene panels, convenience for a user is improved. A gene analysis apparatus (1) configured to analyze gene sequence information includes: a controller (11) configured to obtain read sequence information read by a sequencer (2) and panel information related to a panel including a plurality of genes to be analyzed; and an output unit (13) configured to output an analysis result of the read sequence information based on the panel information obtained by the controller (11).

FIG. 4

**Description**

TECHNICAL FIELD

The present invention relates to a gene analysis method performed by a computer in order to analyze mutations of genes, a gene analysis apparatus, a management server, a gene analysis system, a program, and a storage medium.

BACKGROUND ART

Associated with the development of genetic test technologies in recent years, there is increasing expectancy for individualized medical care in which gene sequences of a subject are analyzed to appropriately select therapies and drugs suitable for characteristics of the subject. As gene sequence analysis, for example, a panel test is known in which abnormalities in specific genes related to specific diseases or abnormalities in exon regions to be translated into proteins are analyzed at a high-throughput by use of a next-generation sequencer.

Patent Literature 1 describes a system in which: whether a gene or the like has an abnormality when compared with a reference sequence is determined; a drug therapy to be used in accordance with the gene or the like having an abnormality is identified; and a therapeutic strategy is determined in accordance with the subject.

CITATION LIST

[PATENT LITERATURE]

[PTL 1] Japanese Translation of PCT International Application Publication No. 2015-200678

SUMMARY OF THE INVENTION

PROBLEMS TO BE SOLVED BY THE INVENTION

In a genetic test, each gene to be analyzed requires a different analysis. For example, in a panel test using a next-generation sequencer, fragmented genes are simultaneously read in a parallel manner, and read sequence information which is the base sequence of each read fragment is mapped on a reference sequence, whereby base sequence analysis is performed. Here, when genes to be analyzed are different for each gene panel, a different analysis program is sometimes required for each gene panel that is used to perform measurement. Therefore, when a panel test is performed, a different analysis program needs to be selectively used for each gene panel, which is inconvenient.

In addition, in a genetic test, when the entire exon region is analyzed, many mutations are detected in genes of a subject. Here, mutations include those of which the clinical significance has not been confirmed or for which therapeutically effective drugs have not been established. Thus, such mutations provide information other than information that can be utilized by doctors for actual therapies. Doctors trying to apply the result of a genetic test to an actual therapy for a subject desire to selectively know mutations that can be utilized in the actual therapy among the many detected mutations.

In such circumstances, a user who is going to perform a panel test needs to prepare, for each panel, a dedicated analysis program to be used in gene analysis performed by a sequencer, in accordance with genes to be tested and a desire, before performing the gene analysis.

An object of an aspect of the present invention is to realize, for analyzing analysis target genes by use of a gene panel, a gene analysis method, a gene analysis apparatus, a management server, a gene analysis system, and the like that are highly convenient for a user and that can be applied to various gene panels.

SOLUTION TO THE PROBLEMS

In order to solve the above problem, a gene analysis method according to an aspect of the present invention is for analyzing gene sequence information, and includes: obtaining read sequence information read by a sequencer (2) and gene panel information related to a gene panel including a plurality of genes to be analyzed; and outputting an analysis result of the read sequence information on the basis of the obtained gene panel information.

According to this aspect, an analysis result of read sequence information is outputted on the basis of the obtained gene panel information. Due to this aspect, for example, when analysis target genes in various combinations are analyzed by use of various gene panels, a user who performs a panel test can obtain an output according to the gene panel. Thus, convenience for the user is improved.

"Gene" includes a sequence on a genome from a start codon to a stop codon, mRNA generated from a sequence on the genome, a promoter region on the genome, and the like. The gene to be analyzed includes mRNA transcribed from a gene on the genome. mRNA includes pre-mRNA.

"Read sequence" means a polynucleotide sequence obtained through sequencing. "Read sequence information" means information of a read sequence outputted from the sequencer 2.

"Gene panel" means a reagent kit for analyzing a plurality of analysis targets by performing a series of analysis processes once (one run). In many cases, the gene panel includes a set of reagents such as a primer and a probe. Here, a "plurality of analysis targets" may be a plurality of gene sequences or may be a plurality of exons of a certain gene. For example, a reagent kit for analyzing the sequence of gene A and the sequence of gene B, a reagent kit for analyzing the sequence of exon 1 of gene A and the sequence of exon 2 of gene A, and the like are included. A more specific example of the gene panel includes a reagent kit for analyzing a plurality of gene sequences related to a specific disease. When this gene panel is used, it is possible to analyze amplification of one or a plurality of genes, substitution, deletion, and insertion of a sequence, methylation of a promoter region, a fused gene, and the like that are important for treatment. The gene panel includes a plurality of genes as analysis targets. As the gene panel, a large panel with which 100 or more genes can be analyzed is useable, for example.

"Gene panel information" may be any information that can be used for specifying a gene panel, and may be, for example, the gene panel name, the name of a gene to be analyzed in the panel test, or the like.

The gene analysis method may include selecting, on the basis of the obtained gene panel information, a gene for which the analysis result is to be outputted.

According to this aspect, an analysis result with respect to an analysis target gene of the gene panel can be outputted.

The gene analysis method may include selecting, on the basis of the obtained gene panel information, an analysis algorithm for analyzing a gene for which the analysis result is to be outputted.

According to this aspect, when target genes of the gene panel is analyzed, it is not necessary to set, for each gene, an analysis program to be used in the analysis.

The gene analysis method may include displaying, on a display unit (16), an input screen for allowing information associated with a plurality of genes to be inputted as the gene panel information.

The gene analysis method may include displaying, on a display unit (16), an input screen for allowing at least one piece of information to be selected from a plurality of pieces of the gene panel information.

The gene analysis method may include displaying, on a display unit (16), an input screen for allowing a reagent kit name to be inputted as the gene panel information.

The gene analysis method may include displaying, on a display unit (16), an input screen for allowing a plurality of genes to be analyzed, to be inputted as the gene panel information.

The gene analysis method may include displaying, on a display unit (16), an input screen for allowing a disease to be analyzed, to be inputted as the gene panel information.

The gene analysis method may include: selecting, on the basis of the obtained gene panel information, reference sequence information with which the read sequence information should be compared; and outputting the analysis result based on comparison between the read sequence information and the selected reference sequence information.

"Reference sequence" is a sequence with respect to which a read sequence is mapped in order to determine which region on the gene the read sequence corresponds to, which mutation on the gene the read sequence corresponds to, and the like. "Mapping" means a process of aligning each read sequence to a corresponding reference sequence. Specifically, the mapping is performed to find, in the genome sequence that is referred to, a region that has a sequence identical or similar to the read sequence having been read, and to cause the read sequence to belong to the region.

The gene analysis method may include: on the basis of the obtained gene panel information, selecting, from a plurality of pieces of reference sequence information each including a mutation sequence, reference sequence information with which the read sequence information should be compared; and outputting the analysis result based on the selected reference sequence information.

"Mutation" means at least one of polymorphism, substitution, Indel, and the like of a gene. "Indel (Insertion and/or Deletion)" means a mutation that includes insertion, deletion, or both of insertion and deletion. "Polymorphism" of a gene includes SNV (single nucleotide variant, single nucleotide polymorphism), VNTR (variable nucleotide of tandem repeat, repeat sequence polymorphism), STRP (short tandem repeat polymorphism, microsatellite polymorphism), and the like.

The gene analysis method may include outputting the analysis result of the read sequence information, using a gene-panel-related information database (121) which stores, for each gene panel, information related to an analysis target gene of the gene panel.

The gene analysis method may include reading a selected reference sequence from a reference sequence database (122), and mapping the read sequence information with respect to the read reference sequence, to perform alignment.

The gene analysis method may include reading a selected reference sequence from a reference sequence database, determining a position of the read sequence information on the basis of a degree of matching between the reference sequence and the read sequence information, and identifying a mutation included in the read sequence information.

The gene analysis method may include outputting an analysis result that includes information related to a mutation associated with the obtained gene panel information, among mutations identified through analysis of the read sequence information.

The gene analysis method may include, on the basis of the obtained gene panel information, outputting drug information related to a mutation identified through analysis of the read sequence information, as the analysis result of the read sequence information.

The gene analysis method may include, on the basis of a mutation identified through analysis of the read sequence information, searching a drug database (124) which stores a mutation of an analysis target gene and a drug related to the gene panel in association with each other.

The gene analysis method may include generating a list of a drug related to the mutation identified through the analysis of the read sequence information and extracted through the search of the drug database (124).

The gene analysis method may include generating a list of a drug related to the mutation identified through the analysis of the read sequence information and extracted through the search of the drug database (124).

The gene analysis method may include outputting, as the analysis result of the read sequence information, drug information including an approval state of a drug.

The gene analysis method may include, on the basis of a mutation identified through analysis of the read sequence information, searching a reference database (125) which stores a mutation of an analysis target gene and reference information related to the mutation in association with each other.

The gene analysis method may include creating a report on the basis of the analysis result of the read sequence information. The report may include information related to a mutation that corresponds to the obtained information related to the gene panel among mutations identified through analysis of the read sequence information.

The gene analysis method may include selecting, on the basis of the gene panel information, a mutation that corresponds to the obtained gene panel information from all identified mutations, and outputting information related to the selected mutation, as the analysis result of the read sequence information.

The report may include information related to the gene panel.

The report may include at least one of a list of a drug and reference information.

The gene analysis method may include transmitting, to a management server (3), information related to an analysis state of the gene sequence information.

The gene analysis method may include transmitting, for each piece of the gene panel information to a management server (3), information related to an analysis state of the gene sequence information.

The gene analysis method may include transmitting, for each piece of the gene panel information to a management server (3), the number of times of sequence analysis of the genes.

The gene analysis method may include transmitting, for each piece of the gene panel information to a management server (3), the number of the genes having being analyzed.

The gene analysis method may include transmitting, for each piece of the gene panel information to a management server (3), information related to an amount of data having been processed in sequence analysis of the genes.

The gene analysis method may include outputting, as the analysis result of the read sequence information, a comparison result obtained by comparing the read sequence information with sequence information of an analysis target gene of the gene panel associated with the obtained gene panel information.

The gene analysis method may include displaying an error when the obtained gene panel information does not match gene panel information that has been registered.

For example, when the obtained gene panel information does not match gene panel information that has been registered in the gene-panel-related information database (121) or the like, if analysis is performed using the gene panel, an inappropriate analysis result may be obtained. According to this aspect, it is possible to prevent outputting an inappropriate result caused by use of an unregistered gene panel, and to prevent performing unnecessary analysis.

The gene analysis method may include displaying an error when the obtained gene panel information does not match gene panel information that has been designated by a medical institution (210).

The gene analysis method may include: when an input that asks permission of use of a gene panel inputted by a user is made after the error has been displayed, permitting analysis that uses the gene panel.

The gene analysis method may include: when the obtained gene panel information does not match gene panel information that has been registered, prohibiting analysis that uses the gene panel.

The gene analysis method may include: when the obtained gene panel information does not match gene panel information that has been designated by a medical institution (210), prohibiting analysis that uses the gene panel.

The obtaining of the gene panel information may have a plurality of modes, and one of the plurality of modes may be selectable.

The gene analysis method may include displaying an error when pieces of the read sequence information include not less than a predetermined number of pieces of the read sequence information that include sequences of genes that are not analysis target genes of the gene panel indicated by the obtained gene panel information.

The read sequence information may include an index sequence associated with the gene panel information.

The index sequence may be different for each piece of gene panel information.

The gene analysis method may include displaying an error when gene panel information associated with the index sequence included in the read sequence information is different from the obtained gene panel information.

The gene analysis method may include: analyzing, with respect to a first sample, first read sequence information read by use of a first gene panel for analyzing a first analysis target gene group; analyzing, with respect to a second sample, second read sequence information read by use of a second gene panel for analyzing a second analysis target gene group; receiving selection of information that specifies the gene panel, to obtain gene panel information; and outputting, on the basis of the selected gene panel information, an analysis result obtained by analyzing the first read sequence information and an analysis result obtained by analyzing the second read sequence information.

Here, a "sample" can be also referred to as a specimen, and is used synonymously with a preparation in this technical field. A "sample" is intended to mean any preparation obtained from a biological material (for example, individual, body fluid, cell strain, cultured tissue, or tissue section) as a supply source.

The gene analysis method may further include evaluating a quality of a gene panel test, and the outputting of the analysis result may include outputting an evaluation result of the quality on the basis of the obtained gene panel information.

According to this aspect, when analysis target genes in various combinations are analyzed by use of various gene panels, appropriate quality control according to the gene panel can be performed.

"Quality evaluation index" is an index for evaluating the quality of a gene panel test. Examples of the quality evaluation index include indexes such as: the reading quality included in read sequence information outputted by the sequencer (2); the proportion of bases read by the sequencer (2), to bases included in a plurality of genes as analysis targets; the depth of reading of read sequence information; the variation of the depth of reading of read sequence information; and whether or not all of mutations of each standard gene included in a quality control sample have been detected.

The evaluating of the quality of the gene panel test may include selecting, on the basis of the obtained gene panel information, a quality control index to be used when evaluating the quality.

The evaluating of the quality of the gene panel test may include selecting, on the basis of the obtained gene panel information, an evaluation criterion for a quality control index to be used when evaluating the quality.

The evaluating of the quality of the gene panel test may include selecting, on the obtained gene panel information, the number of quality control indexes to be used when evaluating the quality.

In order to solve the above problem, a gene analysis apparatus (1) according to an aspect of the present invention is a gene analysis apparatus (1) configured to analyze gene sequence information, and includes: a controller (11) configured to obtain read sequence information read by a sequencer (2) and gene panel information related to a gene panel including a plurality of genes to be analyzed; and an output unit (13). The controller (11) outputs, to the output unit (13), an analysis result of the read sequence information on the basis of the obtained gene panel information.

According to this aspect, the gene analysis apparatus (1) outputs an analysis result of the read sequence information on the basis of the obtained gene panel information. Due to this aspect, when genes are analyzed by use of various gene panels, a user who performs a panel test can obtain an output according to the gene panel that is used. Thus, convenience for the user is improved.

The controller (11) may select, on the basis of the obtained gene panel information, reference sequence information with which the read sequence information should be compared, and may output, to the output unit (13), the analysis result based on comparison between the read sequence information and the selected reference sequence information.

The controller (11) may output, to the output unit (13), an analysis result that includes information related to a mutation associated with the obtained gene panel information, among mutations identified through analysis of the read sequence information.

On the basis of the obtained gene panel information, the controller (11) may output, to the output unit (13), drug information related to a mutation identified through analysis of the read sequence information, as the analysis result of the read sequence information.

On the basis of the obtained gene panel information, the controller (11) may output an evaluation result of a quality of a gene panel test, to the output unit (13).

In order to solve the above problem, a management server (3) according to an aspect of the present invention is configured to receive, from a gene analysis apparatus (1), information that includes information for specifying a user who performs analysis of a sequence of a gene, gene panel information related to a gene panel having been used, and information related to an analysis state of sequence information.

The "information related to an analysis state of sequence information" may be the number of times of sequence analysis an analysis using a predetermined gene panel has been performed in the gene analysis apparatus 1, may be the number of genes that have been analyzed, or may be the accumulated total of the number or the like of mutations that have been identified. Alternatively, the "information related to an analysis state of sequence information" may be information related to the amount of data that has been processed in the analysis.

The management server (3) may receive, from the gene analysis apparatus (1), information related to an analysis state of sequence information of the gene.

For each piece of the gene panel information, the management server (3) may receive, from the gene analysis apparatus (1), information related to an analysis state of sequence information of the gene.

For each piece of the gene panel information, the management server (3) may receive, from the gene analysis apparatus (1), the number of times of the analysis of the sequence of the gene.

For each piece of the gene panel information, the management server (3) may receive, from the gene analysis apparatus (1), the number of the genes having been analyzed.

For each piece of the gene panel information, the management server (3) may receive, from the gene analysis apparatus (1), information related to an amount of data having been processed in the analysis of the sequence of the gene.

On the basis of information related to an analysis state of sequence information of the gene, the management server (3) may calculate a consideration for a case where the user has performed analysis of a sequence using the gene analysis apparatus (1).

The management server (3) may receive, from the gene analysis apparatus (1), an update request for the gene panel information.

In order to solve the above problem, a gene analysis system (100) according to an aspect of the present invention includes a gene analysis apparatus (1) and a management server (3). The gene analysis apparatus (1) includes: a controller (11) configured to obtain read sequence information read by a sequencer (2) and gene panel information related to a gene panel including a plurality of genes to be analyzed; and an output unit (13) configured to output an analysis result of the read sequence information based on the gene panel information obtained by the controller (11). The management server (3) is configured to receive, from the gene analysis apparatus (1) via a network (4), information that includes information for specifying a user who performs analysis of a sequence of a gene, gene panel information related to a gene panel having been used, and information related to an analysis state of the sequence of the gene.

According to this aspect, the gene analysis apparatus (1) outputs an analysis result of the read sequence information on the basis of the obtained gene panel information. Meanwhile, the management server (3) receives, from the gene analysis apparatus (1), information that includes information for specifying a user who performs analysis of a sequence of a gene, gene panel information related to a gene panel having been used, and information related to an analysis state of the sequence of the gene.

According to this aspect, for example, when genes in various combinations are analyzed by use of various gene panels, a user who performs a panel test can obtain an output according to the gene panel that is used. Thus, convenience is improved. Further, the management server (3) can confirm/manage the record of analysis performed by the user using the gene analysis apparatus (1). Therefore, for example, a consideration such as usage fee for the gene analysis system (100) can be appropriately determined, and can be charged on the user.

A consideration for a case where the user has performed analysis of a sequence using the gene analysis apparatus may be calculated on the basis of information related to an analysis state of sequence information of the gene.

The gene analysis apparatus (1) according to each aspect of the present invention may be realized by a computer. In this case, a program that realizes the gene analysis apparatus (1) in the form of a computer by causing the computer to operate as units (software elements) of the gene analysis apparatus (1), and a computer readable storage medium having stored therein the program, are also included in the scope of the present invention.

In order to solve the above problem, a program according to an aspect of the present invention is configured to analyze gene sequence information. The program causes a computer to execute: obtaining read sequence information read by a sequencer and gene panel information related to a gene panel including a plurality of genes to be analyzed; and outputting an analysis result of the read sequence information on the basis of the obtained gene panel information.

According to this aspect, effects similar to those obtained by the gene analysis method according to one aspect of the present invention are exhibited.

A storage medium according to an aspect of the present invention is a computer readable storage medium having stored therein the program according to one aspect of the present invention.

A gene analysis method according to an aspect of the present invention is for analyzing gene sequence information. The gene analysis method includes: obtaining read sequence information read by a sequencer (2) and gene panel information related to a gene panel including a plurality of genes to be analyzed; and outputting an analysis result of the read sequence information on the basis of the obtained gene panel information. When the obtained gene panel information does not match gene panel information that has been registered, an error is displayed.

A gene analysis method according to an aspect of the present invention is for analyzing gene sequence information. The gene analysis method includes: obtaining read sequence information read by a sequencer (2) and gene panel information related to a gene panel including a plurality of genes to be analyzed; and outputting an analysis result of the read sequence information on the basis of the obtained gene panel information. When the obtained gene panel information does not match gene panel information that has been designated by a medical institution (210), an error is displayed.

ADVANTAGEOUS EFFECTS OF THE INVENTION

According to the present invention, when analysis target genes in various combinations are measured by use of various gene panels, convenience for the user can be improved.

BRIEF DESCRIPTION OF THE DRAWINGS

[FIG. 1] FIG. 1 shows an application example of a gene analysis system according to an embodiment of the present invention.
[FIG. 2] FIG. 2 is a sequence diagram showing an example of major processes performed in the gene analysis system.
[FIG. 3] FIG. 3 shows an example of a structure of data stored in a management server.
[FIG. 4] FIG. 4 shows an example of a configuration of a gene analysis apparatus.
[FIG. 5] FIG. 5 is a flow chart showing an example of the flow of a process for receiving an input of gene panel information.
[FIG. 6] FIG. 6 shows an example of a GUI to be used for inputting gene panel information.
[FIG. 7] FIG. 7 shows an example of a data structure of a gene-panel-related information database.
[FIG. 8] FIG. 8 shows an example of a GUI to be used when a user updates gene panel information.
[FIG. 9] FIG. 9 is a flow chart describing an example of a procedure performed by a sequencer from pretreatment to sequencing for analyzing the base sequence of sample DNA.
[FIG. 10] FIG. 10 illustrates examples of a step (a) of fragmentation of a sample, and a step (b) of provision of an index sequence and an adapter sequence.
[FIG. 11] FIG. 11 illustrates an example of a hybridization step.
[FIG. 12] FIG. 12 illustrates an example of a step of collecting DNA fragments to be analyzed.
[FIG. 13] FIG. 13 illustrates an example of a step of applying DNA fragments to a flow cell.
[FIG. 14] FIG. 14 illustrates an example of a step of amplifying DNA fragments to be analyzed.
[FIG. 15] FIG. 15 illustrates an example of a sequencing step.
[FIG. 16] FIG. 16 is a flow chart describing an example of the flow of analysis performed by the gene analysis apparatus.
[FIG. 17] FIG. 17 shows an example of a file format for read sequence information.
[FIG. 18] FIG. 18(a) illustrates alignment performed by a data adjustment unit, and FIG. 18(b) shows an example of a format for a result of alignment performed by the data adjustment unit.
[FIG. 19] FIG. 19 shows an example of a structure of a reference sequence database.
[FIG. 20] FIG. 20 shows an example of known mutations incorporated in reference sequences (that do not indicate wild-type sequences) included in the reference sequence database.
[FIG. 21] FIG. 21 is a flow chart describing in detail an example of a step of alignment.
[FIG. 22] FIG. 22(a) shows an example of score calculation, and FIG. 22(b) shows another example of the score calculation.
[FIG. 23] FIG. 23 shows an example of a format for a result file generated by a mutation identification unit.
[FIG. 24] FIG. 24 shows an example of a structure of a mutation database.
[FIG. 25] FIG. 25 shows a specific example of a structure of mutation information in the mutation database.
[FIG. 26] FIG. 26(a) is a table showing correspondence relationship between analysis target genes and position information, and FIG. 26(b) shows a state where mutations that do not correspond to gene panel information are excluded in a result file.
[FIG. 27] FIG. 27 shows another example of a configuration of a gene analysis apparatus.
[FIG. 28] FIG. 28 is a flow chart showing an example of a process in which a drug search unit generates a list of drugs related to mutations.
[FIG. 29] FIG. 29 shows an example of a data structure of a drug database.
[FIG. 30] FIG. 30 shows an example of a data structure of a drug database.
[FIG. 31] FIG. 31 is a flow chart showing an example of a process in which the drug search unit generates a list that includes information related to drugs that are related to mutations.

[FIG. 32] FIG. 32 is a flow chart showing an example of a process in which, on the basis of information obtained by searching the drug database, the drug search unit determines the presence or absence of a drug having a possibility of off-label use and generates a list that includes the determination result.

[FIG. 33] FIG. 33 shows an example of a data structure of a drug database.

[FIG. 34] FIG. 34 is a flow chart showing an example of a process in which the drug search unit generates a list that includes information related to clinical trials of drugs.

[FIG. 35] FIG. 35 shows another example of a configuration of a gene analysis apparatus.

[FIG. 36] FIG. 36 shows an example of a data structure of a reference database.

[FIG. 37] FIG. 37 shows an example of a report that is created.

[FIG. 38] FIG. 38 shows another example of a configuration of a gene analysis apparatus.

[FIG. 39] FIG. 39 shows an example of a data structure of a gene-panel-related information database.

[FIG. 40] FIG. 40 shows another example of a GUI to be used for inputting gene panel information.

[FIG. 41] FIG. 41 shows another example of a GUI to be used for inputting gene panel information.

[FIG. 42] FIG. 42 is a flow chart showing another example of the flow of a process for receiving an input.

[FIG. 43] FIG. 43 shows another example a gene analysis apparatus.

[FIG. 44] FIG. 44 is a flow chart showing an example of the flow of a process for analyzing a gene sequence.

[FIG. 45] FIG. 45 shows an example of a quality evaluation index.

[FIG. 46] FIG. 46 shows an example of a report that is created.

DESCRIPTION OF EMBODIMENTS

[Embodiment 1]

Hereinafter, an embodiment of the present invention is described in detail.

(Outline of gene analysis method)

In a gene analysis method according to an embodiment of the present invention, gene panel information related to a gene panel is obtained, and on the basis of the obtained gene panel information, an analysis result of a read sequence having been read by a sequencer is outputted. Accordingly, when analysis target genes in various combinations are analyzed by use of various gene panels, appropriate analysis results according to the gene panels can be outputted without the need of selectively using an analysis program for each gene panel, and thus, convenience for the user is improved.

(Application example of gene analysis system 100)

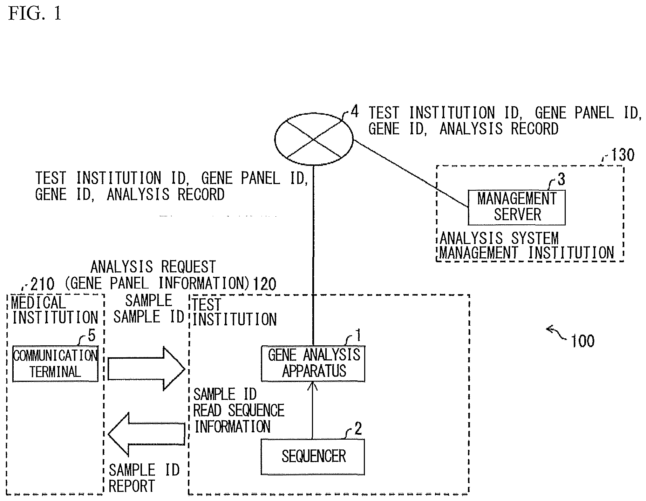

First, the outline of a gene analysis system 100 according to an embodiment of the present invention is described with reference to FIG. 1. FIG. 1 shows an application example of the gene analysis system 100 according to an embodiment of the present invention. The gene analysis system 100 is a system for analyzing gene sequence information, and includes a gene analysis apparatus 1 and a management server 3, at least.

The gene analysis system 100 shown in FIG. 1 is applied in: an analysis system management institution 130 which manages analyses in general performed in a test institution 120; and the test institution 120 which analyzes a provided sample in response to an analysis request from a medical institution 210 and which provides an analysis result to the medical institution 210. The gene analysis apparatus 1 is installed in the test institution 120, and the management server 3 is installed in the analysis system management institution 130. The gene analysis apparatus 1 and the management server 3 form the gene analysis system 100.

The test institution 120 is an institution that tests/analyzes a sample provided from the medical institution 210, that creates a report based on an analysis result, and that provides the report to the medical institution 210. The test institution 120 is provided with, but not limited to, a sequencer 2, the gene analysis apparatus 1, and the like.

The analysis system management institution 130 is an institution that manages analyses in general performed in each test institution 120 that uses the gene analysis system 100. For example, the analysis system management institution 130 is a business entity that allows a gene analysis apparatus 1 to be installed in a test institution 120 and that provides gene analysis services that correspond to various gene panels. The analysis system management institution 130 performs management of the gene analysis system 100 such that: information stored in databases of the gene analysis apparatus 1 is updated; and gene analysis is performed on the basis of the latest information. The analysis system management institution 130 may obtain the state of gene analysis in the gene analysis apparatus 1, and may obtain consideration from the test institution 120 in accordance with the performance of gene analysis.

The medical institution 210 is an institution in which doctors, nurses, pharmacists and the like perform medical activities such as providing diagnoses, therapies, and preparation of medicines to patients, and examples of the medical institution 210 include hospitals, clinics, pharmacies, and the like.

(Process in application example of gene analysis system 100)

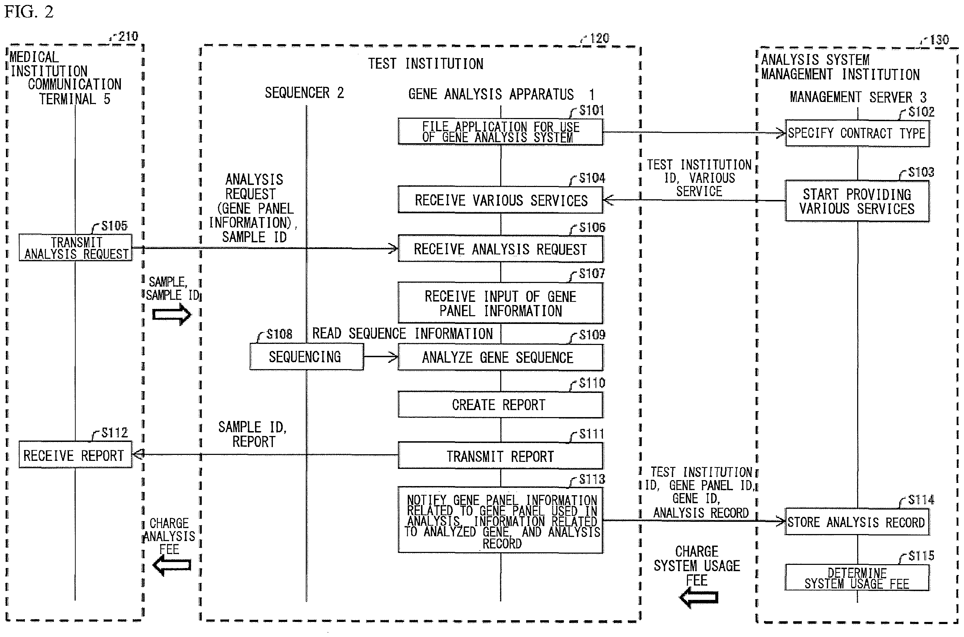

Next, the flow of a process performed in an application example of the gene analysis system 100 shown in FIG. 1 is more specifically described with reference to FIG. 2. FIG. 2 is a sequence diagram showing an example of major processes performed in the gene analysis system 100. The processes shown in FIG. 2 are only part of processes performed in each institution.

<Filing application for use of gene analysis system and start of use>

First, a test institution 120 that is going to use the gene analysis system 100 introduces the gene analysis apparatus 1. Then, the test institution 120 files an application for use of the gene analysis system 100 to the analysis system management institution 130 (step S101).
The test institution 120 and the analysis system management institution 130 can conclude in advance a desired contract with regard to use of the gene analysis system 100, out of a plurality of contract types. For example, service contents provided from the analysis system management institution 130 to the test institution 120, a method of determination of a system usage fee charged on the test institution 120 by the analysis system management institution 130, a method of payment of a system usage fee, and the like may be selected from a plurality of different contract types. The management server 3 of the analysis system management institution 130 specifies the content of the contract concluded with the test institution 120, in response to the application filed from the test institution 120 (step S102).
Next, the management server 3, which is managed by the analysis system management institution 130, provides a test institution ID to the gene analysis apparatus 1 of the test institution 120 that has concluded the contract, and starts providing various types of services (step S103).
The gene analysis apparatus 1 receives various types of services from the management server 3. Such various types of services include provision of programs and information for controlling analysis results of gene sequences that can be outputted from the gene analysis apparatus 1, and reports and the like based on the analysis results. Accordingly, the gene analysis apparatus 1 can output an analysis result, a report, and the like that match gene panel information having been inputted.

<Analysis request to test institution 120>

In the medical institution 210, a doctor or the like collects a sample such as blood and a tissue of a lesion site of a subject, as necessary. When analysis of the collected sample is requested to the test institution 120, an analysis request is transmitted from a communication terminal 5 provided in the medical institution 210, for example (step S105). When requesting analysis of samples to a test institution 120, the medical institution 210 transmits the analysis request and provides the test institution 120 with sample IDs provided to the respective samples. The sample ID provided to each sample associates the sample with information and the like of the subject from which the sample has been collected.
A "subject" herein denotes a human subject, or a subject that is not human such as a mammal, an invertebrate, a vertebrate, a fungus, a yeast, a bacterium, a virus, or a plant. The embodiments herein relate to a human subject, but the concept of the present invention can be applied to a genome derived from an organism such as any animal other than human or any plant, and is useful in fields such as medical care, veterinary medicine, and zoological science.
In the following, an example case in which the medical institution 210 requests a panel test analysis to the test institution 120 is described. The panel test is not limited to a laboratory test, but also includes tests for research use.
When a gene panel test is requested form the medical institution 210, a desired gene panel may be designated. Thus, gene panel information can be included in the analysis request transmitted from the medical institution 210 in step S105 shown in FIG. 2. Here, the gene panel information may be any information that can be used for specifying a gene panel, and may be, for example, the gene panel name, the name of a gene to be analyzed in the panel test, or the like.

<Analysis in test institution 120>

The gene analysis apparatus 1 receives the analysis request from the medical institution 210 (S106). Further, the gene analysis apparatus 1 receives a sample from the medical institution 210, which is the transmit source of the analysis request.
There are a plurality of gene panels that can be used in analysis that the test institution 120 is requested to perform by the medical institution 210, and a gene group to be analyzed is fixed for each gene panel. The test institution 120 can selectively use a plurality of gene panels so as to suit the purpose of the analysis. That is, with respect to a first sample provided from the medical institution 210, a first gene panel can be used to analyze a first analysis target gene group, and with respect to a second sample, a second gene panel can be used to analyze a second analysis target gene group.

The gene analysis apparatus 1 receives, from a user, an input of gene panel information related to a gene panel that is to be used for analyzing the sample (step S107).

In the test institution 120, pretreatment of the received sample is performed, and sequencing using the sequencer 2 is performed (step S108).

Here, the pretreatment can include processes from fragmentation of genes such as DNA contained in the sample to collection of the fragmented genes. The sequencing includes a process of reading the sequence of one or a plurality of DNA fragments to be analyzed that have been collected in the pretreatment. Sequence information read in the sequencing performed by the sequencer 2 is outputted as read sequence information to the gene analysis apparatus 1.

Subsequently, the gene analysis apparatus 1 obtains the read sequence information from the sequencer 2, and performs gene sequence analysis (step S109).

The gene analysis apparatus 1 creates a report on the basis of the analysis result obtained in step S109 (step S110), and transmits the created report to the communication terminal 5 (step 5111).

<Charge of analysis fee on medical institution 210>

As described above, in the test institution 120, a sample is analyzed in response to the analysis request from the medical institution 210, and a report based on the analysis result is created. The medical institution 210 receives the report from the test institution 120 (step S112). The test institution 120 may charge the medical institution 210 for an analysis fee as a consideration for performing the analysis of the sample and providing the report based on the analysis result to the medical institution 210, which is the source of the analysis request.

<Charge of system usage fee>

The analysis system management institution 130 provides various types of information and services in accordance with the content of the contract with each test institution 120 as described above, and may charge the test institution 120 for a consideration such as a system usage fee.

The gene analysis apparatus 1 of the test institution 120 using the gene analysis system 100 notifies the management server 3 of the gene panel information related to the gene panel used in the analysis, information related to the analyzed genes, an analysis record, and the like (step S113). Specifically, the gene analysis apparatus 1 sends a test institution ID, a gene panel ID, gene IDs, an analysis record, and the like, to the management server 3.

The management server 3 stores the obtained test institution ID, gene panel ID, gene IDs, analysis record, and the like in association with one another (step S114).

The test institution ID is information that specifies a user who performs gene sequence analysis. The test institution ID may be a user ID, which is identification information provided to each user that uses the gene analysis apparatus 1.

The gene panel ID is identification information provided in order to specify a gene panel that is used in analysis of target genes. The gene panel ID provided to a gene panel is associated with the gene panel name, the name of the company that provides the gene panel, and the like.

The gene ID is identification information provided for each gene in order to specify an analysis target gene.

The analysis record is information related to the analysis state of gene sequence information. For example, the analysis record may be the number of times of sequence analysis an analysis using a predetermined gene panel has been performed in the gene analysis apparatus 1, may be the number of genes that have been analyzed, or may be the accumulated total of the number or the like of mutations that have been identified. Alternatively, the analysis record may be information related to the amount of data that has been processed in the analysis.

The management server 3 aggregates, for each test institution 120, the analysis records in a predetermined period (for example, any period such as day, week, month, or year), and determines a system usage fee according to the aggregation result and the contract type (step S115). The analysis system management institution 130 may charge the determined system usage fee on the test institution 120, and request payment of the system usage fee to the analysis system management institution 130.

(Configuration of gene analysis system 100)

The gene analysis system 100 is a system for analyzing gene sequence information, and includes the gene analysis apparatus 1 and the management server 3 at least. The gene analysis apparatus 1 is connected to the management server 3 via a network 4 such as an intranet and the internet.

(Sequencer 2)

The sequencer 2 is a base sequence analysis apparatus used for reading base sequences of genes contained in a sample.

Preferably, the sequencer 2 according to the present embodiment is a next-generation sequencer that performs sequencing using a next-generation sequencing technology, or a third-generation sequencer. The next-generation sequencer denotes one of base sequence analysis apparatuses which have been developed in recent years. The next-generation sequencer has a significantly improved analytical capability by performing, in a flow cell, parallel processing of a large amount of a single DNA molecule or a DNA template having been clonally amplified.

The sequencing technology usable in the present embodiment can be a sequencing technology that obtains a plurality of reads by reading the same region multiple times (deep sequencing).

Examples of the sequencing technology usable in the present embodiment include sequencing technologies that can obtain a large number of reads per run, on the basis of a sequencing principle other than that of the Sanger's method, such as ionic semiconductor sequencing, pyrosequencing, sequencing-by-synthesis using a reversible dye terminator, sequencing-by-ligation, and sequencing by use of probe ligation of oligonucleotide.

A sequence primer to be used in sequencing is not limited in particular, and is set as appropriate on the basis of a sequence that is suitable for amplifying the target region. Also, with respect to reagents to be used in sequencing, suitable reagents may be selected in accordance with the sequencing technology and the sequencer 2 to be used. The procedure from pretreatment to sequencing will be described later with reference to a specific example.

(Management server 3)

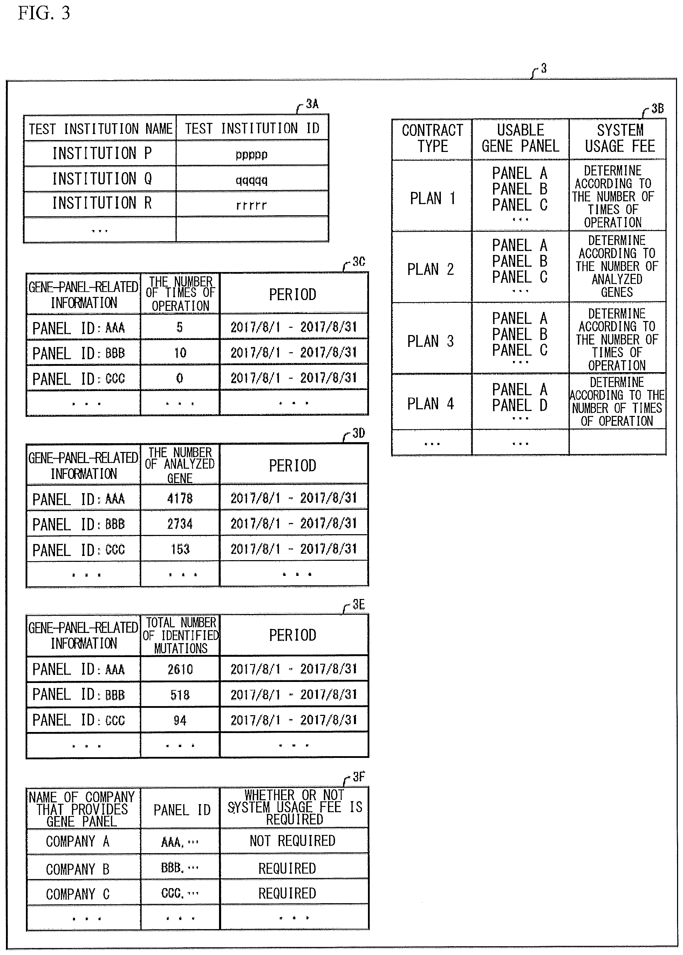

Next, data stored in the management server 3 is described with reference to FIG. 3. FIG. 3 shows an example of a structure of data stored in the management server 3. On the basis of each data shown in FIG. 3, the analysis system management institution 130 determines a system usage fee to be charged on each test institution. The management server 3 receives, from the gene analysis apparatus 1 via the network 4, information that includes: information for specifying a user who performs gene sequence analysis (for example, test institution ID); gene panel information related to the gene panel that has been used; and information related to the state of gene sequence analysis (for example, analysis record).

In data 3A shown in FIG. 3, the name of a test institution that uses the gene analysis system 100 and the test institution ID provided to the test institution are associated with each other. In data 3B shown in FIG. 3, the type of contract concluded between the analysis system management institution 130 and a test institution 120, services to be provided to the test institution that has concluded the contract (for example, usable gene panel), and a system usage fee are associated with one another.

For example, in a case where a test institution "Institution P" has concluded a contract of "Plan 1" with the analysis system management institution 130, the analysis system management institution 130 charges the test institution P for a usage fee that corresponds to the number of times of operation. "The number of times of operation" is the number of times a panel test has been performed by the gene analysis apparatus 1, for example.

Data 3C to 3E shown in FIG. 3 are analysis records related to the number of times of operation that was performed, genes that were analyzed, and the total number of mutations that were identified in a period from August 1, 2017 to August 31, 2017, by the test institution using the gene analysis system 100. These analysis records are transmitted from the gene analysis apparatus 1 to the management server 3, and are stored in the management server 3. On the basis of the data of these analysis records, the analysis system management institution 130 determines a system usage fee to be charged on each test institution. The record aggregation period is not limited to that mentioned above. The recodes may be aggregated in any period such as day, week, month, or year.

When the analysis system management institution 130 determines a system usage fee, the system usage fee may be varied depending on whether the gene panel that was used in the test is from a company that provides (for example, produces or sells) the gene panel. In this case, it is sufficient that data 3F shown in FIG. 3 is stored in the management server 3. In data 3F shown in FIG. 3, the name of a company that provides gene panels, such as "Company A" or "Company B", a gene panel ID, and an agreement as to the system usage fee (for example, whether a system usage fee is required or not) are associated with one another.

An example case in which "Institution P" concluded a contract of "Plan 1" with the analysis system management institution 130 and the analysis records are those shown in FIG. 3 is described. Institution P performed tests using a gene panel (gene panel ID "AAA") provided by Company A, 5 times, and tests using a gene panel (gene panel ID "BBB") provided by Company B, 10 times. According to the data shown in FIG. 3, the system usage fee is not required for the 5 tests using the gene panel provided by Company A. Therefore, for Institution P, the analysis system management institution 130 determines a system usage fee, excluding the number of times of test using the gene panel provided by Company A.

(Configuration of gene analysis apparatus 1)

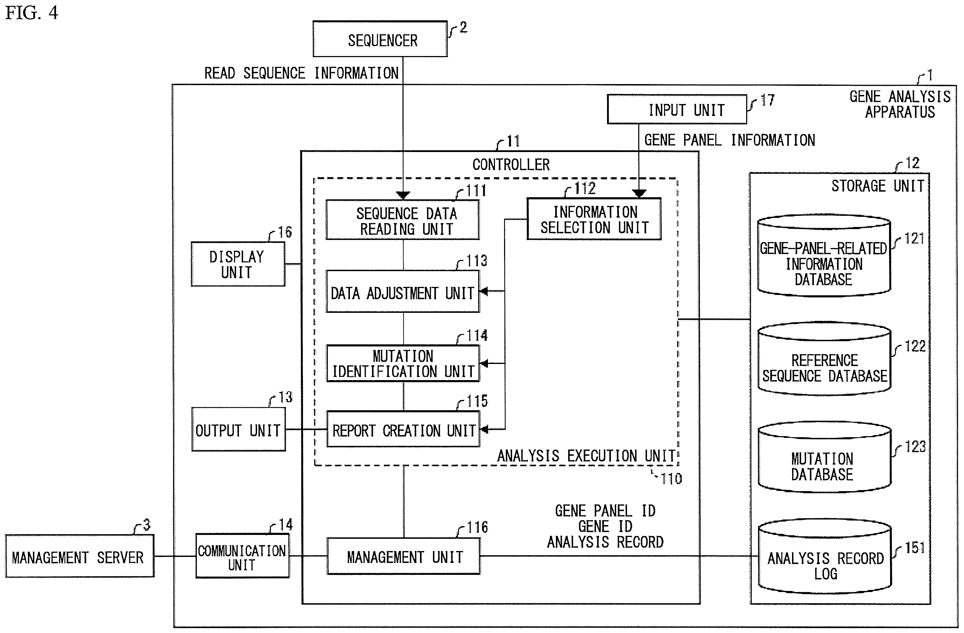

FIG. 4 shows an example of a configuration of the gene analysis apparatus 1. The gene analysis apparatus 1 includes: a controller 11 which obtains read sequence information read by the sequencer 2, and gene panel information related to a gene panel including a plurality of genes to be analyzed; and an output unit 13 which outputs an analysis result of the read sequence information based on the gene panel information obtained by the controller 11. The gene analysis apparatus 1 can be configured by use of a computer. For example, the controller 11 is a processor such as a CPU, and a storage unit 12 is a hard disk drive.

The storage unit 12 also has stored therein a program for sequence analysis, a program for generating a single reference sequence, and the like. The output unit 13 includes a display, a printer, a speaker, and the like. An input unit 17 includes a keyboard, a mouse, a touch sensor, and the like. Alternatively, an apparatus may be used that has both of the functions of an input unit and an output unit, such as a touch panel in which a touch sensor and a display are integrated. A communication unit 14 is an interface through which the controller 11 performs communication with an external apparatus.

The gene analysis apparatus 1 includes: the controller 11 which comprehensively controls the units of the gene analysis apparatus 1; the first storage unit 12 which has stored therein various types of data used by an analysis execution unit 110; the output unit 13; the communication unit 14; a display unit 16; and the input unit 17. The controller 11 includes the analysis execution unit 110 and a management unit 116. Further, the analysis execution unit 110 includes a sequence data reading unit 111, an information selection unit 112, a data adjustment unit 113, a mutation identification unit 114, and a report creation unit 115. A gene-panel-related information database 121, a reference sequence database 122, a mutation database 123, and an analysis record log 151 are stored in the first storage unit 12.

Even when a different gene panel is used for each analysis, the gene analysis apparatus 1 creates a report including an analysis result that corresponds to the gene panel that has been used. Thus, the user who uses the gene analysis system 100 can analyze the result of a panel test by use of a common analysis program irrespective of the type of the gene panel, and create a report. This eliminates inconvenience such as: when a panel test is performed, an analysis program to be used needs to be selected for each gene panel; and special setting needs to be made for the analysis program that is used for the gene panel. Thus, convenience for the user is improved.

When the user of the gene analysis apparatus 1 has inputted gene panel information through the input unit 17, the information selection unit 112 refers to the gene-panel-related information database 121, and controls the algorithms in the analysis program such that the analysis program performs analysis of the analysis target genes in accordance with the inputted gene panel information. That is, the gene analysis apparatus 1 selects an analysis algorithm in accordance with the inputted gene panel information.

Here, the gene panel information may be any information that specifies the gene panel used in the measurement performed by the sequencer 2. For example, the gene panel information is the gene panel name, the names of analysis target genes of the gene panel, the gene panel ID, and the like.

On the basis of the gene panel information inputted through the input unit 17, the information selection unit 112 selects an analysis algorithm for performing analysis so as to correspond to the analysis target genes of the gene panel indicated by the gene panel information. Specific examples of selecting an analysis algorithm in the present embodiment include: (1) selecting a reference sequence; and (2) selecting a region of the mutation database 123 to be referred to for identifying a mutation.

The information selection unit 112 outputs an instruction based on the gene panel information, to at least one of the data adjustment unit 113, the mutation identification unit 114, and the report creation unit 115. With this configuration, the gene analysis apparatus 1 can output an analysis result of the read sequence information on the basis of the inputted gene panel information.

That is, the information selection unit 112 is a function block that performs control so as to obtain gene panel information related to a gene panel including a plurality of genes to be analyzed, and cause the output unit 13 to output an analysis result of the read sequence information on the basis of the obtained gene panel information.

In a case where genes contained in various samples are analyzed by the user who performs a panel test, various gene panels are used in accordance with the analysis target gene group for each sample.

That is, the gene analysis apparatus 1 can obtain: first read sequence information read by use of a first gene panel for analyzing a first analysis target gene group from a first sample; and second read sequence information read by use of a second gene panel for analyzing a second analysis target gene group from a second sample.

Even when analysis target genes in various combinations have been analyzed by use of various gene panels, the gene analysis apparatus 1 can appropriately output analysis results obtained through analysis of read sequence information because the gene analysis apparatus 1 is provided with the information selection unit 112.

That is, if the user merely selects gene panel information, without setting an analysis program to be used in analysis of read sequence information and performing analysis for each analysis target gene, an analysis result of each piece of read sequence information can be appropriately outputted.

For example, when the information selection unit 112 outputs an instruction based on gene panel information to the data adjustment unit 113, the data adjustment unit 113 performs an alignment process and the like reflecting the gene panel information.

In accordance with the gene panel information, the information selection unit 112 issues an instruction so that the reference sequence (reference sequences in which wild type genome sequences and mutation sequences are incorporated) to be used by the data adjustment unit 113 when mapping the read sequence information is limited only to the reference sequence for genes that correspond to the gene panel information.

In this case, since the gene panel information is already reflected in the result of the process performed by the data adjustment unit 113, the information selection unit 112 need not output an instruction based on the gene panel information to the mutation identification unit 114 which subsequently performs a process following the process performed by the data adjustment unit 113.

For example, in a case where the information selection unit 112 outputs an instruction based on the gene panel information to the mutation identification unit 114, the mutation identification unit 114 performs a process reflecting the gene panel information.

For example, in accordance with the gene panel information, the information selection unit 112 issues an instruction so that the region of the mutation database 123 referred to by the mutation identification unit 114 is limited to only mutations related to the genes that correspond to the gene panel information. As a result, the gene panel information is reflected in the result of the process performed by the mutation identification unit 114.

(Input of gene panel information)

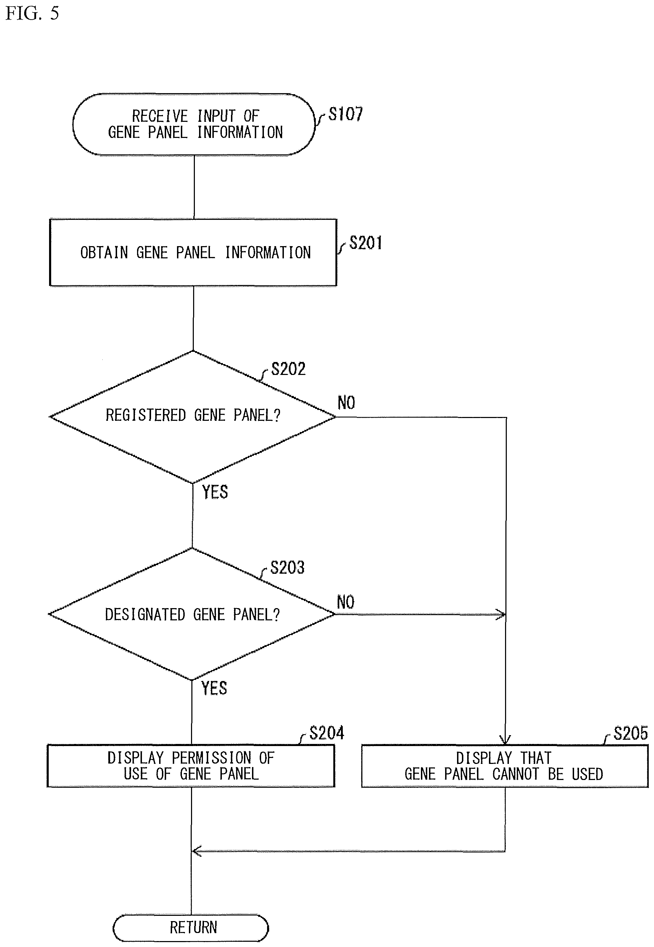

Here, a process for receiving an input of gene panel information shown in step S107 in FIG. 2 is described with reference to FIG. 5. FIG. 5 is a flow chart showing an example of the flow of a process for receiving an input of gene panel information.

Here, an example configuration is described in which the controller 11 causes the display unit 16 to display a GUI for inputting gene panel information, thereby allowing the user to input gene panel information.

In this case, the input unit 17 can be a device (for example, a mouse, a keyboard, etc.) that allows the user to perform an input operation on the presented GUI. In a case where a touch panel is overlaid on the display unit 16, the display unit 16 has a function of the input unit 17. That is, in a case where a touch panel is used as the display unit 16, the display unit 16 also serves as the input unit 17.

First, the controller 11 of the gene analysis apparatus 1 causes the display unit 16 to display a GUI for allowing the user to select gene panel information. On the basis of the input operation on the GUI by the user, the controller 11 obtains the gene panel information (step S201).

On the basis of the information selected by the user in the information displayed as the GUI, the information selection unit 112 searches the gene-panel-related information database 121 and reads gene panel information that corresponds to the selected information.

In addition, the gene analysis apparatus 1 reads gene panel information that is included in the analysis request received from the medical institution 210.

When a gene panel corresponding to the selected information is already registered in the gene-panel-related information database 121 (YES in step S202) and the gene panel matches the gene panel included in the analysis request received from the medical institution 210 (YES in step S203), the information selection unit 112 receives the input. Then, the information selection unit 112 causes the display unit 16 to display a message to the effect that the inputted gene panel can be used (step S204).

Meanwhile, when the gene panel corresponding to the selected information is not registered in the gene-panel-related information database 121, i.e., when an unregistered gene panel has been selected (NO in step S202), the information selection unit 112 causes the display unit 16 to display a message to the effect that the inputted gene panel cannot be used (step S205), and prohibits the analysis from being performed by the gene analysis apparatus 1.

In this case, instead of the message to the effect that the gene panel cannot be used, a message that indicates an error may be displayed. The message may be, for example, "The selected gene panel is not registered." and may further include a message that urges re-input, such as "Please input gene panel information again".

When the gene panel corresponding to the selected information does not match the gene panel included in the analysis request received from the medical institution 210 (NO in step S203), the information selection unit 112 causes the display unit 16 to display a message to the effect that the inputted gene panel cannot be used (step S205), and prohibits analysis from being performed by the gene analysis apparatus 1.

Also in this case, instead of the message to the effect that the gene panel cannot be used, a message that indicates an error may be displayed. The message may be, for example, "The selected gene panel is different from that in the order." and may further include a message that urges re-input, such as "Please input gene panel information again".

This process can prevent performing sequencing by use of an inappropriate gene panel and performing unnecessary analysis operation, and can eliminate wasteful use of gene panels and wasteful operation of the gene analysis system 100.

(Example of GUI used for inputting gene panel information)

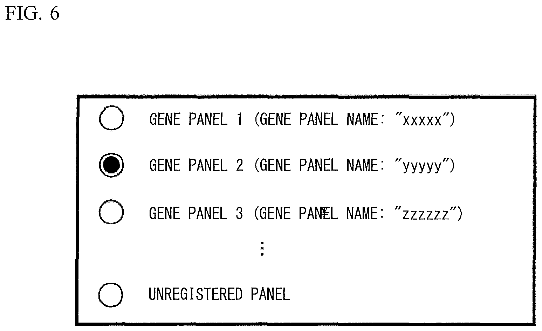

Next, some examples of an input screen for allowing the user to input gene panel information is described with reference to FIG. 6. FIG. 6 shows an example of a GUI to be used for inputting gene panel information.

As shown in FIG. 6, as gene panel information, a list of gene panel names such as "xxxxx" and "yyyyy" may be displayed on the GUI, and the user may be allowed to select a desired gene panel out of the gene panels on the list.

The list of gene panel names on the GUI is displayed on the basis of gene panel names of gene panels that are provided with gene panel IDs and that are already registered in the gene-panel-related information database 121.

In the GUI shown in FIG. 6, "gene panel 2 (gene panel name: "yyyyy")" has been selected by the user. Using the gene panel ID associated with the selected gene panel name "yyyyy" as a key, the information selection unit 112 searches the gene-panel-related information database 121, and obtains gene panel information that corresponds to the inputted gene panel name.

(Gene-panel-related information database 121)

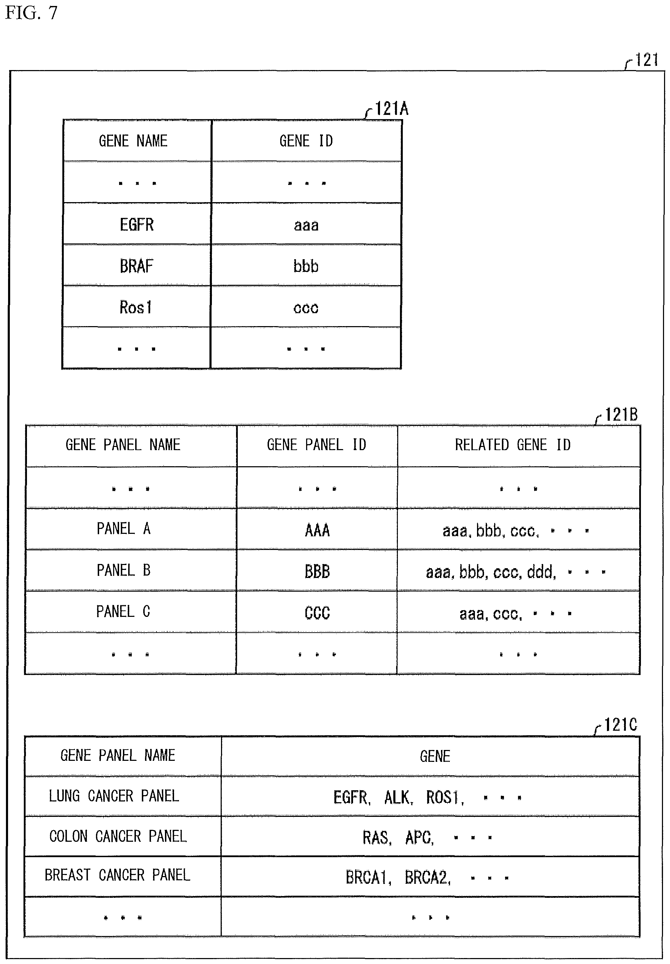

Next, data stored in the gene-panel-related information database 121 referred to by the information selection unit 112 when gene panel information has been inputted through the input unit 17 is described with reference to FIG. 7. FIG. 7 shows an example of a data structure of the gene-panel-related information database 121.

In the gene-panel-related information database 121, as shown in data 121A in FIG. 7, the name of each gene that can be an analysis target and a gene ID provided to the gene are stored for each gene panel.

In addition, in the gene-panel-related information database 121, as shown in data 121B in FIG. 7, the name of each selectable gene panel, the gene panel ID provided to the gene panel, and the gene IDs of analysis target genes of the gene panel (related gene ID) are stored in association with one another. Each gene panel may also be associated with information as to whether or not use of the gene panel is already approved by a public institution (for example, Japanese Ministry of Health, Labour and Welfare).

As shown in FIG. 6, when the user has selected a desired gene panel out of the gene panels presented on the GUI, the information selection unit 112 refers to the gene-panel-related information database 121 and extracts the gene panel ID and related gene IDs that are associated with the selected gene panel name.

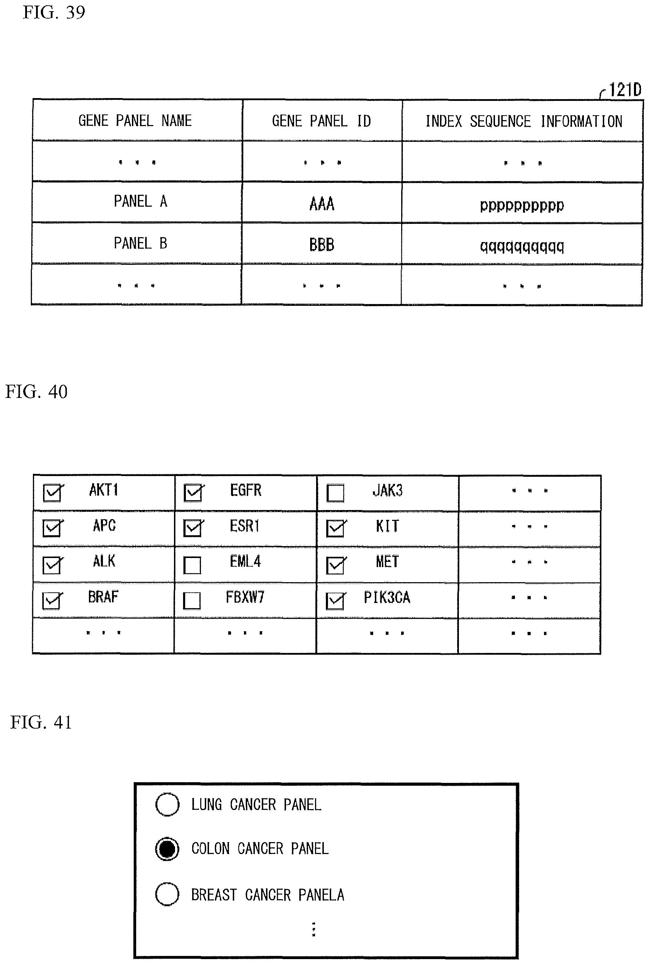

When analysis target genes have been selected out of the gene names presented on the GUI as shown in FIG. 40, the information selection unit 112 refers to the gene-panel-related information database 121 and extracts gene IDs associated with the selected gene names, and the gene panel ID of the gene panel that includes these gene IDs as the related gene IDs.

In the gene-panel-related information database 121, as shown in data 121C in FIG. 7, the name of a gene panel related to a disease, and the names of analysis target genes (or gene IDs) of the gene panel may be stored in association with each other.

When a gene panel related to a disease of interest has been selected from a list of the gene panel names for respective diseases presented on the GUI (i.e., a case as shown in FIG. 41), the information selection unit 112 refers to the gene-panel-related information database 121, and extracts, from the gene names associated with the gene panel name related to the selected disease, the gene IDs thereof, and the gene panel ID of the gene panel that includes these gene IDs as the related gene IDs.

<Update of gene-panel-related information database 121>

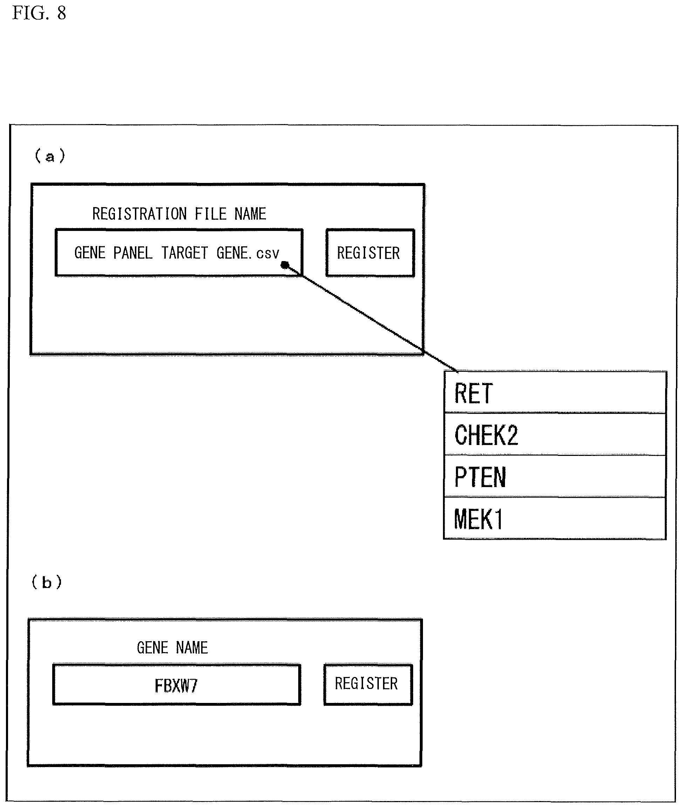

Here, update of information stored in the gene-panel-related information database 121 is described with reference to FIG. 8. FIG. 8 shows an example of a GUI to be used when the user updates the gene-panel-related information database 121.

Update of information stored in the gene-panel-related information database 121 can be performed by use of an update patch provided from the analysis system management institution 130 to the test institution 120. For example, in such a case where an analysis target gene of a gene panel has been changed, or where a new gene panel has been added, information stored in the gene-panel-related information database 121 is updated to the latest information.

Provision of the update patch from the analysis system management institution 130 may be targeted to test institutions 120 that have paid the system usage fee. For example, the analysis system management institution 130 may notify each test institution 120 that the condition for providing an update patch is existence of an update patch that can be provided and payment of the system usage fee. Such a notification can appropriately urge each test institution 120 to pay the system usage fee.

As shown in FIG. 8(a), when a plurality of genes are updated as a batch, a field for inputting a "registration file name" may be displayed, and the name of a file describing gene names, such as "gene panel target gene.csv", may be inputted in the field. In the example shown in FIG. 8(a), the "gene panel target gene.csv" includes a plurality of gene names of RET, CHEK2, PTEN, and MEK1.

When a "register" button is pressed after the file name has been inputted, a request for updating the information related to the genes that correspond to the gene names included in the file is associated with the test institution ID, and the request is transmitted to the management server 3 via the communication unit 14. The generation of the update request and the association of the update request with the test institution ID may be performed by the controller 11 shown in FIG. 4, for example.

The analysis system management institution 130 permits the gene analysis apparatus 1 to download information that includes: the gene IDs provided to the gene names included in the update request received by the management server 3; and the gene panel ID provided to the gene panel that has the genes as the analysis target genes.

Alternatively, as shown in FIG. 8(b), when the user performs update by inputting a gene name individually, a field for inputting a "gene name" may be displayed, and a gene name such as "FBXW7" may be inputted in the field.

When a "register" button is pressed after the gene name has been inputted, a request for updating the information related to the gene that corresponds to the gene name is associated with the test institution ID, and the request is transmitted to the management server 3 via the communication unit 14. The analysis system management institution 130 permits the gene analysis apparatus 1 to download information that includes: the gene ID provided to the gene name included in the update request received by the management server 3; and the gene panel ID provided to the gene panel that has the gene as the analysis target gene.

The field for inputting a "registration file name" in FIG. 8(a), and the field for inputting a "gene name" in FIG. 8(b) may include a configuration for displaying input candidates as a suggestion.

For example, information of input candidates to be displayed is provided in advance from the management server 3 to the gene analysis apparatus 1, and is stored in the first storage unit 12. Then, when a click operation onto the GUI in the input field has been detected, all of the gene names that can be updated may be presented as input candidates to allow selection by the user therefrom, or a gene name that can be updated and that matches the character string inputted by the user may be presented as an input candidate. Alternatively, for example, at the time point when the user has inputted "E" in the field for inputting a "gene name" in FIG. 8(b), a list of gene names that can be updated such as "EGFR" and "ESR" may be displayed to allow selection by the user from the list. By presenting the input candidates in this manner, it is possible to prevent the user from making an erroneous input.

The gene-panel-related information database 121 may store each gene name, the gene ID of the gene, and the name of protein coded by the gene in association with one another.

In this case, even when the inputted character string is not a gene name but a protein coded by the gene, the information selection unit 112 can obtain a gene name and a gene ID that are associated with the inputted protein name, by referring to the gene-panel-related information database 121.

When a protein name has been inputted in the field for inputting a "gene name" and the register button has been pressed, a GUI may be displayed that shows a gene name associated with the protein name to allow the user to confirm that the gene name is the correct one.

(Management unit 116)

The management unit 116 stores, in the analysis record log 151, as appropriate, an analysis record that includes the number of times of operation performed by the analysis execution unit 110, the number of analyzed genes, the total number of identified mutations, and the like, in association with the gene panel IDs and the gene IDs. At a desired frequency (for example, each day, each week, or each month), the management unit 116 reads data including the analysis record and the like from the analysis record log 151, and transmits the data in association with the test institution ID, to the management server via the communication unit 14.

(Communication unit 14)

The communication unit 14 allows the gene analysis apparatus 1 to communicate with the management server 3 via the network 4. Data transmitted from the communication unit 14 to the management server 3 can include the test institution ID, gene panel IDs, gene IDs, analysis records, update requests, and the like. Data received from the management server 3 can include gene panel information, gene names that can be updated, and the like.

(Reading of read sequence by sequencer 2)

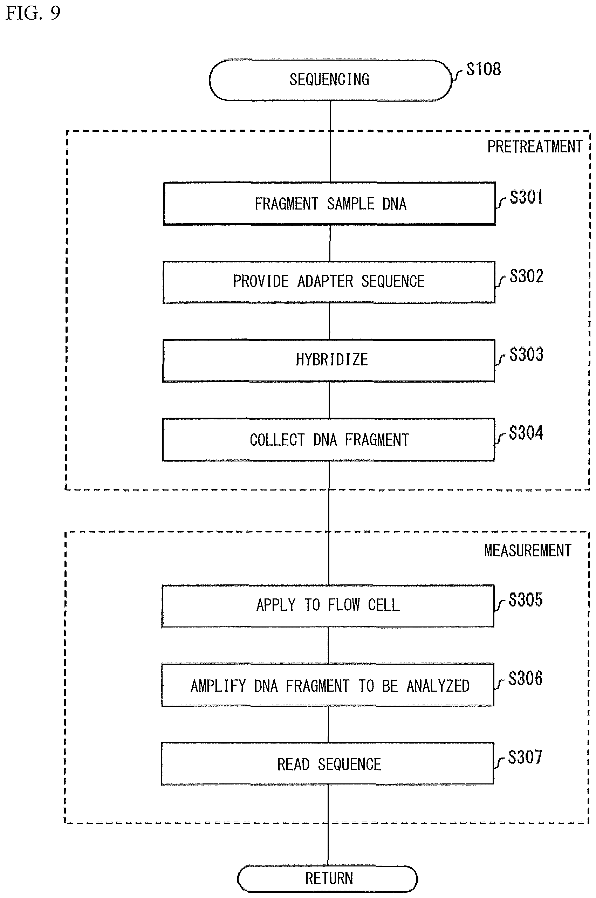

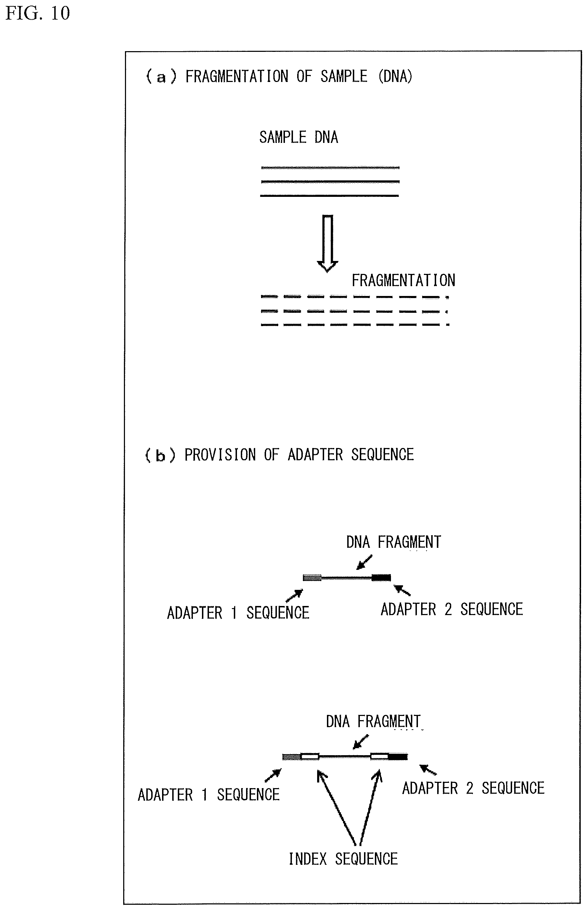

Here, the procedure of sequencing shown in S108 in FIG. 2 is described, while following the process flow shown in FIG. 9 with reference to FIG. 10 to FIG. 15 as appropriate. FIG. 9 is a flow chart describing an example of a procedure performed by the sequencer 2 from pretreatment to sequencing for analyzing the base sequence of sample DNA.

The type of the sequencer 2 that can be used in the present embodiment is not limited in particular, and any sequencer that can analyze a plurality of analysis targets in one run can be suitably used. In the following, an example case in which a sequencer of Illumina, Inc. (San Diego, CA) (for example, MySeq, HiSeq, NextSeq, or the like), or an apparatus that employs a method similar to that of the sequencer of Illumina, Inc. is used.

Through a combination of a Bridge PCR method and a Sequencing-by-synthesis technique, the sequencer of Illumina, Inc. can perform sequencing, with a target DNA being amplified and synthesized to a huge number on a flow cell.

(a. Pretreatment)

First, as shown in FIG. 10(a), a sample (DNA) is fragmented so as to have a length with which the sequencer 2 reads the sequence (step S301 in FIG. 9). The sample DNA can be fragmented by a known method such as sonication or a process using a reagent that fragments nucleic acid. Each obtained DNA fragment (nucleic acid fragment) can have a length of several ten to several hundred bp, for example. In the following, an example case in which the gene to be analyzed is DNA is described, but the gene to be analyzed may be RNA.

Next, as shown in FIG. 10(b), adapter sequences according to the type of the sequencer 2 and the sequencing protocol to be used are provided to both ends (3' end and 5' end) of each DNA fragment obtained in step S301 (step S302 in FIG. 9). This step is indispensable when the sequencer 2 is a sequencer of Illumina, Inc. or an apparatus that employs a method similar to that of the sequencer of Illumina, Inc. However, when another type of sequencer 2 is used, this step can be omitted in some cases.

The adapter sequence is a sequence to be used for performing sequencing in a later step. In one embodiment, the adapter sequence in Bridge PCR can be a sequence that is hybridized with an oligo DNA immobilized on the flow cell.

In one aspect, as shown in the upper part of FIG. 10(b), the adapter sequences (for example, adapter 1 sequence and adapter 2 sequence in FIG. 10) may be added directly to both ends of the DNA fragment. The adapter sequences may be added to the DNA fragment by using a known technique in this technical field. For example, the DNA sequence may be blunted and ligated with the adapter sequences.

In another aspect, as shown in the lower part of FIG. 10(b), index sequences may be inserted between both ends of the DNA fragment and the adapter sequences.

The index sequence is a sequence for distinguishing data of each sample. The index sequence is unique to each sample, each gene panel, and each company that provides gene panels. For example, a base sequence used as the index sequence has, but not limited to: a given length; and a sequence pattern such as 10 to 14 consecutive adenines, or 5 to 7 consecutive adenines followed by 5 to 7 consecutive guanines.

The index sequence can be used for identifying, on the basis of the sequence pattern and the length thereof, information related to the following: with respect to the sequence of the DNA fragment having the index sequence added thereto, which sample is the source of the read sequence information, which gene panel was used, which company provides the gene panel having been used, and the like. A configuration for identifying information related to a panel by use of the index sequence will be described later in detail (see embodiment 4).

For example, the index sequence in an analysis using a gene panel A may have a sequence pattern of 14 consecutive adenines, and the index sequence in an analysis using a gene panel B may have a sequence pattern of 7 consecutive adenines followed by 7 consecutive guanines. Alternatively, the index sequence in an analysis using the gene panel A may have a sequence of 14 consecutive adenines (i.e., the length of the index sequence is 14), and the index sequence in an analysis using a gene panel C may have a sequence of 10 consecutive adenines (i.e., the length of the index sequence is 10).

The index sequence and the adapter sequences can be added to the DNA fragment by using a known technique in this technical field. For example, the DNA fragment may be blunted and ligated with the index sequence, and then, further ligated with the adapter sequence.

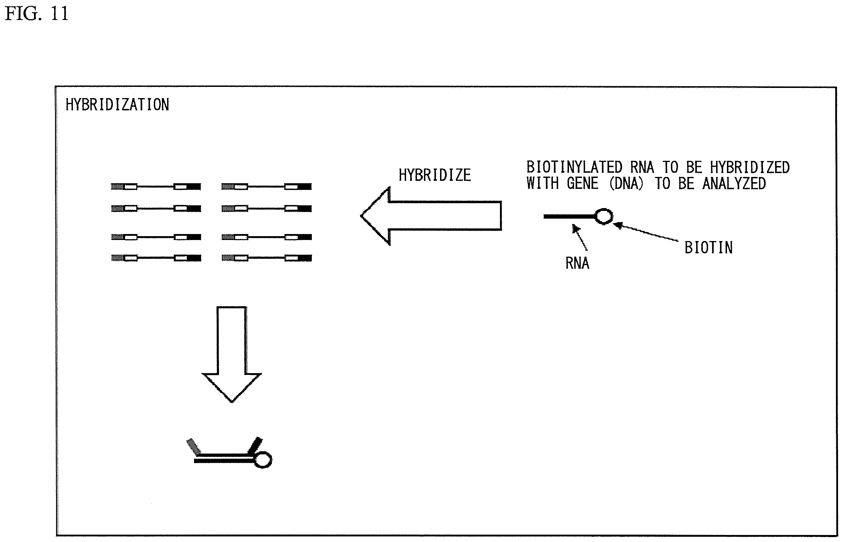

Next, as shown in FIG. 11, a biotinylated RNA bait library is caused to be hybridized with the DNA fragments provided with the adapter sequences (step S303 in FIG. 9). The biotinylated RNA bait library is composed of biotinylated RNAs (hereinafter, referred to as RNA bait) that are to be hybridized with genes to be analyzed. The RNA bait may have any length. However, in order to enhance specificity, a long oligo RNA bait of about 120 bp may be used, for example.

In a panel test using the sequencer 2 in the present embodiment, a large number of genes (for example, 100 or more) are analyzed. The reagent to be used in the panel test includes a set of RNA baits that respectively correspond to the large number of genes. When a different panel is used, the number and the types of genes to be tested are different. Thus, the set of RNA baits included in the reagent to be used in the panel test is also different.

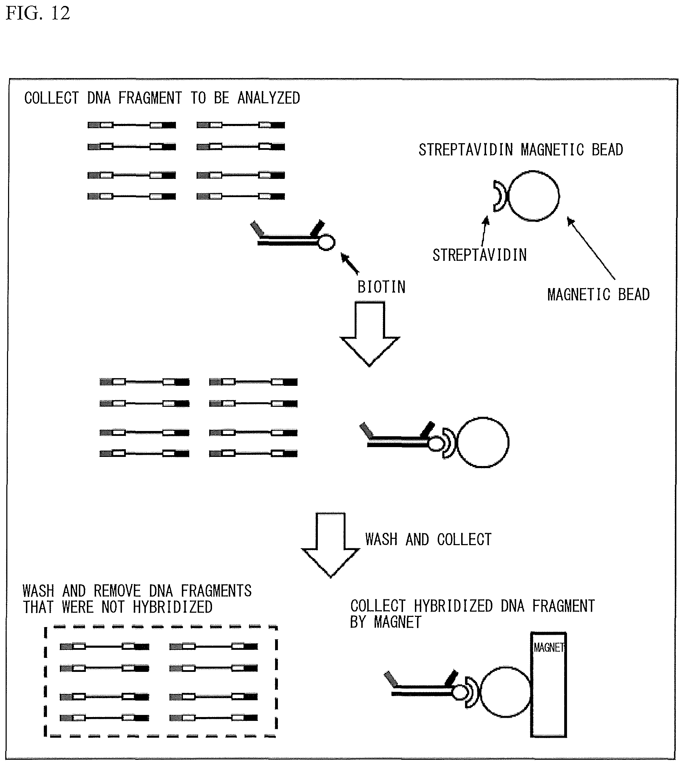

Then, as shown in FIG. 12, the DNA fragments to be analyzed are collected (step S304 in FIG. 9). Specifically, as shown in the upper part of FIG. 12, the DNA fragments hybridized with the biotinylated RNA bait library are mixed with streptavidin magnetic beads which are each composed of streptavidin and a magnetic bead bound to each other. Accordingly, as shown in the middle part of FIG. 12, the streptavidin part of the streptavidin magnetic bead and the biotin part of the RNA bait are bound to each other.

Then, as shown in the lower part of FIG. 12, the streptavidin magnetic beads are collected by a magnet, and fragments that are not hybridized with the RNA baits (i.e., DNA fragments that are not to be analyzed) are removed by washing. Accordingly, the DNA fragments hybridized with the RNA baits, i.e., the DNA fragments to be analyzed can be selected and concentrated. The sequencer 2 reads the nucleic acid sequences of the DNA fragments thus selected by use of a plurality of RNA baits, thereby obtaining a plurality of read sequences.

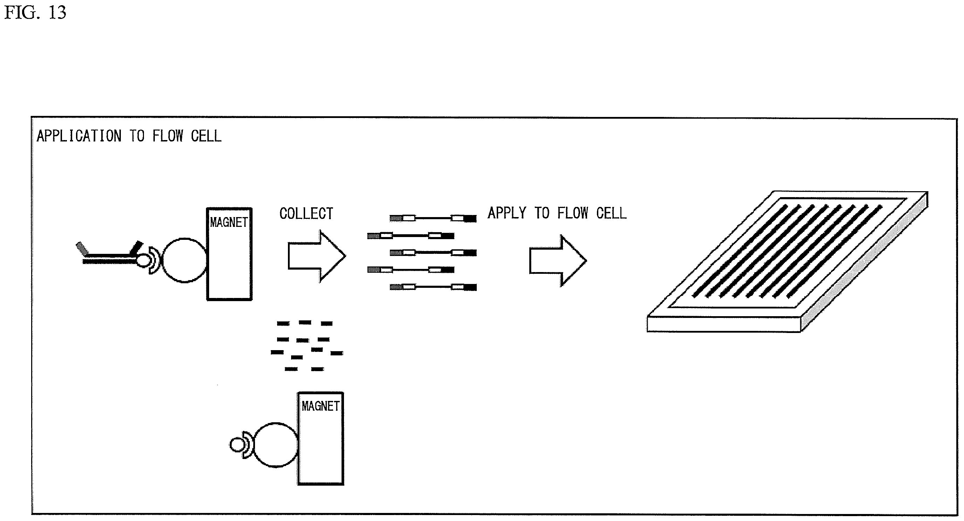

Further, as shown from the left part to the center part of FIG. 13, the streptavidin magnetic beads and the RNA baits are removed from the concentrated DNA fragments, and the resultant DNA fragments are amplified through PCR, whereby the pretreatment is completed.

(b. Sequencing)

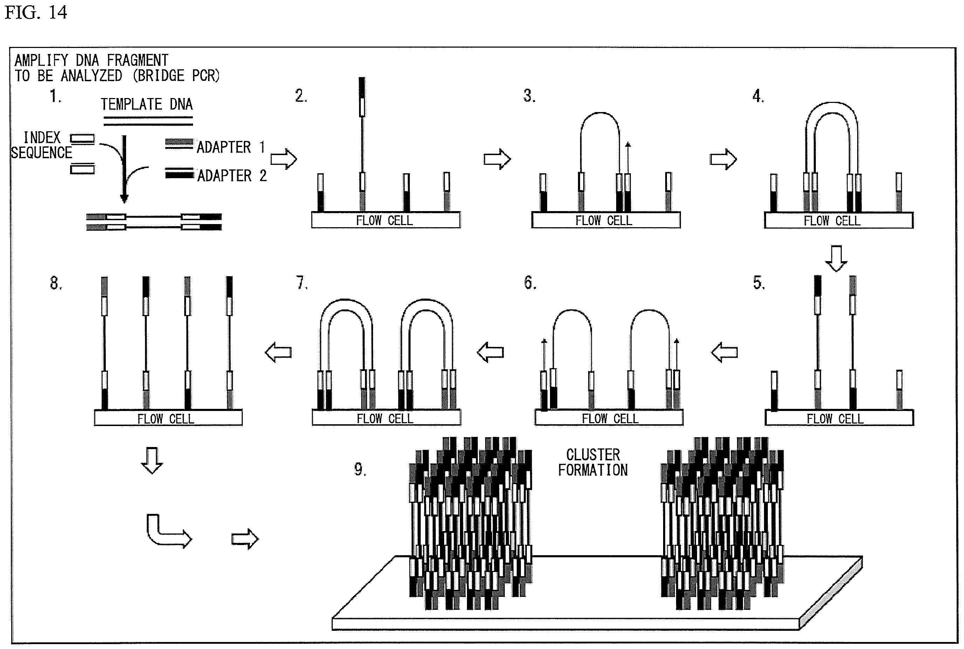

First, as shown in the right section of FIG. 13, the sequences of the amplified DNA fragments are applied to a flow cell (step S305 in FIG. 9). Subsequently, as shown in FIG. 14, the DNA fragments to be analyzed are amplified on the flow cell through Bridge PCR (step S306 in FIG. 9).

That is, each DNA fragment to be analyzed (for example, Template DNA in FIG. 14) is in a state where both ends of the DNA fragment have two different types of adapter sequences (for example, adapter 1 sequence and adapter 2 sequence in FIG. 14) added thereto through the pretreatment described above ("1" in FIG. 14). This DNA fragment is separated into single strands, and the adapter 1 sequence on the 5' end side is immobilized on the flow cell ("2" in FIG. 14). On the flow cell, the adapter 2 sequence on the 5' end side is immobilized in advance. The adapter 2 sequence on the 3' end side of the DNA fragment is bound to the adapter 2 sequence on the 5' end side on the flow cell to produce a bridge-like state, whereby a bridge is formed ("3" in FIG. 14). When DNA elongation is caused by DNA polymerase in this state ("4" in FIG. 14) and then denaturation is caused, two single-stranded DNA fragments are obtained ("5" in FIG. 14). Through repetition of the bridge formation, the DNA elongation, and the denaturation in this order, a large number of single-stranded DNA fragments can be locally amplified and immobilized, whereby clusters can be formed ("6" to "10" in FIG. 14).

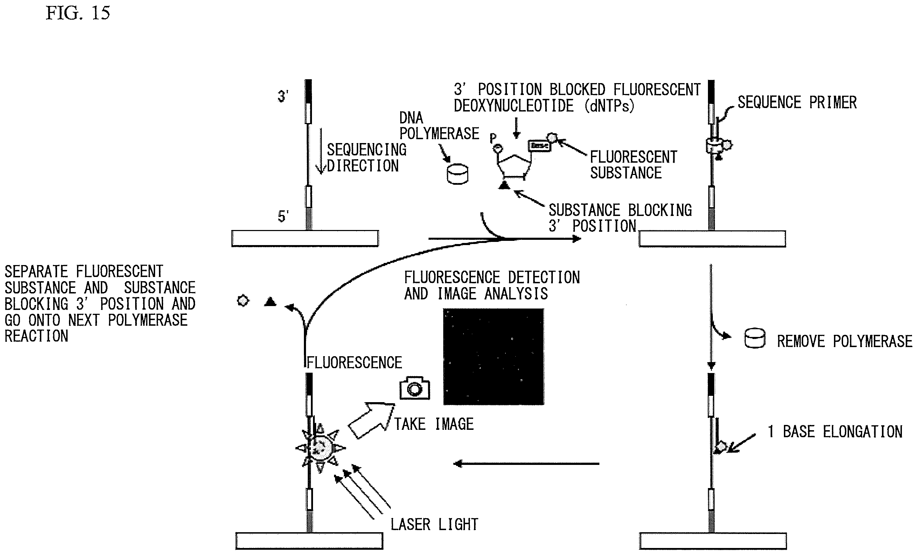

Then, as shown in FIG. 15, while the single-stranded DNA forming a cluster is used as a template, the sequence is read through Sequencing-by-synthesis (step S307 in FIG. 9).

First, to the single-stranded DNA immobilized on the flow cell (the upper left part of FIG. 15), DNA polymerase, and dNTP that is labeled with fluorescence and of which the 3' end side is blocked, are added (the upper center part of FIG. 15), and a sequence primer is further added thereto (the upper right part of FIG. 15). The sequence primer may be any sequence primer that is designed to be hybridized with a part of the adapter sequence, for example. In other words, it is sufficient that the sequence primer is designed to amplify the DNA fragment derived from the sample DNA. In a case where an index sequence is added, it is sufficient that the sequence primer is designed to further amplify the index sequence.

After the sequence primer is added, one base elongation is caused by the DNA polymerase, using dNTP labeled with fluorescence and having the 3' end blocked. Since the dNTP having the 3' end side blocked is used, the polymerase reaction stops when one base elongation has been realized. Then, the DNA polymerase is removed (the right middle part of FIG. 15), laser light is applied to the single-stranded DNA elongated by one base (lower right part of FIG. 15) to excite the fluorescent substance bound to the base, and a photograph of light generated at this time is taken and recorded (the lower left part of FIG. 15). In order to determine four kinds of bases, a photograph is taken by a fluorescence microscope for each of fluorescent colors respectively corresponding to A, C, G, and T, while wavelength filters are changed. After all photographs have been obtained, bases are determined from the photograph data. Then, the fluorescent substance and the protecting group blocking the 3' end side are removed, and the reaction goes onto the next polymerase reaction. With this flow assumed as one cycle, the second cycle, the third cycle, and so on are performed, whereby sequencing of the entire length can be performed.

According to the technique described above, the length of the chain that can be analyzed reaches 150 bases × 2, and analysis in a unit much smaller than the unit of a picotiter plate can be performed. Thus, due to the high density, a huge amount of sequence information of 40 to 200 Gb can be obtained in one analysis.

(c. Gene panel)

The gene panel to be used for reading the read sequences by the sequencer 2 means an analysis kit for analyzing a plurality of analysis targets in one run as described above. In one embodiment, the gene panel can be an analysis kit for analyzing a plurality of gene sequences related to a specific disease.

When used herein, the term "kit" is intended to mean a package that includes containers (for example, bottles, plates, tubes, and dishes) each containing a specific material. Preferably, the kit includes instructions for using each material. When used in the context of a kit herein, "include (is included)" is intended to mean a state of being included in any of individual containers that form a kit. The kit can be a single package of a plurality of different compositions, and the forms of the compositions can be those described above. In the case of a solution form, the solution may be contained in a container. The kit may include a substance A and a substance B that are mixed in one container, or that are in separate containers. The "instructions" indicate the procedure of applying each component in the kit to a therapy and/or diagnosis. The "instructions" may be written or printed on paper or any other medium, or may be stored in a magnetic tape, or an electronic medium such as a computer readable disk or tape or a CD-ROM. The kit can include a container that contains a diluent, a solvent, a washing liquid, or another reagent. Further, the kit may also include an apparatus that is necessary for the kit to be applied to a therapy and/or diagnosis.

In one embodiment, the gene panel may be provided with one or more of: reagents such as the reagent for fragmenting nucleic acid, the ligation reagent, the washing liquid, and the PCR reagent (dNTP, DNA polymerase, etc.); and magnetic beads, which have been described above. The gene panel may be provided with one or more of: oligonucleotides for adding the adapter sequences to the fragmented DNA; oligonucleotides for adding the index sequence to the fragmented DNA; the RNA bait library; and the like.

In particular, the index sequence provided to each gene panel can be a sequence that is unique to the gene panel and that identifies the gene panel. The RNA bait library provided to each gene panel can be a library that is unique to the gene panel and that includes RNA baits that correspond to the test genes of the gene panel.

(Sequence data reading unit 111, data adjustment unit 113, and mutation identification unit 114)

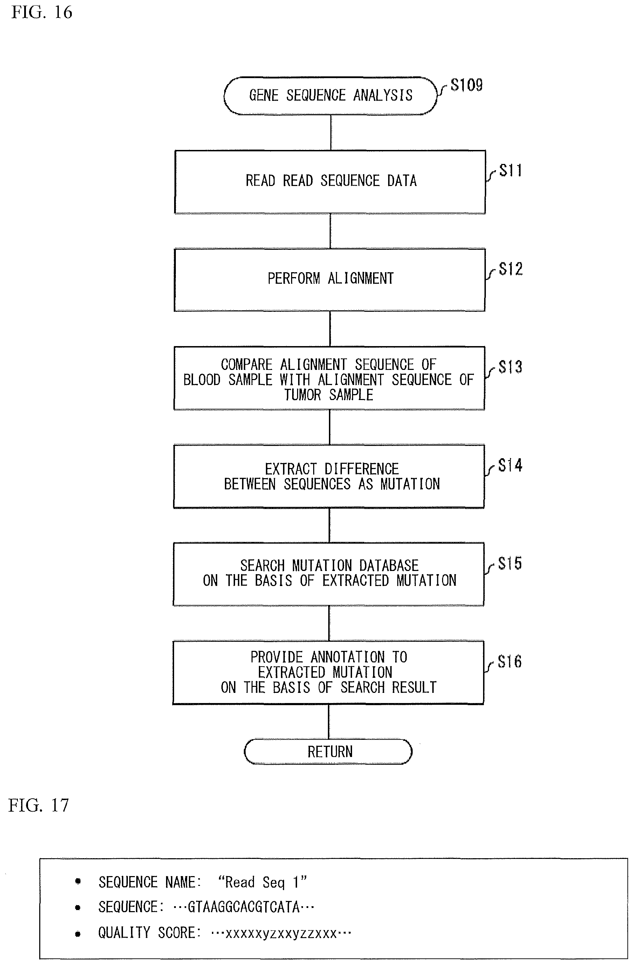

Next, the processes performed by the sequence data reading unit 111, the data adjustment unit 113, and the mutation identification unit 114 of the analysis execution unit 110 are described, while following the process flow shown in FIG. 16 with reference to FIG. 17 to FIG. 26 as appropriate.

FIG. 16 is a flow chart describing an example of the flow of analysis performed by the gene analysis apparatus 1. The process shown in FIG. 16 corresponds to the step S109 shown in FIG. 2.

<Sequence data reading unit 111>

First, in step S11 in FIG. 16, the sequence data reading unit 111 reads read sequence information provided from the sequencer 2.

The read sequence information is data that indicates a base sequence read by the sequencer 2. The sequencer 2 performs sequencing on a large number of nucleic acid fragments obtained by use of a specific gene panel, reads sequence information thereof, and provides the sequence information as read sequence information, to the gene analysis apparatus 1.

In one aspect, the read sequence information may include the sequence having been read and a quality score of each base in the sequence. Both of read sequence information obtained by subjecting an FFPE sample from a lesion site of a subject to the sequencer 2, and read sequence information obtained by subjecting a blood sample of the subject to the sequencer 2 are inputted to the gene analysis apparatus 1.

FIG. 17 shows an example of a file format for read sequence information. In the example shown in FIG. 17, the read sequence information includes a sequence name, a sequence, and a quality score. The sequence name may be a sequence ID or the like provided to the read sequence information outputted by the sequencer 2. The sequence indicates the base sequence read by the sequencer 2. The quality score indicates the probability of incorrect base assignment performed by the sequencer 2. Any base sequence quality score (Q) is represented by the following equation.

$$Q = -10 \log_{10} E$$

In this equation, E represents an estimated value of the probability of incorrect base assignment. The greater the value of Q is, the lower the probability of the error is. The smaller the value of Q, the greater the portion of the read that cannot be used is. In addition, false-positive mutation assignment also increases, which could result in lowered accuracy of the result. "False-positive" means that the read sequence is determined as having a mutation although the read sequence does not have a true mutation as a determination target. "Positive" means that the read sequence has a true mutation as a determination target, and "negative" means that the read sequence does not have any mutation as a determination target.

<Data adjustment unit 113>

Next, in step S12 in FIG. 16, on the basis of the read sequence information read by the sequence data reading unit 111, the data adjustment unit 113 performs alignment of the read sequence of each nucleic acid fragment included in the read sequence information.

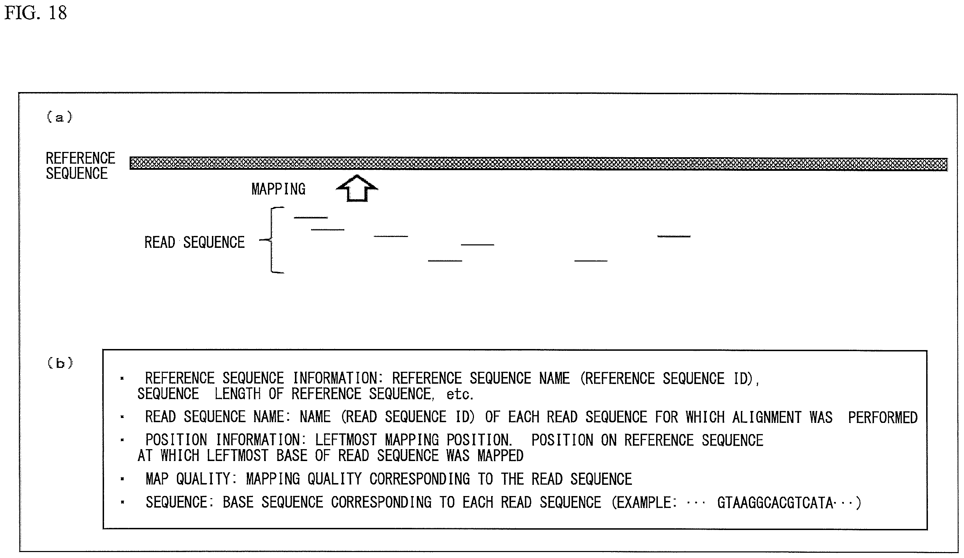

FIG. 18(a) illustrates alignment performed by the data adjustment unit 113. The data adjustment unit 113 refers to reference sequences stored in the reference sequence database 122, and maps the read sequence of each nucleic acid fragment to a reference sequence with which the read sequence information should be compared, thereby performing alignment. In one aspect, a plurality of types of reference sequences that correspond to respective analysis target genes are stored in the reference sequence database 122.

The data adjustment unit 113 performs alignment for both of read sequence information obtained by subjecting an FFPE sample from a lesion site of a subject to the sequencer 2, and read sequence information obtained by subjecting a blood sample of the subject to the sequencer 2.

FIG. 18(b) shows an example of a format for a result of alignment performed by the data adjustment unit 113. The format for the alignment result is not limited in particular, and may be any format that can specify the read sequence, the reference sequence, and the mapping position. As shown in FIG. 18(b), the format may include reference sequence information, read sequence name, position information, map quality, and sequence.

The reference sequence information is information indicating the reference sequence name (reference sequence ID), the sequence length of the reference sequence, and the like in the reference sequence database 122. Preferably, the reference sequence information can identify the reference sequence, and includes the reference sequence name and the reference sequence ID, for example. The read sequence name is information indicating the name (read sequence ID) of each read sequence for which the alignment has been performed.

The position information is information indicating the position (leftmost mapping position) on the reference sequence at which the leftmost base of the read sequence has been mapped. The map quality is information indicating the quality of mapping that corresponds to the read sequence. The sequence is information indicating the base sequence (example: GTAAGGCACGTCATA...) that corresponds to each read sequence.

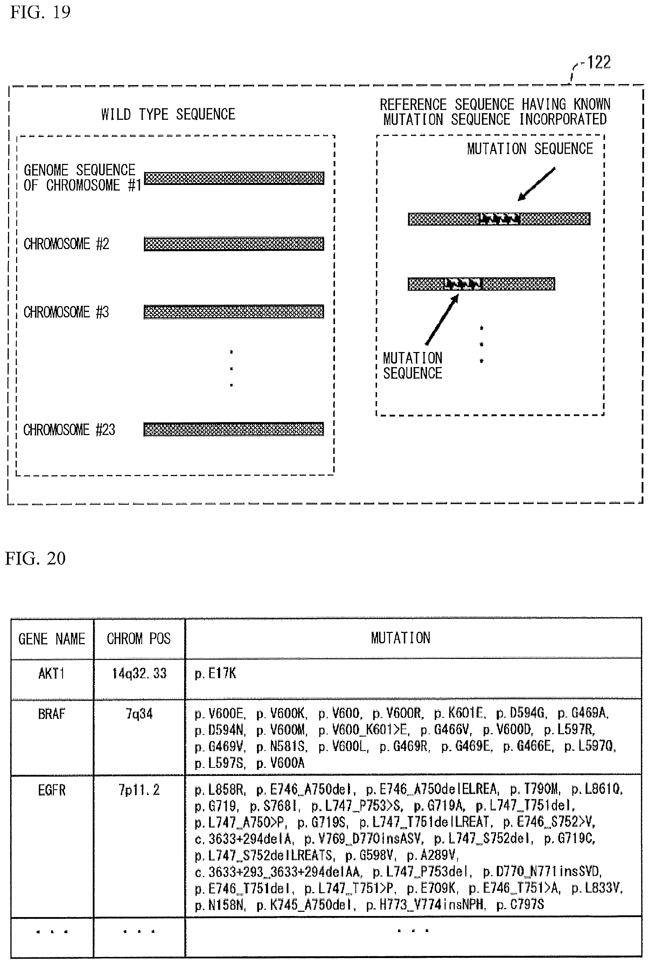

FIG. 19 shows an example of a structure of the reference sequence database 122. As shown in FIG. 19, the reference sequence database 122 stores reference sequences indicating wild type sequences (for example, genome sequences of chromosomes #1 to 23), and reference sequences in which known mutations are incorporated in wild type sequences.

Further, each reference sequence in the reference sequence database 122 is provided with metadata that indicates gene panel information. For example, the gene panel information provided to each reference sequence can be information that directly or indirectly indicates an analysis target gene that corresponds to the reference sequence.

In one embodiment, the information selection unit 112 may perform control such that, when the data adjustment unit 113 obtains a reference sequence from the reference sequence database 122, the data adjustment unit 113 refers to the inputted gene panel information and the metadata of each reference sequence, and selects a reference sequence that corresponds to the gene panel information. For example, in one aspect, the information selection unit 112 may control the data adjustment unit 113 so as to select a reference sequence that corresponds to an analysis target gene that is specified by the inputted gene panel information. This allows the data adjustment unit 113 to perform mapping only on the reference sequence related to the gene panel having been used, and thus, efficiency of the analysis can be improved.

In another embodiment, the information selection unit 112 may not necessarily perform the above-described control. In this case, the information selection unit 112 only needs to control the mutation identification unit 114 or the report creation unit 115 as described later.

FIG. 20 shows an example of known mutations incorporated in reference sequences (that do not indicate wild-type sequences) included in the reference sequence database 122.

The known mutations are mutations registered in external databases (for example, COSMIC, ClinVar, etc.), and the chromosome position, the gene name, and the mutation have been identified as shown in FIG. 20.

In the example shown in FIG. 20, mutations of amino acids are specified. However, mutations of nucleic acids may be specified. The types of mutation are not limited in particular. The mutation may be any of various mutations such as substitution, insertion, and deletion, or may be a mutation in which a sequence of a part of another chromosome or reverse complement sequence is bound.

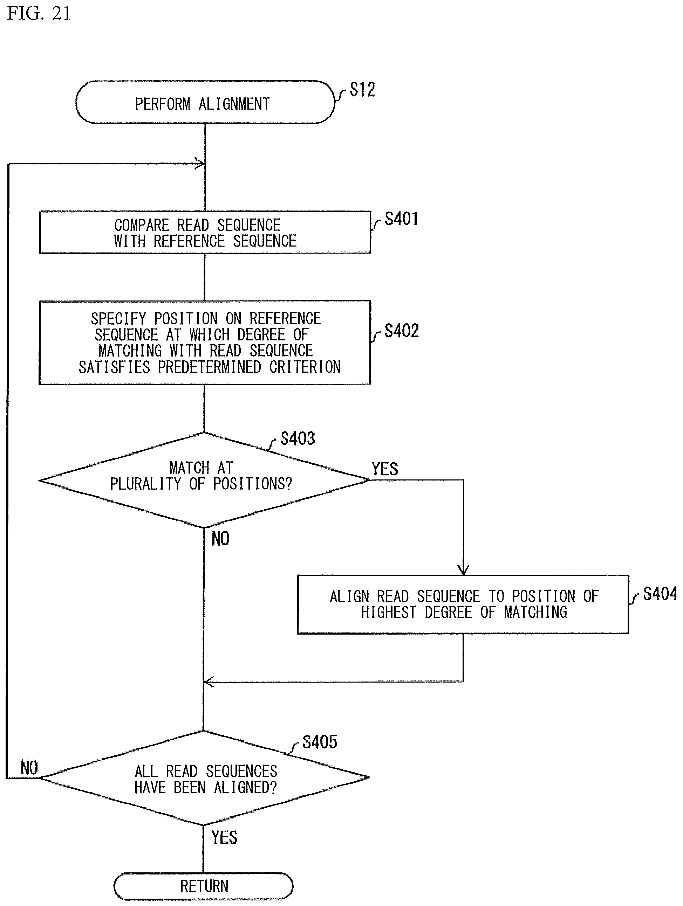

FIG. 21 is a flow chart describing in detail an example of a step of alignment performed in step S12 in FIG. 16. In one aspect, the alignment in step S12 in FIG. 16 is performed in steps S401 to S405 shown in FIG. 21.

In step S401 in FIG. 21, the data adjustment unit 113 selects a read sequence that has not been subjected to alignment, out of the read sequences of the nucleic acid fragments included in the read sequence information obtained by the sequence data reading unit 111, and compares the selected read sequence with a reference sequence obtained from the reference sequence database 122. Then, in step S402, the data adjustment unit 113 specifies a position, on the reference sequence, at which the degree of matching with the read sequence satisfies a predetermined criterion. The degree of matching is a value that indicates how much the obtained read sequence information and the reference sequence match each other. Examples of the degree of matching include the number or proportion of bases that match each other.

In one aspect, the data adjustment unit 113 calculates a score that indicates the degree of matching between the read sequence and the reference sequence. The score indicating the degree of matching can be, for example, a percentage identity between two sequences.

For example, the data adjustment unit 113 specifies the number of positions at which bases of the read sequence and bases of the reference sequence are the same, obtains the number of the matched positions, and divides the number of the matched positions by the number (the number of bases in the comparison window) of bases of the read sequence compared with the reference sequence, thereby calculating the percentage.

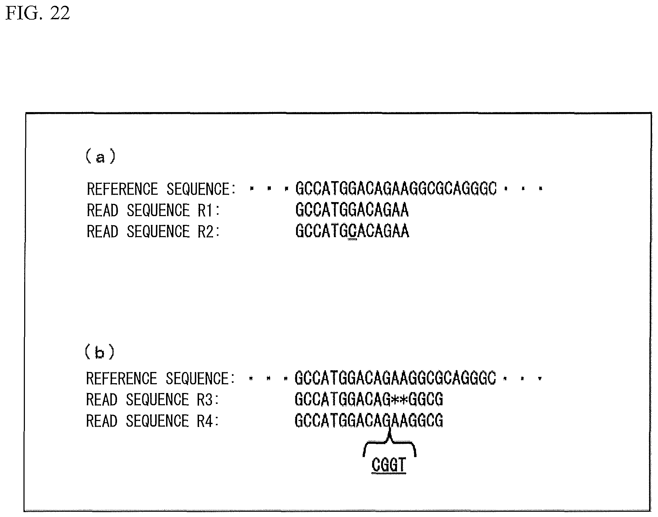

FIG. 22(a) shows an example of score calculation. In one aspect, at the positions shown in FIG. 22(a), the score of the degree of matching between a read sequence R1 and the reference sequence is 100% because 13 bases out of 13 bases of the read sequence match the bases of the reference sequence. The score of the degree of matching between a read sequence R2 and the reference sequence is 92.3% because 12 bases out of 13 bases of the read sequence match the bases of the reference sequence.

In the calculation of the score indicating the degree of matching between a read sequence and a reference sequence, the data adjustment unit 113 may perform calculation such that, when the read sequence includes a predetermined mutation (for example, Indel: Insertion/Deletion) with respect to the reference sequence, a score lower than that calculated in the normal calculation is obtained.

In one aspect, for a read sequence that includes at least one of insertion and deletion with respect to a reference sequence, the data adjustment unit 113 may correct the score by, for example, multiplying the score calculated in the above-described normal calculation, by a weighting factor according to the number of bases corresponding to the insertion/deletion. The weighting factor W may be calculated as, for example, $W=\{1-(1/100)\times(\text{the number of bases corresponding to insertion/deletion})\}$.

FIG. 22(b) shows another example of the score calculation. In one aspect, at the positions shown in FIG. 22(b), the score of the degree of matching between a read sequence R3 and the reference sequence is 88% in the normal calculation because 15 bases out of 17 bases of the read sequence (the symbol * indicating a deletion is also calculated as one base) match the bases of the reference sequence. The corrected score is $86\%=88\%\times0.98$.

The score of the degree of matching between a read sequence R4 and the reference sequence is 81% in the normal calculation because 17 bases out of 21 bases of the read sequence match the bases of the reference sequence. The corrected score is $77.8\%=81\%\times0.96$.

The data adjustment unit 113 calculates the score of the degree of matching while changing the mapping position of the read sequence with respect to each reference sequence, thereby specifying a position on the reference sequence at which the degree of matching with the read sequence satisfies a predetermined criterion. At this time, an algorithm known in this technical field, such as dynamic programming, the FASTA method, and the BLAST method, may be used.

With reference back to FIG. 21, next, when the degree of matching with the read sequence satisfies the predetermined criterion at a single position on the reference sequence (NO in step S403), the data adjustment unit 113 aligns the read sequence to this position. When the degree of matching with the read sequence satisfies the predetermined criterion at a plurality of positions on the reference sequence (YES in step S403), the data adjustment unit 113 aligns the read sequence to the position at which the degree of matching is highest (step S404).

When all the read sequences included in the read sequence information obtained by the sequence data reading unit 111 have not been aligned (NO in step S405), the data adjustment unit 113 returns to step S401. When all the read sequences included in the read sequence information have been aligned (YES in step S405), the data adjustment unit 113 completes the process of step S12.

<Other functions of data adjustment unit 113>

The data adjustment unit 113 may output, as an analysis result of the read sequence information, a comparison result obtained by comparing the read sequence information with sequence information of an analysis target gene of the gene panel associated with the obtained gene panel information.

The sequence information of an analysis target gene of the gene panel can include the sequence of the gene to be analyzed (for example, exon), and an index sequence added to the sequence of the gene to be analyzed.

For example, in the cases of (1) and (2) below, the data adjustment unit 113 may cause the display unit 16 to display an error as an analysis result of the read sequence information.

(1) When mapping of a read sequence is performed, the index sequence included in the read sequence information read by the sequence data reading unit 111 is different from the index sequence (see FIG. 39, for example) corresponding to the gene panel information obtained by the information selection unit 112.

(2) The read sequence information includes not less than a predetermined number of sequences of genes that are not analysis target genes of the gene panel corresponding to the gene panel information obtained by the information selection unit 112; or the read sequence information only includes less than a predetermined number of sequences of analysis target genes of the gene panel indicated by the gene panel information obtained by the information selection unit.

These cases are highly likely to be caused by an erroneous input of gene panel information by the user. Thus, the data adjustment unit 113 may cause the display unit 16 to display an error such as "Analysis cannot be performed" and "There is an error in gene panel information", and the like.

Alternatively, the data adjustment unit 113 may cause the display unit 16 to further display a message such as "Please input gene panel information again", to urge the user to input the gene panel name, the name of the analysis target gene, and the like again.

The display unit 16 may display an error only when the number of pieces of read sequence information that include the sequences of genes that are not analysis target genes of the gene panel corresponding to the gene panel information is not less than a predetermined number. Alternatively, an error may be displayed only when pieces of read sequence information include not less than a predetermined number of pieces of read sequence information for which mapping has been performed with respect to genes that are not analysis target genes of the gene panel corresponding to the gene panel information.

An example has been described in which the display unit 16 is used as the destination to which an error is outputted. However, the configuration for outputting an error is not limited thereto. For example, an error content may be outputted as sound from a speaker. Alternatively, an error may be indicated to the user by lighting or blinking a lamp or the like.

<Mutation identification unit 114>

Next, with reference back to FIG. 16, in step S13, the mutation identification unit 114 compares the sequence of the reference sequence (alignment sequence) with which the read sequences obtained from the sample collected from a lesion site of the subject have been aligned, with the sequence of the reference sequence with which the read sequences obtained from the blood sample of the same subject have been aligned.

Then, in step S14 in FIG. 16, the difference between the alignment sequences is extracted as a mutation. For example, if, at the same position of the same analysis target gene, the alignment sequence derived from the blood specimen is ATCGA, and the alignment sequence derived from a tumor tissue is ATCCA, the mutation identification unit 114 extracts the difference of G and C as a mutation.

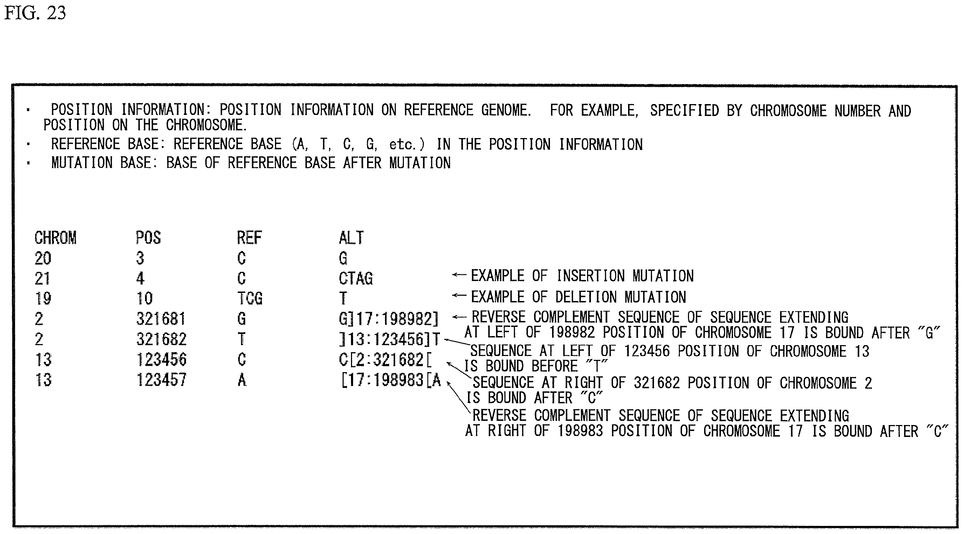

In one aspect, the mutation identification unit 114 generates a result file on the basis of the extracted mutation. FIG. 23 shows an example of a format for the result file generated by the mutation identification unit 114. The format can be based on the Variant Call Format (VCF), for example.

As shown in FIG. 23, in the result file, position information, reference base, and mutation base are described for each extracted mutation. The position information indicates the position on the reference genome, and includes the chromosome number and the position on the chromosome, for example. The reference base indicates a reference base (A, T, C, G, etc.) at the position indicated by the position information. The mutation base indicates the base after the mutation of the reference base. The reference base is the base on the alignment sequence derived from the blood specimen, and the mutation base is the base on the alignment sequence derived from the tumor tissue.

In FIG. 23, the mutation in which the reference base is C and the mutation base is G is an example of substitution mutation, the mutation in which the reference base is C and the mutation base is CTAG is an example of insertion mutation, and the mutation in which the reference base is TCG and the mutation base is T is an example of deletion mutation. The mutation in which the mutation base is G]17:198982], ]13:123456]T, C[2:321682[, or [17:198983[A is an example of mutation in which a sequence of a part of another chromosome or a reverse complement sequence is bound.

With reference back to FIG. 16, next, in step S15, the mutation identification unit 114 searches the mutation database 123. Then, in step S16, the mutation identification unit 114 refers to mutation information in the mutation database 123, and provides annotation to each mutation included in the result file, to identify the mutation.

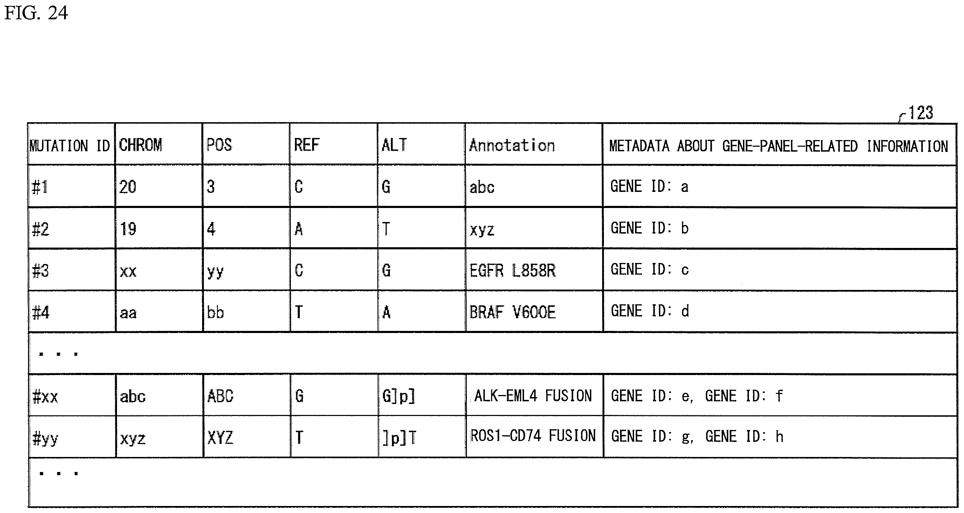

FIG. 24 shows an example of a structure of the mutation database 123. The mutation database 123 is constructed on the basis of an external database such as COSMIC or ClinVar, for example. In one aspect, each piece of mutation information in the database is provided with metadata about gene panel information. In the example shown in FIG. 24, each piece of mutation information in the database is provided, as metadata, a gene ID of an analysis target gene.

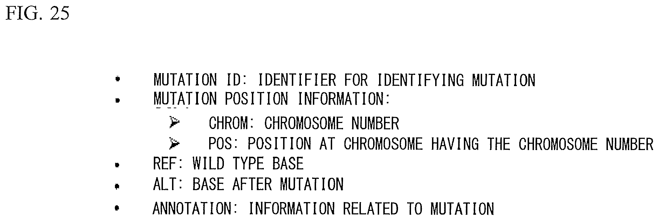

FIG. 25 shows a specific example of a structure of mutation information in the mutation database 123. In one aspect, as shown in FIG. 25, the mutation information included in the mutation database 123 may include mutation ID, mutation position information (for example, "CHROM" and "POS"), "REF", "ALT", and "Annotation". The mutation ID is an identifier for identifying a mutation. In the mutation position information, "CHROM" indicates the chromosome number and "POS" indicates the position on the chromosome having the chromosome number. "REF" indicates a base in the wild type, and "ALT" indicates a base after the mutation.

"Annotation" indicates information related to the mutation. For example, "Annotation" may be information that indicates a mutation of an amino acid such as "EGFR C2573G", "EGFR L858R", or the like. For example, "EGFR C2573G" indicates a mutation in which cysteine at the 2573rd residue of protein "EGFR" is substituted by glycine.

As in the example described above, "Annotation" of mutation information may be information for converting a mutation according to base information into a mutation according to amino acid information. In this case, on the basis of the information of "Annotation" that has been referred to, the mutation identification unit 114 can convert a mutation according to base information into a mutation according to amino acid information.

Using the information that specifies each mutation included in the result file as a key (for example, base information corresponding to the mutation position information and the mutation), the mutation identification unit 114 searches the mutation database 123. For example, using any one of pieces of information "CHROM", "POS", "REF", and "ALT" as a key, the mutation identification unit 114 may search the mutation database 123. When a mutation extracted by comparing the alignment sequence derived from the blood specimen and the alignment sequence derived from the lesion site has been registered in the mutation database 123, the mutation identification unit 114 identifies the mutation as a mutation existing in the sample, and provides annotation (for example, "EGFR L858R", "BRAF V600E", etc.) to the mutation included in the result file.

In one embodiment, before the mutation identification unit 114 searches the mutation database 123 on the basis of the result file, the information selection unit 112 may mask (exclude), in the result file, mutations that do not correspond to the gene panel information inputted to the mutation identification unit 114.

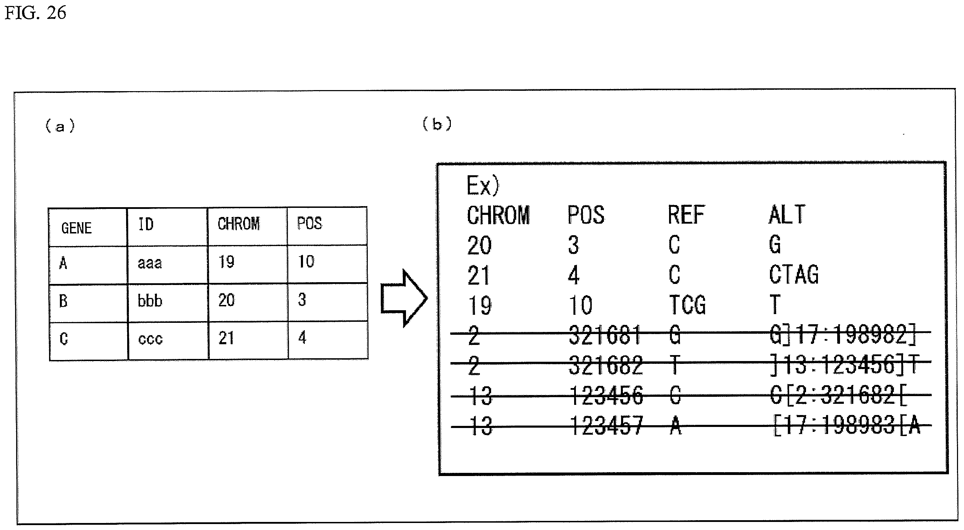

For example, in one aspect, the mutation identification unit 114 having been notified of the gene panel information from the information selection unit 112 may refer to a table indicating the correspondence relationship between each analysis target gene and the position information (for example, "CHROM" and "POS") as shown in FIG. 26(a), may specify the positions of mutations that correspond to the analysis target genes specified by the notified gene panel information, and may mask (exclude), in the result file, mutations at the other positions as shown in FIG. 26(b). Accordingly, the mutation identification unit 114 only has to provide annotation to the mutations, in the result file, that are related to the gene panel having been used. Thus, the mutation identifying efficiency can be improved.

In one embodiment, the information selection unit 112 may perform control such that, when the mutation identification unit 114 refers to mutation information in the mutation database 123 in order to provide annotation, the mutation identification unit 114 refers to the inputted gene panel information and the metadata of each piece of mutation information, and selectively refers to mutation information that corresponds to the gene panel information.

For example, in one aspect, the information selection unit 112 may control the mutation identification unit 114 such that the mutation identification unit 114 refers to mutation information that corresponds to the analysis target genes specified by the inputted gene panel information. Accordingly, the mutation identification unit 114 only has to refer to the mutation information, in the mutation database 123, that is related to the gene panel having been used. Thus, annotation providing efficiency can be improved.

It should be noted that, from all the identified mutations, a mutation that corresponds to the inputted gene panel information may be selected on the basis of the gene panel information, and information that is related to the selected mutation may be outputted as an analysis result of the read sequence information.

In this case, for example, it is sufficient that metadata of each piece of mutation information stored in the mutation database includes the gene ID of the analysis target gene, and, for each mutation of the gene, information as to whether or not the mutation is an analysis target of the gene panel.

According to this configuration, the mutation identification unit 114 may be controlled to refer to the gene panel information from the information selection unit 112 and metadata of each piece of mutation information, and to select, from all the identified mutations, only mutation information that corresponds to the gene panel information. For example, there may be cases where different gene panels have analysis target genes having the same gene ID, but mutations to be analyzed are different between the gene panels.

Even in such a case, if the above-described configuration is employed, the mutation identification unit 114 can output, to the report creation unit 115, only the mutation information that corresponds to the gene panel information inputted by the user. As the analysis result of the read sequence information, mutation information may be outputted from the output unit 13 or may be displayed on the display unit 16.

(Report creation unit 115)

When the entire exon region is analyzed by a panel test, many mutations are detected in genes of a subject. Here, mutations include those of which the clinical significance has not been confirmed or for which therapeutically effective drugs have not been established. Thus, such mutations provide information other than information that can be utilized by doctors for actual therapies. Doctors trying to apply the result of a genetic test to an actual therapy for a subject desire to selectively know mutations that can be utilized in the actual therapy among many detected mutations.

The report creation unit 115 creates a report on the basis of the information outputted by the mutation identification unit 114 and the gene panel information provided from the information selection unit 112 (corresponding to step S110 in FIG. 2). Information included in the created report includes the gene panel information, and the information related to the identified mutations.

On the basis of the gene panel information from the information selection unit 112, the report creation unit 115 selects the target to be included in the report and deletes, from the report, the information that has not been selected. Alternatively, the information selection unit 112 may control the report creation unit 115 such that information related to genes that correspond to the gene panel information inputted through the input unit 17 is selected as the target to be included in the report, and information that has not been selected is deleted from the report.

(Output unit 13)

The report created by the report creation unit 115 may be transmitted in the form of data, as an analysis result of the read sequence information, from the output unit 13 to the terminal device 5 provided at the medical institution 210 (corresponding to step S111 in FIG. 2). Alternatively, the report may be transmitted to a printer (not shown) that is connected to the gene analysis apparatus 1, printed by the printer, and then sent in the form of a paper medium from the test institution 120 to the medical institution 210.

[Embodiment 2]

Another embodiment of the present invention is describe below. For convenience of description, the members having the same functions as those of the members described in the above embodiment are denoted by the same reference characters, and description thereof is not repeated.

(Configuration of gene analysis apparatus 1a)

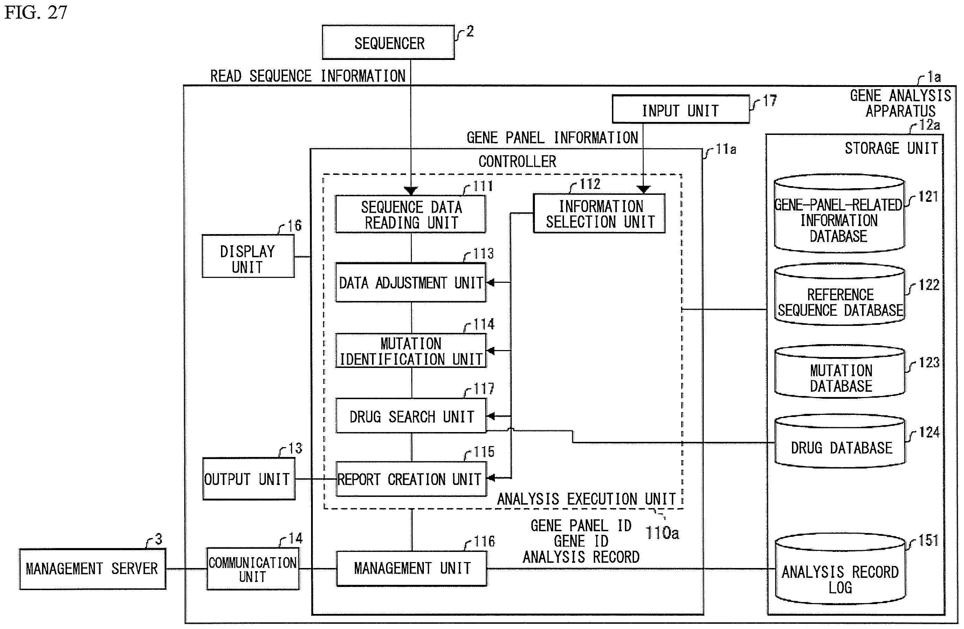

Here, a gene analysis apparatus 1a capable of creating a report that includes information related to drugs (drug information) that are related to mutations identified by the mutation identification unit 114 is described with reference to FIG. 27.

FIG. 27 shows an example of a configuration of the gene analysis apparatus 1a. The gene analysis apparatus 1a is different from the gene analysis apparatus 1 shown in FIG. 4 in that an analysis execution unit 110a further includes a drug search unit 117, and a first storage unit 12a further includes a drug database 124.

(Drug search unit 117)

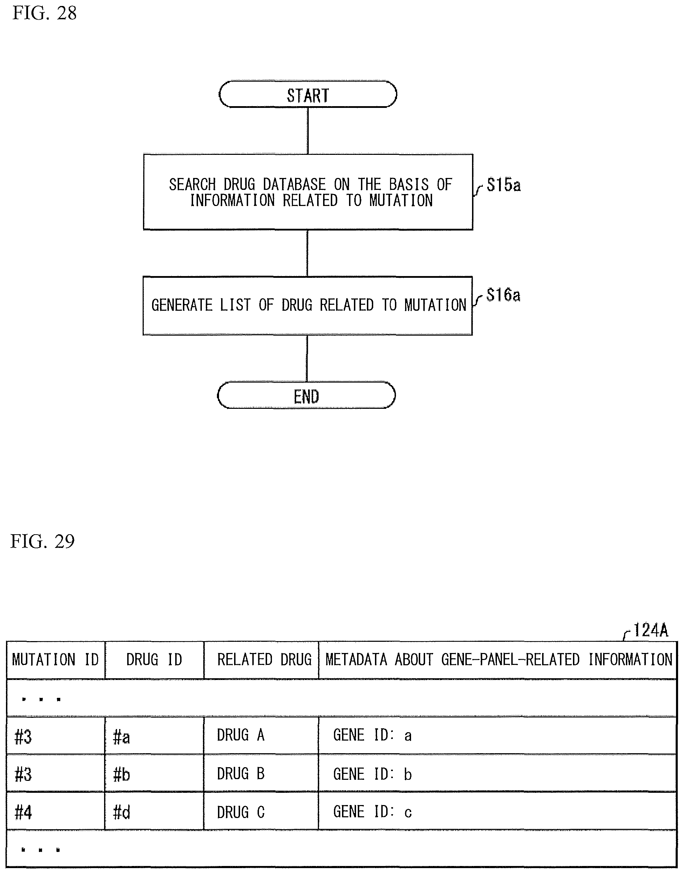

The flow of a process in which the drug search unit 117 generates a list that includes information related to drugs is described with reference to FIG. 28. FIG. 28 is a flow chart showing an example of a process in which the drug search unit 117 generates a list of drugs related to mutations.

Using the mutation ID provided to each mutation identified by the mutation identification unit 114 as a key, the drug search unit 117 searches the drug database 124 (step S15a). On the basis of the search result, the drug search unit 117 generates a list that includes information related to drugs that are related to mutations (step S16a). The generated list is incorporated into the report created by the report creation unit 115.

(Drug database 124)

Data 124A stored in the drug database 124 and used when the drug search unit 117 searches the drug database 124 and generates a drug list is described with reference to FIG. 29. FIG. 29 shows an example of a data structure of the drug database 124.

As shown in FIG. 29, a mutation ID provided to each mutation, a related drug name, and a drug ID provided to each drug are stored in association with one another in the drug database 124. As in the case of mutation ID "#3" in FIG. 29 with which "drug A" and "drug B" are associated, each mutation ID may be associated with a plurality of related drugs.

Each mutation ID in the drug database 124 may be provided with "metadata about gene-panel-related information", which is metadata related to gene panel information. The drug search unit 117 refers to the "metadata about gene-panel-related information" in accordance with an instruction from the information selection unit 112.

Then, the drug search unit 117 changes the range in which the drug database 124 is searched, to a range indicated by the metadata. Accordingly, in accordance with "metadata about gene-panel-related information" provided to each drug and the inputted gene panel information, the drug search unit 117 can narrow the drugs that should be referred to in the drug database, and can generate a list that includes information related to drugs according to the gene panel information.

<Modification 1>

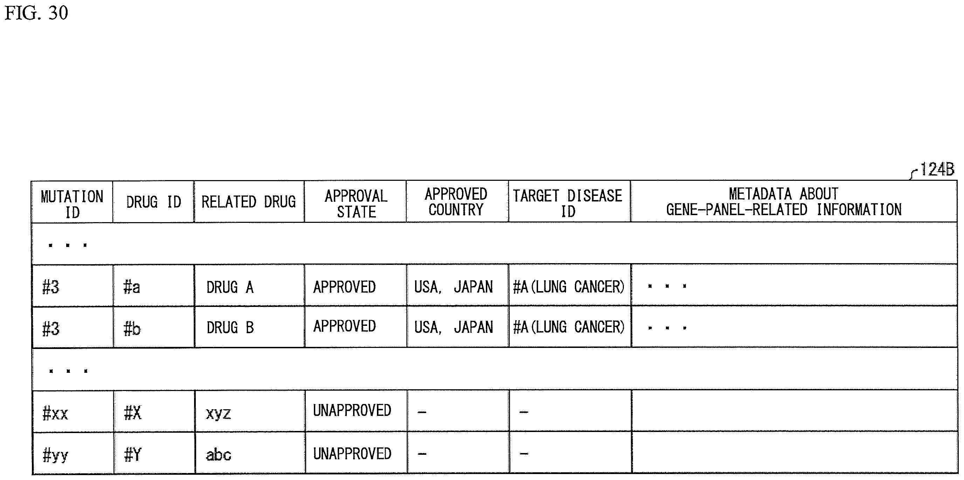

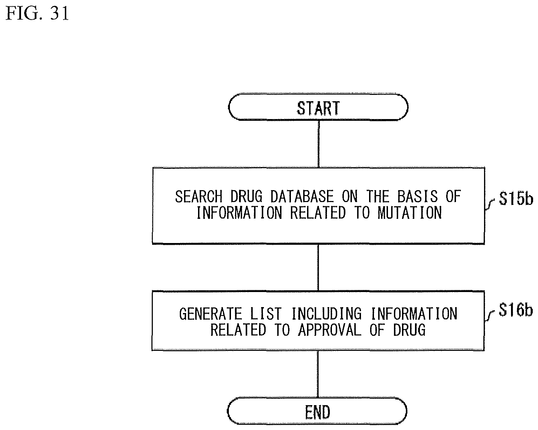

The drug search unit 117 may search the drug database 124 having the data structure shown in FIG. 30, and generate a list that includes another type of information related to drugs that are related to mutations. Specifically, in addition to the list of drugs related to mutations that is generated in Embodiment 2, drug approval information is added. This is described below with reference to FIG. 31. FIG. 31 is a flow chart showing an example of a process in which the drug search unit 117 generates a list that includes information related to drugs that are related to mutations.

The drug search unit 117 searches the drug database 124 storing the data shown in FIG. 30, as to whether the related drug has been approved by an authority (FDA, PMDA, or the like). Specifically, for example, by using the information related to a mutation such as "mutation ID" as a key, the drug search unit 117 searches for "approval state" which indicates whether the related drug corresponding to the mutation has been approved by an authority, and "approved country" which indicates which country's authority has approved (step S15b).

On the basis of the search result, the drug search unit 117 generates a list that includes the mutation, the related drug corresponding to the mutation, information related to approval of the related drug, and the like (step S16b).

<Modification 2>

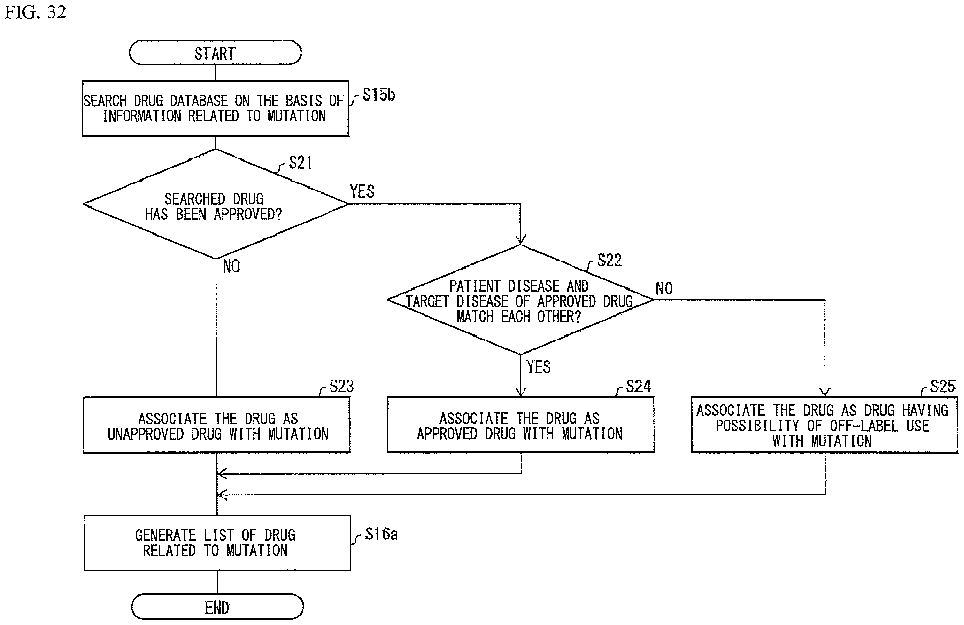

The drug search unit 117 may search the drug database 124 having the data structure shown in FIG. 30 and generate a list that includes still another type of information related to drugs that are related to mutations. Specifically, in addition to the list of drugs related to mutations that is generated in Embodiment 2, information of drugs corresponding to the disease of the subject is added. This is described below with reference to FIG. 32. FIG. 32 is a flow chart showing an example of a process in which, on the basis of information obtained by searching the drug database 124, the drug search unit 117 determines the presence or absence of a drug having a possibility of off-label use and generates a list that includes the determination result.

The drug search unit 117 searches the drug database 124 storing data 124B shown in FIG. 30, as to whether the related drug has been approved by an authority (FDA, PMDA, or the like) (step S15b). When the searched drug has not been approved (NO in step S21), the drug search unit 117 associates the drug, as an unapproved drug, with the mutation (step S23), and generates a report of drugs related to mutation (step S16a).

When the searched drug has been approved (YES in step S21), the drug search unit 117 determines whether the disease of the subject from whom the sample has been collected, and the disease (for example, "target disease" shown in FIG. 30) that corresponds to the related drug retrieved from the drug database 124 match each other (step S22).

When the disease of the subject and the "target disease" match each other (YES in step S22), the drug search unit 117 associates the drug of the search result, as an approved drug, with the mutation (step S24), and generates a list that includes the mutation, the related drug corresponding to the mutation, information related to the approval of the related drug, and the like (step S16a).

Meanwhile, when the disease of the subject and the "target disease" is different from each other (NO in step S22), the drug search unit 117 determines that the searched related drug is a drug having a possibility of off-label use, associates the determination result with the mutation (step S25), and generates a list that includes the mutation, the related drug corresponding to the mutation, information related to approval of the related drug, and the like (step S16a).

The information related to the disease of the subject can be inputted through the input unit 17 by an operator or the like when performing gene analysis, for example. In addition, for example, a header region of the read sequence information may include the disease ID which is identification information corresponding to the disease of the subject.

<Modification 3>

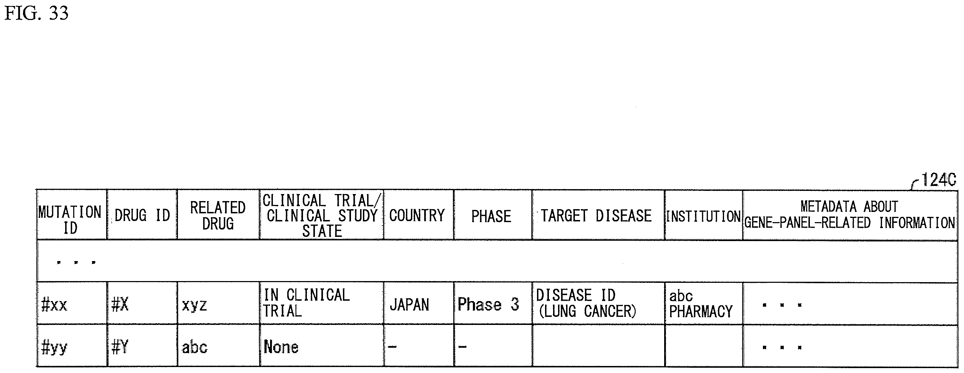

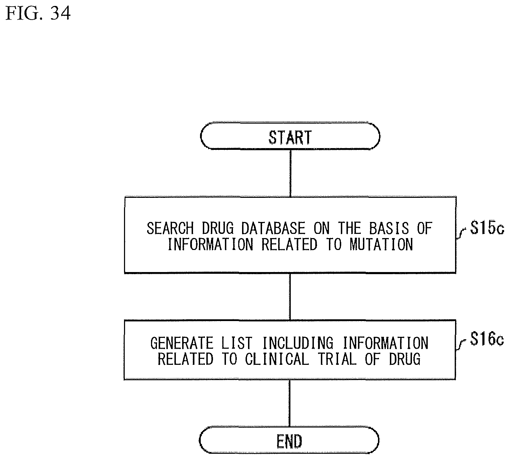

The drug search unit 117 may search the drug database 124 having the data structure shown in FIG. 33, and generate a list that includes information related to clinical trials of drugs that are related to mutations. Specifically, in addition to the list of drugs related to mutations that is generated in Embodiment 2, drug clinical trial information is added. This is described below with reference to FIG. 34. FIG. 34 is a flow chart showing an example of a process in which the drug search unit 117 generates a list that includes information related to clinical trials of drugs.

The drug search unit 117 searches the drug database 124 storing data 124C shown in FIG. 33, for information such as the progress of a clinical trial of a related drug, and the like. Specifically, using a mutation ID or the like as a key, the drug search unit 117 searches for information related to a clinical trial with respect to a mutation, such as, for example, "clinical trial/clinical study state", "country", and "institution" in which the clinical trial is being performed, as shown in FIG. 33 (step S15c in FIG. 34). On the basis of the search result, the drug search unit 117 generates a list that includes the mutation, the related drug corresponding to the mutation, and information related to the clinical trial of the related drug (step S16c in FIG. 34).

It should be noted that the data 124A shown in FIG. 29, the data 124B shown in FIG. 30, and the data 124C shown in FIG. 33 may be integrated together and stored in the drug database 124, or may be discretely stored in a plurality of databases including the drug database 124.

[Embodiment 3]

Another embodiment of the present invention is described below. For convenience of description, the members having the same functions as those of the members described in the above embodiments are denoted by the same reference characters, and description thereof is not repeated.

(Configuration of gene analysis apparatus 1b)

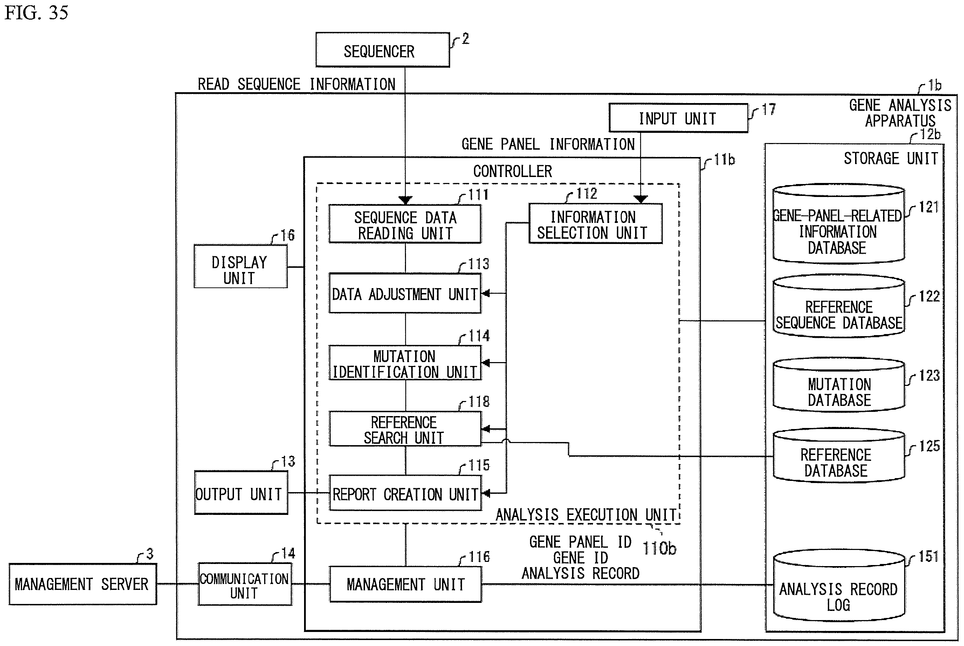

Here, a gene analysis apparatus 1b that can create a report including various types of reference information related to each mutation identified by the mutation identification unit 114 is described with reference to FIG. 35.

FIG. 35 shows an example of a configuration of the gene analysis apparatus 1b. The gene analysis apparatus 1b is different from the gene analysis apparatus 1 shown in FIG. 4 in that an analysis execution unit 1 of the gene analysis apparatus 1b further includes a reference search unit 118 and a first storage unit 12b further includes a reference database 125.

(Reference search unit 118)

Using the mutation ID provided to each mutation identified by the mutation identification unit 114 as a key, the reference search unit 118 searches the reference database 125. On the basis of the search result, the reference search unit 118 extracts reference information related to the mutation. The extracted reference information is incorporated into a report created by the report creation unit 115.

(Reference database 125)

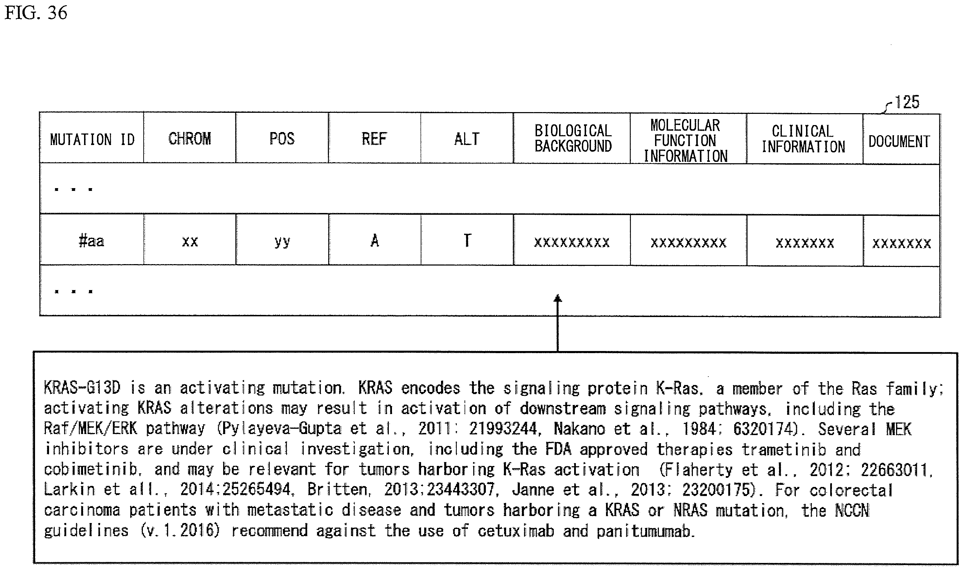

Data stored in the reference database 125 searched by the reference search unit 118 is described with reference to FIG. 36. FIG. 36 shows an example of a data structure of the reference database 125.

As shown in FIG. 36, a mutation ID, information related to biological background of the mutation, molecular function information, clinical information, document information such as books and scientific literature related to the mutation, and the like are stored in association with one another in the reference database 125.

Each of mutation ID in the reference database 125 may be provided with "metadata about gene-panel-related information" (not shown) which is metadata related to gene panel information. In this case, in accordance with an instruction from the information selection unit 112, the reference search unit 118 refers to the "metadata about gene-panel-related information" and changes the range in which the reference database 125 is searched, to a range indicated by the metadata. Accordingly, in accordance with the "metadata about gene-panel-related information" associated with each mutation and the inputted gene panel information, the reference search unit 118 can narrow the reference information that should be referred to in the drug database, and can extract reference information according to the gene panel information.

(Report creation unit 115 of gene analysis apparatus 1a, 1b)

The report creation unit 115 may create a report on the basis of information outputted by the drug search unit 117, or may create a report on the basis of information outputted by the reference search unit 118. Further, the report creation unit 115 may create a report on the basis of both of information outputted by the drug search unit 117 and information outputted by the reference search unit 118.

Information related to each identified mutation, information of a drug related to the mutation, reference related to the mutation (including, for example, molecular biological findings of the mutation, information related to documents, and the like), or information in which these types of information are combined as desired can be included in the report created by the report creation unit 115.

The information selection unit 112 performs control such that: for example, information related to each target gene that corresponds to the inputted gene panel information is selected as a target to be included in a report; and the report creation unit 115 creates a report in which the selected information is included.

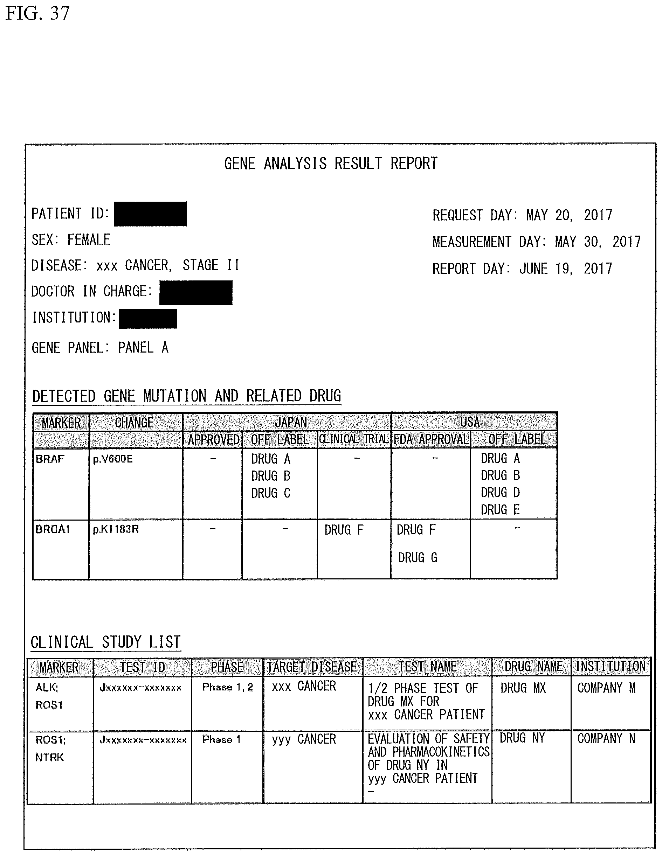

FIG. 37 shows an example of a report created by the report creation unit 115. In the upper left part of the report shown in this example, "patient ID" indicating the subject ID, "sex of patient", "name of disease of patient", "name of doctor in charge" which is the name of the doctor who is in charge of the subject in the medical institution 210, and "institution name" indicating the medical institution name are described. Further, a gene panel name "panel A" is also included as the gene panel information. In this report, the column "detected gene mutation and related drug" includes information related to mutations identified by the mutation identification unit 114 and a list generated on the basis of search results obtained by the drug search unit 117 searching the drug database 124.

The column "clinical study list" includes a list of information related to clinical trials of drugs generated on the basis of search results obtained by the drug search unit 117 searching the drug database 124.

[Embodiment 4]

Another embodiment of the present invention is described below. For convenience of description, the members having the same functions as those of the members described in the above embodiments are denoted by the same reference characters, and description thereof is not repeated.

(Configuration of gene analysis apparatus 1c)

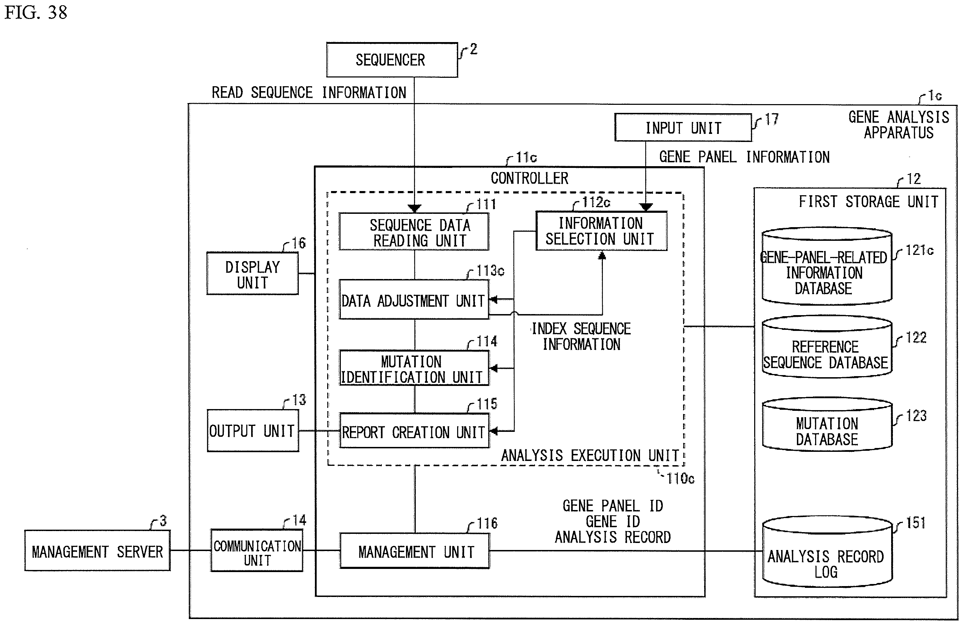

Here, a gene analysis apparatus 1c is described in which an information selection unit 112c also has a function of obtaining gene panel information on the basis of the index sequence included in the read sequence information, in addition to the function of allowing the user to input gene panel information. In the following, a gene-panel-related information database 121c, a data adjustment unit 113c, and the information selection unit 112c shown in FIG. 38 are described in particular with reference to FIG. 39.

FIG. 38 is a function block diagram showing an example of a configuration of the gene analysis apparatus 1c. The read sequence information read by the sequence data reading unit 111 may have inserted therein an index sequence for identifying read sequence information for each sample or each type of gene panel, for example.

The index sequence may be inserted only in a sequence of a specific gene among the analysis target genes of the gene panel. In the case of read sequence information having no index sequence inserted therein, the user may be caused to input gene panel information as shown in FIG. 6.

<Gene-panel-related information database 121c>

First, data 121D stored in the gene-panel-related information database 121c referred to by the information selection unit 112c is described with reference to FIG. 39. FIG. 39 shows an example of a data structure of the gene-panel-related information database 121c. The name of each selectable gene panel, the gene panel ID provided to the gene panel, and the index sequence information inserted for the gene panel are stored in association with one another in the gene-panel-related information database 121c.

The example in FIG. 39 shows data that indicates the following: read sequence information analyzed by use of a gene panel "panel A" having a gene panel ID "AAA" includes an index sequence "pppppppppp"; and read sequence information analyzed by use of a gene panel "panel B" having a gene panel ID "BBB" includes an index sequence "qqqqqqqqqq". "p" and "q" each indicate a base.

<Data adjustment unit 113c>

The data adjustment unit 113c analyzes read sequence information read by the sequence data reading unit 111, and determines whether or not the sequences include an index sequence "pppppppppp", "qqqqqqqqqq", or the like stored in the gene-panel-related information database 121c. When the index sequence is not included, the data adjustment unit 113c notifies the information selection unit 112c that the index sequence is not included. Meanwhile, when the index sequence is included, the data adjustment unit 113c outputs the detected index sequence (for example, "pppppppppp") to the information selection unit 112c.

<Information selection unit 112c>

When the information selection unit 112c has been notified by the data adjustment unit 113c that the index sequence is not included, the information selection unit 112c causes the display unit 16 to display the GUI shown in FIG. 6 together with a message such as "Please input gene panel information", or the like. Meanwhile, when the information selection unit 112c has received the index sequence from the data adjustment unit 113c, the information selection unit 112c searches the gene-panel-related information database 121c using the index sequence as a key, and specifies gene-panel-related information such as the gene panel name corresponding to the index sequence, the gene panel ID, and the like. For example, when the index sequence received from the data adjustment unit 113c is "qqqqqqqqqq", the information selection unit 112c searches the gene-panel-related information database 121c, identifies that "panel B" has been used as the gene panel, and obtains gene-panel-related information of the gene panel. As described above, the obtained gene-panel-related information is applied to controlling of the data adjustment unit 113c, the mutation identification unit 114, the report creation unit 115, and the like.

As described above, when an index sequence is inserted in the read sequence information, it is possible to specify gene-panel-related information without causing the user to input gene-panel-related information. Therefore, enhanced convenience can be provided to the user.

The present invention is not limited to the embodiments described above, and various modifications can be made without departing from the scope of the claims. Embodiments obtained by combining as appropriate technological means disclosed in different embodiments are also included in the technological scope of the present invention.

For example, one medical institution 210 and one test institution 120 are shown in FIG. 1, but the present invention is not limited thereto. That is, the medical institution 210 may request an analysis to a plurality of test institutions 120, and the test institution 120 may receive analysis requests from a plurality of medical institutions 210. That is, a plurality of medical institutions 210 and a plurality of test institutions 120 may be included.

In FIG. 1 and FIG. 2, the test institution 120 is provided with one sequencer 2 and one gene analysis apparatus 1. However, the present invention is not limited thereto. That is, the test institution 120 may be provided with a plurality of sequencers 2 and a plurality of gene analysis apparatuses 1.

The gene analysis system 100 can be suitably applied also to an institution that has the functions of both of the medical institution 210 and the test institution 120 (for example, research institutes that have both a clinical facility and a test facility, university hospitals, and the like). This is not limited to the gene analysis system 100. The gene analysis method performed by the gene analysis apparatus 1, a program for controlling the gene analysis apparatus 1 implemented by a computer that realizes the gene analysis method, and a computer readable storage medium having stored therein the program are also suitably applied to an institution that has functions of both of the medical institution 210 and the test institution 120.

The analysis using the gene panel may be used in analysis of polymorphism such as Single Nucleotide Polymorphism (SNP) and Copy Number Variation (CNV, Copy Number Polymorphism). The gene panel may be used for obtaining an output of information related to the amount of mutations in the entire genes that are analyzed (also referred to as Tumor Mutation Burden), or may be used for calculating the methylation frequency.

As means for allowing the user to input gene panel information, an example of displaying a GUI for inputting has been shown. However, the present invention is not limited thereto. For example, the input unit 17 may be a bar code reader that allows the user to read a bar code. In a case where a bar code is provided on, for example, a label of a container of each reagent of each gene panel and the surface of a box housing a set of reagents of the gene panel, if the bar code is read by use of the bar code reader, gene panel information is inputted.

When the controller 11 causes the display unit 16 to display a GUI for inputting gene panel information, the user may be caused to select an analysis target gene. In this case, as shown in FIG. 40, a list of genes as candidates may be displayed on the GUI, and the user may be caused to select an analysis target gene of the gene panel.

The gene names displayed on the GUI are based on the gene names of genes provided with gene IDs and registered in the gene-panel-related information database 121. The gene names on the list shown as alternatives are displayed on the basis of gene panel information registered in the gene-panel-related information database 121.

FIG. 40 shows an example in which a list including a plurality of gene names that can be analyzed (for example, "AKT1", "APC", and the like) is shown and check boxes are provided on the left side of the gene names. In the example shown in FIG. 40, the gene names "AKT1", "APC", etc., are selected, and the gene names "EML4", "JAK3", etc., are not selected. On the basis of the selected gene names, the information selection unit 112 specifies a gene panel ID associated with these gene names, and searches the gene-panel-related information database 121, to obtain gene panel information that corresponds to the inputted gene panel name.

Alternatively, as shown in FIG. 41, a list of gene panel names for respective diseases such as "lung cancer panel", "colon cancer panel", and the like may be displayed on a GUI, and the user may be allowed to select a gene panel related to a disease of interest out of the gene panels on the list. A list of disease names such as "lung cancer" and "colon cancer" may be displayed on a GUI, and the user may be allowed to select a disease of interest out of the disease names on the list.

In this case, on the basis of the selected disease name, the information selection unit 112 specifies a gene panel ID associated with the disease name, and searches the gene-panel-related information database 121, to obtain gene panel information that corresponds to the selected disease name.

The gene names displayed on a GUI as the alternatives that allow selection of a gene panel related to the selected disease are based on the information registered in the gene-panel-related information database 121.

The gene panel name of a gene panel related to a disease may be a reagent kit name. The gene panel includes a set of reagents such as various types of buffers, enzymes, and primers that are used in target sequencing which is performed by the sequencer 2 in order to read the sequences of the analysis target genes. The set of reagents is provided with a reagent kit name or a gene panel name.

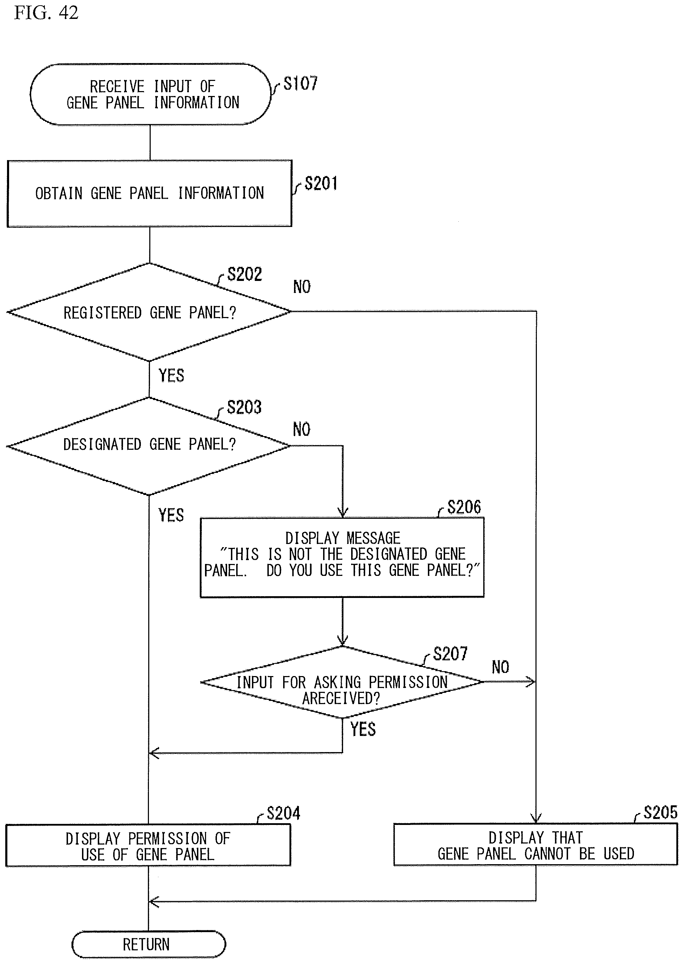

Here, another example of the flow of a process for receiving an input of gene panel information, shown in step S107 in FIG. 2, is described with reference to FIG. 42. For convenience of description, the processes that are the same as those described with reference to FIG. 5 are denoted by the same reference characters, and description thereof is not repeated.

The flow of a process shown in FIG. 5 assumes a case where, for example, in the test institution 120 that has received an analysis request from the medical institution 210, a panel test using a gene panel designated by the medical institution 210 is performed. However, not limited thereto, there could be a case where a gene panel other than the gene panel designated by the sample provision source is used to perform analysis. For example, in a research institution that searches for an optimum gene panel or that seeks for a more effective usage of a gene panel, there may be a case where after a sample is obtained from the medical institution 210, panel tests using various gene panels are performed in addition to an analysis using the designated gene panel.

When the gene panel that corresponds to the selected information does not match the gene panel included in the analysis request received from the medical institution 210 (NO in step S203), the information selection unit 112 causes the display unit 16 to display an indication that the inputted gene panel is different from the designated gene panel, and a message asking whether or not to use the inputted gene panel (step S206). When the information selection unit 112 has received an input for asking permission to use the inputted gene panel (YES in step S207), the information selection unit 112 receives the input. Then, the information selection unit 112 causes the display unit 16 to display a message to the effect that the inputted gene panel can be used (step S204).

Meanwhile, when the information selection unit 112 has received an input for asking permission to use the inputted gene panel (NO in step S207), the information selection unit 112 causes the display unit 16 to display a message to the effect that the inputted gene panel cannot be used (step S205), and prohibits the analysis from being performed by the gene analysis apparatus 1.

A configuration may be employed in which, when the gene analysis apparatus 1 receives an input of gene panel information, either the input mode shown in FIG. 5 or the input mode shown in FIG. 42 can be selected. For example, if a panel test is performed by use of the gene panel designated by the medical institution 210, the input mode shown in FIG. 5 is preferably selected. If an analysis is performed by use of a gene panel other than the designated gene panel, the input mode shown in FIG. 42 is preferably selected. Since a plurality of modes of the process for receiving an input of gene panel information are provided, the user who uses the gene analysis apparatus 1 can select an input mode in accordance with the usage.

[Embodiment 5]

Another embodiment of the present invention is described below. For convenience of description, the members having the same functions as those of the members described in the above embodiments are denoted by the same reference characters, and description thereof is not repeated.

In a gene analysis method according to the present embodiment, gene panel information is obtained, and on the basis of the obtained gene panel information, an analysis algorithm for evaluating the quality of a panel test is selected. Accordingly, when analysis target genes in various combinations are analyzed by use of various gene panels, appropriate quality control according to the gene panel can be performed.

Examples of the quality evaluation process selected in accordance with the gene panel include: (1) selecting the quality evaluation index to be used in quality evaluation of a panel test; (2) selecting the criterion to be used in determination as to whether a sufficient reliability is obtained when the same quality evaluation index is used; and (3) selecting the number of quality evaluation indexes to be used in quality evaluation of a panel test.

Examples of the quality evaluation index include indexes such as: the reading quality included in read sequence information outputted by the sequencer 2; the proportion of bases read by the sequencer 2, to bases included in a plurality of genes as analysis targets; the depth of reading of read sequence information; the variation of the depth of reading of read sequence information; and whether or not all of mutations of each standard gene included in a quality control sample have been detected.

(Configuration of gene analysis apparatus Id)

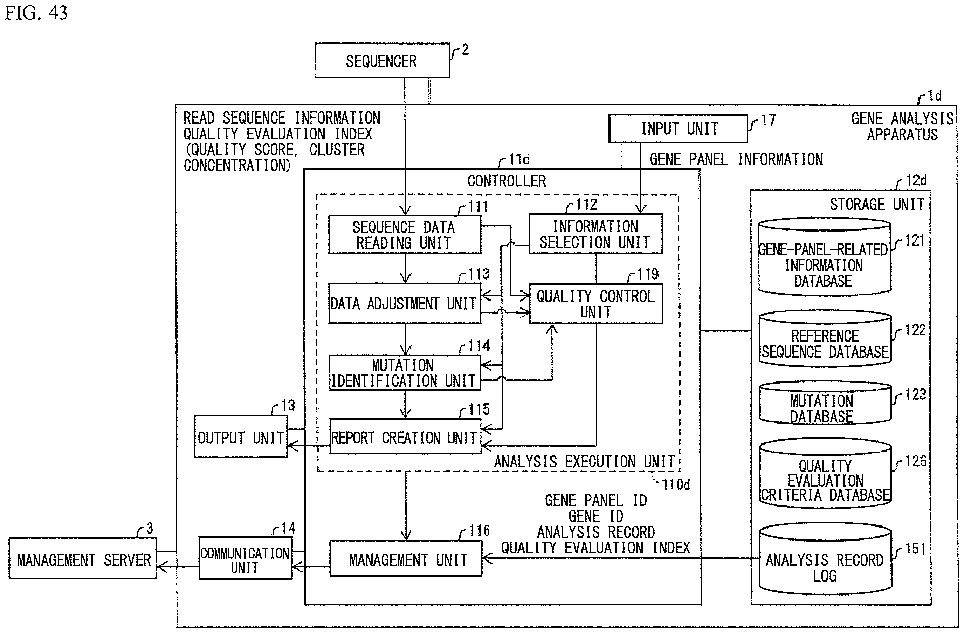

Here, a gene analysis apparatus 1d having a function of evaluating the quality of a panel test on the basis of a quality evaluation index is described with reference to FIG. 43. FIG. 43 shows an example of a configuration of the gene analysis apparatus 1d. The gene analysis apparatus 1d can create a report including an evaluation result of the quality of a panel test. In FIG. 43, the flows of data are indicated by arrows.

An analysis execution unit 110d of the gene analysis apparatus 1d is different from the gene analysis apparatus 1 shown in FIG. 4 in that the analysis execution unit 110d further includes a quality control unit 119, and a storage unit 12d further includes a quality evaluation criteria database 126.

The quality evaluation criteria database 126 stores criterion values which each specify whether or not the reliability of the analysis result in a panel test reaches a certain level. Here, the certain level is used in determining whether or not a reliability required for applying an analysis result of a panel test to a therapy or a diagnosis has been attained.

The information selection unit 112 according to the present embodiment selects the criterion value of the quality evaluation index on the basis of gene panel information inputted through the input unit 17.

(Quality evaluation index)

Examples of the quality evaluation index generated by the quality control unit 119 for a measurement include indexes such as: the reading quality included in read sequence information outputted by the sequencer 2; the proportion of bases read by the sequencer 2, to bases included in a plurality of genes as analysis targets; the depth of reading of read sequence information; the variation of the depth of reading of read sequence information; and whether or not all of mutations of each standard gene included in a quality control sample have been detected.

The examples of the quality evaluation index above are described in detail.

Quality evaluation index (1): quality score

The quality score is an index that indicates the correctness of each base in a gene sequence read by the sequencer 2.

For example, when read sequence information is outputted in a FASTQ file from the sequencer 2, the quality score is included in the read sequence information (see FIG. 17). Details of the quality score are described in Embodiment 1, and thus, description thereof is omitted here.

Quality evaluation index (2): cluster concentration

The cluster concentration is an index that indicates reading quality included in read sequence information outputted by the sequencer 2. The sequencer 2 locally amplifies and immobilizes a large number of single-stranded DNA fragments on a flow cell, to form clusters (see 9 in FIG. 14). Then, images of the cluster group on the flow cell are captured by use of a fluorescence microscope, and fluorescences having different wavelengths respectively corresponding to A, C, G, and T are detected, whereby each sequence is read. The cluster density is an index that indicates how close the clusters of genes formed on the flow cell are with one another.

For example, when the densities of clusters are excessively increased, and the clusters are excessively close to each other or overlap each other, the contrast, i.e., the S/N ratio, of the image of the flow cell is reduced, and the fluorescence microscope is less likely to focus. Thus, fluorescence cannot be accurately detected, and as a result, the sequence reading accuracy could be reduced.

Quality evaluation index (3): index that indicates the proportion of base sequences in the target region read by the sequencer 2, to base sequences read by the sequencer 2.

This index is an index that indicates how many bases in the target region have been read, among bases including bases in the region other than the target region read by the sequencer 2. The index is calculated as a ratio between the total number of bases that have been read and the total number of bases in the target region.

Quality evaluation index (4): index that indicates the reading depth of read sequence information.

This index is an index, with respect to each base included in a gene as an analysis target, that is based on the total number of read sequences in which the base has been read. The index is calculated as a ratio between the total number of bases, among bases having been read, that have depth greater than or equal to a predetermined value, and the total number of bases having been read.

The reading depth means the total number of pieces of read sequence information read with respect to the same base, and is also referred to as coverage, or depth of coverage.

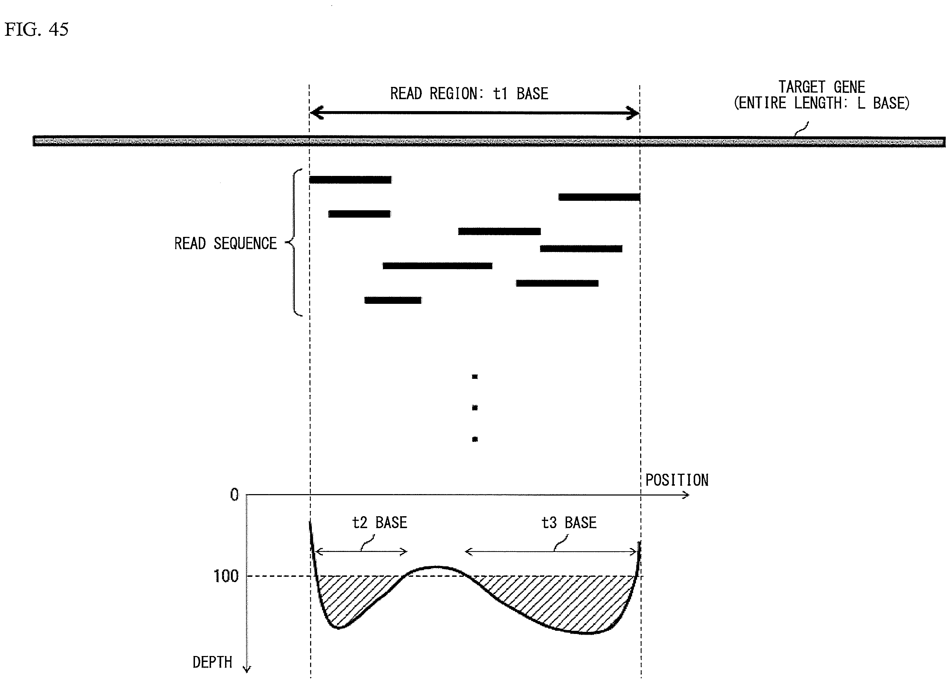

FIG. 45 shows a graph indicating the depth of each base in a case where L base represents the entire length of the analysis target gene ("target gene in FIG. 45), and t1 base represents the bases in the read region. In the graph in FIG. 45, the horizontal axis represents the position of each base, and the vertical axis represents the depth of each base. In the example shown in FIG. 45, the total number of bases in the region in which the depth is greater than or equal to a predetermined value (for example, 100), in the t1 base in the region having been read, is (t2+t3) bases. In this case, the quality evaluation index (4) is generated as a value of (t2+t3)/t1.

Quality evaluation index (5): index that indicates the variation of reading depth of read sequence information.

This index is an index that indicates uniformity of the depth. When the number of pieces of read sequence information having been read in a certain portion in the region having been read is extremely great, uniformity of the depth is low. Meanwhile, when pieces of read sequence information are evenly present over the entirety of the region having been read, the uniformity of the depth is high. For example, the uniformity of the depth can be represented as numbers by use of the interquartile range (IQR). The greater the IQR is, the lower the uniformity is. The less the IQR is, the higher the uniformity is.

Quality evaluation index (6): index that indicates whether or not all the mutations in each standard gene included in the quality control sample have been detected.

This index is an index indicating that the mutation in each standard gene included in the quality control sample has been detected and accurately identified when the quality control sample and a sample collected from a subject have been measured. For example, whether or not the position of a known mutation in each standard gene included in the quality control sample, the type of the mutation, and the like have been accurately identified, is used as the quality evaluation index. The quality control sample is prepared by mixing a plurality of standard genes.

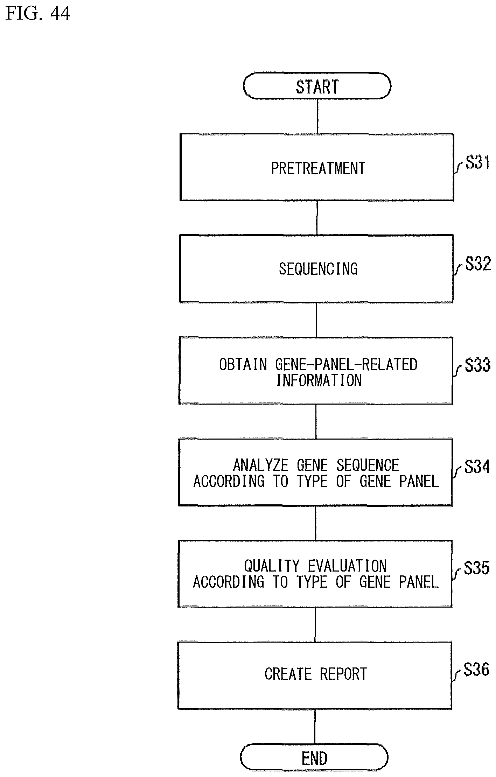

The flow of a process of performing quality evaluation of a panel test is described with reference to FIG. 44. FIG. 44 is a flow chart showing an example of the flow of a process for analyzing a gene sequence.

First, in step S31 in FIG. 44, pretreatment for analyzing a gene sequence is performed. The pretreatment includes processes from fragmentation of genes such as DNA contained in a sample to collection of the fragmented genes. Here, the analysis target in the panel test to be subjected to quality evaluation may be a sample collected from a subject, or may be a quality control sample prepared by mixing a plurality of standard genes.

The quality control sample includes at least two of: a standard gene including SNV, a standard gene including Insertion, a standard gene including Deletion, a standard gene including CNV, and a standard gene including Fusion. For example, the quality control sample includes, as standard genes, a partial sequence of gene A including "SNV" with respect to the wild type and a partial sequence of gene B including "Insertion" with respect to the wild type.

Next, in step S32, the sequencer 2 reads base sequences of DNA contained in the pretreated sample.

Subsequently, in step S33, a controller 11d of the gene analysis apparatus 1d causes the input unit 17 to display a GUI for allowing the user to select gene panel information. On the basis of the input operation on the GUI by the user, the gene panel information is obtained. The gene panel information may not necessarily be obtained through an input on the GUI by the user. For example, the gene panel information may be obtained by use of an identifier such as a bar code attached to the gene panel, or may be identified by reading an index sequence.

The controller 11d of the gene analysis apparatus 1d determines the type of the gene panel on the basis of the obtained gene panel information. The gene analysis apparatus 1d selects an analysis algorithm so as to perform quality control of the panel test in accordance with the obtained type of the gene panel.

In S34, the gene analysis apparatus 1d analyzes a gene sequence in accordance with the type of the gene panel, and identifies the presence or absence of a mutation in the base sequence, the position of a mutation, the type of the mutation, and the like. Through the analysis of the read gene sequence, the detected mutation is identified.

(Quality control unit 119)

The gene analysis apparatus 1d evaluates the quality of the panel test on the basis of the generated quality evaluation index. The quality control unit 119 obtains the quality score (quality evaluation index 1) and the cluster concentration (quality evaluation index 2) from the sequence data reading unit 111. In addition, the quality control unit 119 obtains the proportion (quality evaluation index 3) of the bases in the target region read by the sequencer 2, the reading depth of the read sequence information (quality evaluation index 4), and the variation of the reading depth of the read sequence information (quality evaluation index 5), from the data adjustment unit 113. Further, the quality control unit 119 obtains whether or not all the mutations in each standard gene included in the quality control sample have been detected (quality evaluation index 6), from the mutation identification unit 114. The quality control unit 119 need not obtain all of the quality evaluation indexes, and may obtain one or a plurality of desired indexes.

The quality control unit 119 compares the obtained quality evaluation index with the criterion value of the quality evaluation index stored in the quality evaluation criteria database 126, and determines whether the analysis result has sufficient reliability. Here, in the quality evaluation criteria database 126, each criterion value of a corresponding quality evaluation index is stored in association with information that specifies a gene panel.

For example, when the type of the gene panel is panel A in S35, determination is performed by use of a criterion value a with respect to a quality evaluation index A, and determination is performed by use of a criterion value b with respect to a quality evaluation index B. Meanwhile, when the type of the gene panel is panel B in S35, determination is performed by use of a criterion value c with respect to the quality evaluation index A, and determination is performed by use of the criterion value b with respect to the quality evaluation index B. In this manner, in the analysis of panel A and the analysis of panel B, the same quality control index A is used, whereas different criteria are used for the evaluations. In the analysis of panel A, the quality control indexes A and B are used, whereas in the analysis of panel B, the quality control indexes A and C are used, and quality control indexes different from each other are used.

When the type of the gene panel is panel C in S35, determination is performed by use of a criterion value e with respect to a quality evaluation index D. In this manner, in the analysis of panel A, quality evaluation is performed on the basis of two indexes, i.e., the quality evaluation indexes A and B, but in the analysis of panel C, quality evaluation is performed by use of only the quality evaluation index D. In this manner, the number of quality evaluation indexes to be used may be changed in accordance with the gene panel.

Lastly, in S36, the gene analysis apparatus 1d creates a report that includes the identified mutation and the evaluation result of the quality of the panel test determined in step S34.

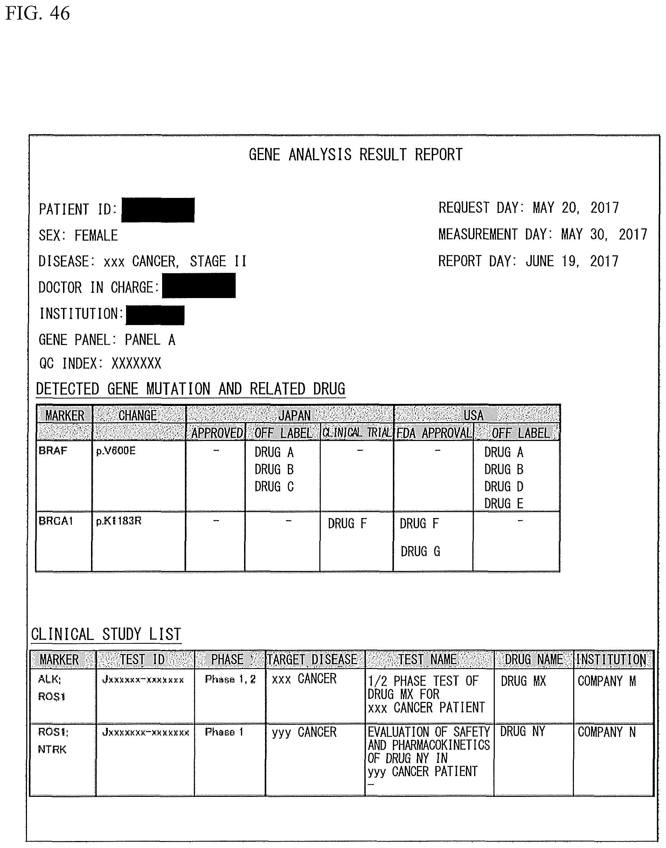

FIG. 46 shows an example of a report created by the report creation unit 115. In the upper left part of the report shown in this example, "patient ID" indicating the subject ID, "sex of patient", "name of disease of patient", "name of doctor in charge" which is the name of the doctor who is in charge of the subject in the medical institution 210, and "institution name" indicating the medical institution name are described.

Below these items, the gene panel name "panel A" is also included as gene panel information. Further, the quality evaluation index "QC index", which is information related to the quality of the panel test, is outputted in the report.

When the quality evaluation index is less than a predetermined criterion, the detected gene mutation may be marked with *. In addition, or instead, a comment for indicating that the reliability is low can be added.

The present invention is not limited to the above-described embodiments. Various modifications can be made without departing from the scope of claims. Embodiments obtained by combining as appropriate technological means disclosed in different embodiments are also included in the technical scope of the present disclosure.

DESCRIPTION OF THE REFERENCE CHARACTERS

| | |
|---|---|
| 1, 1a, 1b, 1c, 1d | gene analysis apparatus |
| 2 | sequencer |
| 3 | management server |
| 4 | network |
| 11, 11a, 11b, 11c, 11d | controller |
| 13 | output unit |
| 16 | display unit |
| 100 | gene analysis system |
| 121, 121c | gene-panel-related information database |
| 122 | reference sequence database |
| 124 | drug database |
| 125 | reference database |

**Claims**

1. A gene analysis method for analyzing gene sequence information, the gene analysis method comprising:

   obtaining read sequence information read by a sequencer and gene panel information related to a gene panel including a plurality of genes to be analyzed; and
   outputting an analysis result of the read sequence information on the basis of the obtained gene panel information.

2. The gene analysis method of claim 1, comprising
   selecting, on the basis of the obtained gene panel information, a gene for which the analysis result is to be outputted.

3. The gene analysis method of claim 1 or 2, comprising
   selecting, on the basis of the obtained gene panel information, an analysis algorithm for analyzing a gene for which the analysis result is to be outputted.

4. The gene analysis method of any one of claims 1 to 3, comprising
   displaying, on a display unit, an input screen for allowing the gene panel information to be inputted.

5. The gene analysis method of any one of claims 1 to 4, comprising
   displaying, on a display unit, an input screen for allowing at least one piece of information to be selected from a plurality of pieces of the gene panel information.

6. The gene analysis method of any one of claims 1 to 5, comprising
displaying, on a display unit, an input screen for allowing a reagent kit name to be inputted as the gene panel information.

7. The gene analysis method of any one of claims 1 to 6, comprising
displaying, on a display unit, an input screen for allowing names of a plurality of genes to be analyzed, to be inputted as the gene panel information.

8. The gene analysis method of any one of claims 1 to 7, comprising
displaying, on a display unit, an input screen for allowing a name of a disease to be analyzed, to be inputted as the gene panel information.

9. The gene analysis method of any one of claims 1 to 8, comprising:

   selecting, on the basis of the obtained gene panel information, reference sequence information with which the read sequence information should be compared; and
   outputting the analysis result based on comparison between the read sequence information and the selected reference sequence information.

10. The gene analysis method of any one of claims 1 to 9, comprising:

    on the basis of the obtained gene panel information, selecting, from a plurality of pieces of reference sequence information each including a mutation sequence, reference sequence information with which comparison is to be performed in order to specify a mutation included in the read sequence information; and
    outputting the analysis result based on the selected reference sequence information.

11. The gene analysis method of any one of claims 1 to 10, comprising
outputting the analysis result of the read sequence information, using a gene-panel-related information database which stores, for each gene panel, information related to an analysis target gene of the gene panel.

12. The gene analysis method of any one of claims 1 to 11, comprising
reading a selected reference sequence from a reference sequence database, and mapping the read sequence information with respect to the read reference sequence, to perform alignment.

13. The gene analysis method of any one of claims 1 to 12, comprising
reading a selected reference sequence from a reference sequence database, determining a position of the read sequence information on the basis of a degree of matching between the reference sequence and the read sequence information, and identifying a mutation included in the read sequence information.

14. The gene analysis method of any one of claims 1 to 13, comprising
outputting an analysis result that includes information related to a mutation associated with the obtained gene panel information, among mutations identified through analysis of the read sequence information.

15. The gene analysis method of any one of claims 1 to 14, comprising
on the basis of the obtained gene panel information, outputting drug information related to a mutation identified through analysis of the read sequence information, as the analysis result of the read sequence information.

16. The gene analysis method of any one of claims 1 to 15, comprising
on the basis of a mutation identified through analysis of the read sequence information, searching a drug database which stores a mutation of an analysis target gene and a drug related to the gene panel in association with each other.

17. The gene analysis method of claim 16, comprising
generating a list of a drug related to the mutation identified through the analysis of the read sequence information and extracted through the search of the drug database.

18. The gene analysis method of any one of claims 1 to 17, comprising

outputting, as the analysis result of the read sequence information, drug information including an approval state of a drug.

19. The gene analysis method of any one of claims 1 to 18, comprising
on the basis of a mutation identified through analysis of the read sequence information, searching a reference database which stores a mutation of an analysis target gene and reference information related to the mutation in association with each other.

20. The gene analysis method of any one of claims 1 to 19, comprising
creating a report on the basis of the analysis result of the read sequence information, wherein
the report includes information related to a mutation that corresponds to the obtained information related to the gene panel among mutations identified through analysis of the read sequence information.

21. The gene analysis method of any one of claims 1 to 20, comprising
on the basis of the gene panel information, selecting a mutation that corresponds to the obtained gene panel information from all identified mutations, and outputting information related to the selected mutation, as the analysis result of the read sequence information.

22. The gene analysis method of claim 20, wherein
the report includes information related to the gene panel.

23. The gene analysis method of claim 20 or 22, wherein
the report includes at least one of a list of a drug and reference information.

24. The gene analysis method of any one of claims 1 to 23, comprising
transmitting, to a management server, information related to an analysis state of the gene sequence information.

25. The gene analysis method of any one of claims 1 to 24, comprising
transmitting, for each piece of the gene panel information to a management server, information related to an analysis state of the gene sequence information.

26. The gene analysis method of any one of claims 1 to 25, comprising
transmitting, for each piece of the gene panel information to a management server, the number of times of sequence analysis of the genes.

27. The gene analysis method of any one of claims 1 to 26, comprising
transmitting, for each piece of the gene panel information to a management server, the number of the genes having being analyzed.

28. The gene analysis method of any one of claims 1 to 27, comprising
transmitting, for each piece of the gene panel information to a management server, information related to an amount of data having been processed in sequence analysis of the genes.

29. The gene analysis method of any one of claims 1 to 28, comprising
outputting, as the analysis result of the read sequence information, a comparison result obtained by comparing the read sequence information with sequence information of an analysis target gene of the gene panel associated with the obtained gene panel information.

30. The gene analysis method of any one of claims 1 to 29, comprising
displaying an error when the obtained gene panel information does not match gene panel information that has been registered.

31. The gene analysis method of any one of claims 1 to 30, comprising
displaying an error when the obtained gene panel information does not match gene panel information that has been designated by a medical institution.

32. The gene analysis method of claim 30 or 31, comprising
when an input that asks permission of use of a gene panel inputted by a user is made after the error has been displayed, permitting analysis that uses the gene panel.

33. The gene analysis method of any one of claims 1 to 32, comprising
when the obtained gene panel information does not match gene panel information that has been registered, prohibiting analysis that uses the gene panel.

34. The gene analysis method of any one of claims 1 to 33, comprising
when the obtained gene panel information does not match gene panel information that has been designated by a medical institution, prohibiting analysis that uses the gene panel.

35. The gene analysis method of any one of claims 1 to 34, wherein
the obtaining of the gene panel information has a plurality of modes, and one of the plurality of modes is selectable.

36. The gene analysis method of any one of claims 1 to 35, comprising
displaying an error when pieces of the read sequence information include not less than a predetermined number of pieces of the read sequence information that include sequences of genes that are not analysis target genes of the gene panel indicated by the obtained gene panel information.

37. The gene analysis method of any one of claims 1 to 36, wherein
the read sequence information includes an index sequence associated with the gene panel information.

38. The gene analysis method of claim 37, wherein
the index sequence is different for each piece of gene panel information.

39. The gene analysis method of claim 38, comprising
displaying an error when gene panel information associated with the index sequence included in the read sequence information is different from the obtained gene panel information.

40. The gene analysis method of any one of claims 1 to 39, comprising:

analyzing, with respect to a first sample, first read sequence information read by use of a first gene panel for analyzing a first analysis target gene group;
analyzing, with respect to a second sample, second read sequence information read by use of a second gene panel for analyzing a second analysis target gene group;
receiving selection of information that specifies the gene panel, to obtain gene panel information; and
outputting, on the basis of the selected gene panel information, an analysis result obtained by analyzing the first read sequence information and an analysis result obtained by analyzing the second read sequence information.

41. The gene analysis method of any one of claims 1 to 40, further comprising
evaluating a quality of a gene panel test, wherein
an evaluation result of the quality is outputted on the basis of the obtained gene panel information.

42. The gene analysis method of claim 41, wherein
the evaluating of the quality of the gene panel test comprises selecting, on the basis of the obtained gene panel information, a quality control index to be used when evaluating the quality.

43. The gene analysis method of claim 41 or 42, wherein
the evaluating of the quality of the gene panel test comprises selecting, on the basis of the obtained gene panel information, an evaluation criterion for a quality control index to be used when evaluating the quality.

44. The gene analysis method of claim 41 or 42, wherein
the evaluating of the quality of the gene panel test comprises selecting, on the obtained gene panel information, the number of quality control indexes to be used when evaluating the quality.

45. A gene analysis apparatus configured to analyze gene sequence information, the gene analysis apparatus comprising:

a controller configured to obtain read sequence information read by a sequencer and gene panel information related to a gene panel including a plurality of genes to be analyzed; and
an output unit, wherein
the controller outputs, to the output unit, an analysis result of the read sequence information on the basis of the obtained gene panel information.

46. The gene analysis apparatus of claim 45, wherein
the controller

selects, on the basis of the obtained gene panel information, reference sequence information with which the read sequence information should be compared, and
outputs, to the output unit, the analysis result based on comparison between the read sequence information and the selected reference sequence information.

47. The gene analysis apparatus of claim 45 or 46, wherein
the controller outputs, to the output unit, an analysis result that includes information related to a mutation associated with the obtained gene panel information, among mutations identified through analysis of the read sequence information.

48. The gene analysis apparatus of any one of claims 45 to 47 wherein
on the basis of the obtained gene panel information, the controller outputs, to the output unit, drug information related to a mutation identified through analysis of the read sequence information, as the analysis result of the read sequence information.

49. The gene analysis apparatus of claim 42, wherein
on the basis of the obtained gene panel information, the controller outputs an evaluation result of a quality of a gene panel test to the output unit.

50. A management server configured to receive, from a gene analysis apparatus, information that includes information for specifying a user who performs analysis of a sequence of a gene, gene panel information related to a gene panel having been used, and information related to an analysis state of sequence information.

51. The management server of claim 50, wherein
the management server receives, from the gene analysis apparatus, information related to an analysis state of sequence information of the gene.

52. The management server of claim 50 or 51, wherein
for each piece of the gene panel information, the management server receives, from the gene analysis apparatus, information related to an analysis state of sequence information of the gene.

53. The management server of any one of claims 50 to 52, wherein
for each piece of the gene panel information, the management server receives, from the gene analysis apparatus, the number of times of the analysis of the sequence of the gene.

54. The management server of any one of claims 50 to 53, wherein
for each piece of the gene panel information, the management server receives, from the gene analysis apparatus, the number of the genes having been analyzed.

55. The management server of any one of claims 50 to 54, wherein
for each piece of the gene panel information, the management server receives, from the gene analysis apparatus, information related to an amount of data having been processed in the analysis of the sequence of the gene.

56. The management server of any one of claims 50 to 55, wherein
on the basis of information related to an analysis state of sequence information of the gene, the management server calculates a consideration for a case where the user has performed analysis of a sequence using the gene analysis apparatus.

57. The management server of any one of claims 50 to 56, wherein

the management server receives, from the gene analysis apparatus, an update request for the gene panel information.

**58.** A gene analysis system comprising:

a gene analysis apparatus including

a controller configured to obtain read sequence information read by a sequencer and gene panel information related to a gene panel including a plurality of genes to be analyzed, and
an output unit configured to output an analysis result of the read sequence information based on the gene panel information obtained by the controller; and

a management server configured to receive, from the gene analysis apparatus, information that includes information for specifying a user who performs analysis of a sequence of a gene, gene panel information related to a gene panel having been used, and information related to an analysis state of the sequence of the gene.

**59.** The gene analysis system of claim 58, wherein
a consideration for a case where the user has performed analysis of a sequence using the gene analysis apparatus is calculated on the basis of information related to an analysis state of sequence information of the gene.

**60.** A program configured to analyze gene sequence information, the program causing a computer to execute:

obtaining read sequence information read by a sequencer and gene panel information related to a gene panel including a plurality of genes to be analyzed; and
outputting an analysis result of the read sequence information on the basis of the obtained gene panel information.

**61.** A computer readable storage medium having stored therein the program of claim 60.

**62.** A gene analysis method for analyzing gene sequence information in which an analysis result of a read sequence information read by a sequencer is obtained on the basis of obtained gene panel information including a plurality of genes to be analyzed, the gene analysis method comprising:
displaying an error when the obtained gene panel information does not match gene panel information that has been registered.

**63.** A gene analysis method for analyzing gene sequence information in which an analysis result of a read sequence information read by a sequencer is obtained on the basis of obtained gene panel information including a plurality of genes to be analyzed , the gene analysis method comprising:
displaying an error when the obtained gene panel information does not match gene panel information that has been designated by a medical institution.

FIG. 1

4 TEST INSTITUTION ID, GENE PANEL ID,
GENE ID, ANALYSIS RECORD

130

TEST INSTITUTION ID, GENE PANEL ID,
GENE ID, ANALYSIS RECORD

3
MANAGEMENT
SERVER

ANALYSIS SYSTEM
MANAGEMENT INSTITUTION

ANALYSIS REQUEST
210 (GENE PANEL INFORMATION) 120

MEDICAL
INSTITUTION | SAMPLE
SAMPLE ID | TEST
INSTITUTION

5
COMMUNICATION
TERMINAL

100

GENE ANALYSIS
APPARATUS

1

SAMPLE ID
READ SEQUENCE
INFORMATION

2

SEQUENCER

SAMPLE ID
REPORT

37

FIG. 2

EP 3 702 473 A1

FIG. 3

<r3>

**3A**

| TEST INSTITUTION NAME | TEST INSTITUTION ID |
|---|---|
| INSTITUTION P | ppppp |
| INSTITUTION Q | qqqqq |
| INSTITUTION R | rrrrr |
| ... | |

**3C**

| GENE-PANEL-RELATED INFORMATION | THE NUMBER OF TIMES OF OPERATION | PERIOD |
|---|---|---|
| PANEL ID: AAA | 5 | 2017/8/1 - 2017/8/31 |
| PANEL ID: BBB | 10 | 2017/8/1 - 2017/8/31 |
| PANEL ID: CCC | 0 | 2017/8/1 - 2017/8/31 |
| ... | ... | ... |

**3D**

| GENE-PANEL-RELATED INFORMATION | THE NUMBER OF ANALYZED GENE | PERIOD |
|---|---|---|
| PANEL ID: AAA | 4178 | 2017/8/1 - 2017/8/31 |
| PANEL ID: BBB | 2734 | 2017/8/1 - 2017/8/31 |
| PANEL ID: CCC | 153 | 2017/8/1 - 2017/8/31 |
| ... | ... | ... |

**3E**

| GENE-PANEL-RELATED INFORMATION | TOTAL NUMBER OF IDENTIFIED MUTATIONS | PERIOD |
|---|---|---|
| PANEL ID: AAA | 2610 | 2017/8/1 - 2017/8/31 |
| PANEL ID: BBB | 518 | 2017/8/1 - 2017/8/31 |
| PANEL ID: CCC | 94 | 2017/8/1 - 2017/8/31 |
| ... | ... | ... |

**3F**

| NAME OF COMPANY THAT PROVIDES GENE PANEL | PANEL ID | WHETHER OR NOT SYSTEM USAGE FEE IS REQUIRED |
|---|---|---|
| COMPANY A | AAA, ... | NOT REQUIRED |
| COMPANY B | BBB, ... | REQUIRED |
| COMPANY C | CCC, ... | REQUIRED |
| ... | ... | ... |

**3B**

| CONTRACT TYPE | USABLE GENE PANEL | SYSTEM USAGE FEE |
|---|---|---|
| PLAN 1 | PANEL A PANEL B PANEL C ... | DETERMINE ACCORDING TO THE NUMBER OF TIMES OF OPERATION |
| PLAN 2 | PANEL A PANEL B PANEL C ... | DETERMINE ACCORDING TO THE NUMBER OF ANALYZED GENES |
| PLAN 3 | PANEL A PANEL B PANEL C ... | DETERMINE ACCORDING TO THE NUMBER OF TIMES OF OPERATION |
| PLAN 4 | PANEL A PANEL D ... | DETERMINE ACCORDING TO THE NUMBER OF TIMES OF OPERATION |
| ... | ... | |

FIG. 4

SEQUENCER ⌐2

READ SEQUENCE INFORMATION

GENE ANALYSIS APPARATUS ⌐1

INPUT UNIT ⌐17

GENE PANEL INFORMATION

CONTROLLER ⌐11

STORAGE UNIT ⌐12

SEQUENCE DATA READING UNIT ⌐111

INFORMATION SELECTION UNIT ⌐112

GENE-PANEL-RELATED INFORMATION DATABASE ⌐121

DISPLAY UNIT ⌐16

DATA ADJUSTMENT UNIT ⌐113

REFERENCE SEQUENCE DATABASE ⌐122

MUTATION IDENTIFICATION UNIT ⌐114

OUTPUT UNIT ⌐13

REPORT CREATION UNIT ⌐115

MUTATION DATABASE ⌐123

ANALYSIS EXECUTION UNIT ⌐110

GENE PANEL ID
GENE ID
ANALYSIS RECORD

MANAGEMENT SERVER ⌐3

COMMUNICATION UNIT ⌐14

MANAGEMENT UNIT ⌐116

ANALYSIS RECORD LOG ⌐151

EP 3 702 473 A1

FIG. 5

```
        ╭─────────────────────────╮
        │   RECEIVE INPUT OF      │╱ S107
        │ GENE PANEL INFORMATION  │
        ╰─────────────────────────╯
                    │
        ┌─────────────────────────┐
        │ OBTAIN GENE PANEL INFORMATION │╱ S201
        └─────────────────────────┘
                    │
                   ╱╲  ╱ S202
                  ╱  ╲
                 ╱    ╲          NO
        ╱ REGISTERED GENE PANEL? ╲──────────┐
                 ╲    ╱                      │
                  ╲  ╱                       │
                   ╲╱                        │
                  YES                        │
                    │                        │
                   ╱╲  ╱ S203                │
                  ╱  ╲                        │
                 ╱    ╲          NO           │
        ╱ DESIGNATED GENE PANEL? ╲───────────┼──►
                 ╲    ╱                       │
                  ╲  ╱                        │
                   ╲╱                         │
                  YES                         │
                    │                         │
        ┌──────────────────┐ ╱S204   ┌──────────────────────┐ ╱S205
        │ DISPLAY PERMISSION OF │    │    DISPLAY THAT       │
        │  USE OF GENE PANEL    │    │ GENE PANEL CANNOT BE USED │
        └──────────────────┘        └──────────────────────┘
                    │                         │
                    │◄────────────────────────┘
                    │
            ╭──────────────╮
            │    RETURN     │
            ╰──────────────╯
```

FIG. 6

GENE PANEL 1 (GENE PANEL NAME: "xxxxx")

GENE PANEL 2 (GENE PANEL NAME: "yyyyy")

GENE PANEL 3 (GENE PANEL NAME: "zzzzzz")

UNREGISTERED PANEL

FIG. 7

121

121A

| GENE NAME | GENE ID |
|---|---|
| . . . | . . . |
| EGFR | aaa |
| BRAF | bbb |
| Ros1 | ccc |
| . . . | . . . |

121B

| GENE PANEL NAME | GENE PANEL ID | RELATED GENE ID |
|---|---|---|
| . . . | . . . | . . . |
| PANEL A | AAA | aaa, bbb, ccc, · · · |
| PANEL B | BBB | aaa, bbb, ccc, ddd, · · · |
| PANEL C | CCC | aaa, ccc, · · · |
| . . . | . . . | . . . |

121C

| GENE PANEL NAME | GENE |
|---|---|
| LUNG CANCER PANEL | EGFR, ALK, ROS1, · · · |
| COLON CANCER PANEL | RAS, APC, · · · |
| BREAST CANCER PANEL | BRCA1, BRCA2, · · · |
| . . . | . . . |

FIG. 8

(a)

REGISTRATION FILE NAME

| GENE PANEL TARGET GENE.csv | REGISTER |

| RET |
| CHEK2 |
| PTEN |
| MEK1 |

(b)

GENE NAME

| FBXW7 | REGISTER |

## FIG. 9

```
                    ( SEQUENCING )──S108

      ┌─────────────────────────────────────── PRETREATMENT ┐

           ┌─────────────────────────────┐
           │     FRAGMENT SAMPLE DNA      │──S301
           └─────────────────────────────┘

           ┌─────────────────────────────┐
           │   PROVIDE ADAPTER SEQUENCE   │──S302
           └─────────────────────────────┘

           ┌─────────────────────────────┐
           │          HYBRIDIZE           │──S303
           └─────────────────────────────┘

           ┌─────────────────────────────┐
           │     COLLECT DNA FRAGMENT     │──S304
           └─────────────────────────────┘

      └──────────────────────────────────────────────────────┘

      ┌─────────────────────────────────────── MEASUREMENT ┐

           ┌─────────────────────────────┐
           │       APPLY TO FLOW CELL     │──S305
           └─────────────────────────────┘

           ┌───────────────────────────────────┐
           │ AMPLIFY DNA FRAGMENT TO BE ANALYZED│──S306
           └───────────────────────────────────┘

           ┌─────────────────────────────┐
           │        READ SEQUENCE         │──S307
           └─────────────────────────────┘

      └──────────────────────────────────────────────────────┘

                    ( RETURN )
```

FIG. 10

(a) FRAGMENTATION OF SAMPLE (DNA)

SAMPLE DNA

FRAGMENTATION

(b) PROVISION OF ADAPTER SEQUENCE

DNA FRAGMENT

ADAPTER 1 SEQUENCE          ADAPTER 2 SEQUENCE

DNA FRAGMENT

ADAPTER 1 SEQUENCE          ADAPTER 2 SEQUENCE

INDEX SEQUENCE

FIG. 11

HYBRIDIZATION

HYBRIDIZE

BIOTINYLATED RNA TO BE HYBRIDIZED
WITH GENE (DNA) TO BE ANALYZED

BIOTIN

RNA

FIG. 12

FIG. 13

APPLICATION TO FLOW CELL

MAGNET

COLLECT

APPLY TO FLOW CELL

MAGNET

EP 3 702 473 A1

FIG. 14

EP 3 702 473 A1

FIG. 15

3'

SEQUENCING
DIRECTION

5'

DNA
POLYMERASE

3' POSITION BLOCKED FLUORESCENT
DEOXYNUCLEOTIDE (dNTPs)

P

Base

FLUORESCENT
SUBSTANCE

SUBSTANCE BLOCKING
3' POSITION

SEQUENCE PRIMER

REMOVE POLYMERASE

1 BASE ELONGATION

SEPARATE FLUORESCENT
SUBSTANCE AND  SUBSTANCE
BLOCKING 3' POSITION AND
GO ONTO NEXT POLYMERASE
REACTION

FLUORESCENCE DETECTION
AND IMAGE ANALYSIS

FLUORESCENCE

TAKE IMAGE

LASER LIGHT

FIG. 16

GENE SEQUENCE ANALYSIS  S109

READ READ SEQUENCE DATA  S11

PERFORM ALIGNMENT  S12

COMPARE ALIGNMENT SEQUENCE OF
BLOOD SAMPLE WITH ALIGNMENT SEQUENCE OF
TUMOR SAMPLE  S13

EXTRACT DIFFERENCE
BETWEEN SEQUENCES AS MUTATION  S14

SEARCH MUTATION DATABASE
ON THE BASIS OF EXTRACTED MUTATION  S15

PROVIDE ANNOTATION TO
EXTRACTED MUTATION
ON THE BASIS OF SEARCH RESULT  S16

RETURN

FIG. 17

- SEQUENCE NAME: "Read Seq 1"
- SEQUENCE: ···GTAAGGCACGTCATA···
- QUALITY SCORE: ···xxxxxyzxxyzzxxx···

FIG. 18

(a)

REFERENCE
SEQUENCE

MAPPING

READ SEQUENCE

(b)

- REFERENCE SEQUENCE INFORMATION: REFERENCE SEQUENCE NAME (REFERENCE SEQUENCE ID),
  SEQUENCE  LENGTH OF REFERENCE SEQUENCE, etc.
- READ SEQUENCE NAME: NAME (READ SEQUENCE ID) OF EACH READ SEQUENCE FOR WHICH ALIGNMENT WAS  PERFORMED
- POSITION INFORMATION: LEFTMOST MAPPING POSITION.  POSITION ON REFERENCE SEQUENCE
  AT WHICH LEFTMOST BASE OF READ SEQUENCE WAS MAPPED
- MAP QUALITY: MAPPING QUALITY CORRESPONDING TO THE READ SEQUENCE
- SEQUENCE: BASE SEQUENCE CORRESPONDING TO EACH READ SEQUENCE (EXAMPLE: ··· GTAAGGCACGTCATA···)

EP 3 702 473 A1

FIG. 19

FIG. 20

| GENE NAME | CHROM POS | MUTATION |
|---|---|---|
| AKT1 | 14q32.33 | p.E17K |
| BRAF | 7q34 | p.V600E, p.V600K, p.V600, p.V600R, p.K601E, p.D594G, p.G469A, p.D594N, p.V600M, p.V600_K601>E, p.G466V, p.V600D, p.L597R, p.G469V, p.N581S, p.V600L, p.G469R, p.G469E, p.G466E, p.L597Q, p.L597S, p.V600A |
| EGFR | 7p11.2 | p.L858R, p.E746_A750del, p.E746_A750delELREA, p.T790M, p.L861Q, p.G719, p.S768I, p.L747_P753>S, p.G719A, p.L747_T751del, p.L747_A750>P, p.G719S, p.L747_T751delLREAT, p.E746_S752>V, c.3633+294delA, p.V769_D770insASV, p.L747_S752del, p.G719C, p.L747_S752delLREATS, p.G598V, p.A289V, c.3633+293_3633+294delAA, p.L747_P753del, p.D770_N771insSVD, p.E746_T751del, p.L747_T751>P, p.E709K, p.E746_T751>A, p.L833V, p.N158N, p.K745_A750del, p.H773_V774insNPH, p.C797S |
| ... | ... | ... |

FIG. 21

PERFORM ALIGNMENT — S12

COMPARE READ SEQUENCE
WITH REFERENCE SEQUENCE — S401

SPECIFY POSITION ON REFERENCE
SEQUENCE AT WHICH DEGREE OF
MATCHING WITH READ SEQUENCE
SATISFIES PREDETERMINED CRITERION — S402

MATCH AT
PLURALITY OF POSITIONS? — S403

YES

NO

ALIGN READ SEQUENCE TO POSITION OF
HIGHEST DEGREE OF MATCHING — S404

ALL READ SEQUENCES
HAVE BEEN ALIGNED? — S405

NO

YES

RETURN

FIG. 22

```
(a)

REFERENCE SEQUENCE:  · · · GCCATGGACAGAAGGCGCAGGGC · · ·
READ SEQUENCE R1:          GCCATGGACAGAA
READ SEQUENCE R2:          GCCATGCACAGAA




(b)

REFERENCE SEQUENCE:  · · · GCCATGGACAGAAGGCGCAGGGC · · ·
READ SEQUENCE R3:          GCCATGGACAG**GGCG
READ SEQUENCE R4:          GCCATGGACAGAAGGCG
                                      ⌒
                                     CGGT
```

FIG. 23

EP 3 702 473 A1

- POSITION INFORMATION: POSITION INFORMATION ON REFERENCE GENOME. FOR EXAMPLE, SPECIFIED BY CHROMOSOME NUMBER AND POSITION ON THE CHROMOSOME.
- REFERENCE BASE: REFERENCE BASE (A, T, C, G, etc.) IN THE POSITION INFORMATION
- MUTATION BASE: BASE OF REFERENCE BASE AFTER MUTATION

| CHROM | POS | REF | ALT | |
|-------|-----|-----|-----|---|
| 20 | 3 | C | G | |
| 21 | 4 | C | CTAG | ← EXAMPLE OF INSERTION MUTATION |
| 19 | 10 | TCG | T | ← EXAMPLE OF DELETION MUTATION |
| 2 | 321681 | G | G]17:198982] | ← REVERSE COMPLEMENT SEQUENCE OF SEQUENCE EXTENDING AT LEFT OF 198982 POSITION OF CHROMOSOME 17 IS BOUND AFTER "G" |
| 2 | 321682 | T | ]13:123456]T | SEQUENCE AT LEFT OF 123456 POSITION OF CHROMOSOME 13 IS BOUND BEFORE "T" |
| 13 | 123456 | C | C[2:321682[ | SEQUENCE AT RIGHT OF 321682 POSITION OF CHROMOSOME 2 IS BOUND AFTER "C" |
| 13 | 123457 | A | [17:198983[A | REVERSE COMPLEMENT SEQUENCE OF SEQUENCE EXTENDING AT RIGHT OF 198983 POSITION OF CHROMOSOME 17 IS BOUND AFTER "C" |

FIG. 24

| MUTATION ID | CHROM | POS | REF | ALT | Annotation | METADATA ABOUT GENE-PANEL-RELATED INFORMATION |
|---|---|---|---|---|---|---|
| #1 | 20 | 3 | C | G | abc | GENE ID: a |
| #2 | 19 | 4 | A | T | xyz | GENE ID: b |
| #3 | xx | yy | C | G | EGFR L858R | GENE ID: c |
| #4 | aa | bb | T | A | BRAF V600E | GENE ID: d |
| . . . | | | | | | |
| #xx | abc | ABC | G | G]p] | ALK-EML4 FUSION | GENE ID: e, GENE ID: f |
| #yy | xyz | XYZ | T | ]p]T | ROS1-CD74 FUSION | GENE ID: g, GENE ID: h |
| . . . | | | | | | |

123

EP 3 702 473 A1

FIG. 25

- MUTATION ID: IDENTIFIER FOR IDENTIFYING MUTATION
- MUTATION POSITION INFORMATION:
  - ➤ CHROM: CHROMOSOME NUMBER
  - ➤ POS: POSITION AT CHROMOSOME HAVING THE CHROMOSOME NUMBER
- REF: WILD TYPE BASE
- ALT: BASE AFTER MUTATION
- ANNOTATION: INFORMATION RELATED TO MUTATION

FIG. 26

(a)

| GENE | ID | CHROM | POS |
|------|------|-------|-----|
| A | aaa | 19 | 10 |
| B | bbb | 20 | 3 |
| C | ccc | 21 | 4 |

(b)

```
Ex)
CHROM      POS        REF        ALT
20         3          C          G
21         4          C          CTAG
19         10         TCG        T
2          321681     G          G]17:198982]
2          321682     T          ]13:123456]T
13         123456     C          C[2:321682[
13         123457     A          [17:198983[A
```

EP 3 702 473 A1

FIG. 27

GENE ANALYSIS APPARATUS 1a

STORAGE UNIT 12a
- GENE-PANEL-RELATED INFORMATION DATABASE 121
- REFERENCE SEQUENCE DATABASE 122
- MUTATION DATABASE 123
- DRUG DATABASE 124
- ANALYSIS RECORD LOG 151

CONTROLLER 11a
- INFORMATION SELECTION UNIT 112
- SEQUENCE DATA READING UNIT 111
- DATA ADJUSTMENT UNIT 113
- MUTATION IDENTIFICATION UNIT 114
- DRUG SEARCH UNIT 117
- REPORT CREATION UNIT 115
- MANAGEMENT UNIT 116

ANALYSIS EXECUTION UNIT 110a
GENE PANEL ID
GENE ID
ANALYSIS RECORD

INPUT UNIT 17
GENE PANEL INFORMATION

SEQUENCER 2
READ SEQUENCE INFORMATION

DISPLAY UNIT 16
OUTPUT UNIT 13
COMMUNICATION UNIT 14

MANAGEMENT SERVER 3

FIG. 28

```
              ┌─────────────────┐
              │      START       │
              └─────────────────┘
                       │
        ┌──────────────────────────────┐
        │ SEARCH DRUG DATABASE ON THE BASIS OF │  S15a
        │ INFORMATION RELATED TO MUTATION      │
        └──────────────────────────────┘
                       │
        ┌──────────────────────────────┐
        │ GENERATE LIST OF DRUG RELATED TO MUTATION │  S16a
        └──────────────────────────────┘
                       │
              ┌─────────────────┐
              │       END        │
              └─────────────────┘
```

FIG. 29

124A

| MUTATION ID | DRUG ID | RELATED DRUG | METADATA ABOUT GENE-PANEL-RELATED INFORMATION |
|---|---|---|---|
| . . . | | | |
| #3 | #a | DRUG A | GENE ID: a |
| #3 | #b | DRUG B | GENE ID: b |
| #4 | #d | DRUG C | GENE ID: c |
| . . . | | | |

FIG. 30

| MUTATION ID | DRUG ID | RELATED DRUG | APPROVAL STATE | APPROVED COUNTRY | TARGET DISEASE ID | METADATA ABOUT GENE-PANEL-RELATED INFORMATION |
|---|---|---|---|---|---|---|
| ▪ ▪ ▪ | | | | | | |
| #3 | #a | DRUG A | APPROVED | USA, JAPAN | #A(LUNG CANCER) | ▪ ▪ ▪ |
| #3 | #b | DRUG B | APPROVED | USA, JAPAN | #A(LUNG CANCER) | ▪ ▪ ▪ |
| ▪ ▪ ▪ | | | | | | |
| #xx | #X | xyz | UNAPPROVED | – | – | |
| #yy | #Y | abc | UNAPPROVED | – | – | |

124B

EP 3 702 473 A1

FIG. 31

```
                    ( START )
                        |
 ┌──────────────────────────────────────┐
 │  SEARCH DRUG DATABASE ON THE BASIS OF │ ⌐ S15b
 │   INFORMATION RELATED TO MUTATION     │
 └──────────────────────────────────────┘
                        |
 ┌──────────────────────────────────────┐
 │  GENERATE LIST INCLUDING INFORMATION  │ ⌐ S16b
 │     RELATED TO APPROVAL OF DRUG       │
 └──────────────────────────────────────┘
                        |
                    (  END  )
```

FIG. 32

START

SEARCH DRUG DATABASE ON THE BASIS OF INFORMATION RELATED TO MUTATION  —S15b

S21
SEARCHED DRUG HAS BEEN APPROVED?

YES

NO

S22
PATIENT DISEASE AND TARGET DISEASE OF APPROVED DRUG MATCH EACH OTHER?

NO

YES

S23
ASSOCIATE THE DRUG AS UNAPPROVED DRUG WITH MUTATION

S24
ASSOCIATE THE DRUG AS APPROVED DRUG WITH MUTATION

S25
ASSOCIATE THE DRUG AS DRUG HAVING POSSIBILITY OF OFF-LABEL USE WITH MUTATION

GENERATE LIST OF DRUG RELATED TO MUTATION  —S16a

END

FIG. 33

124C

| MUTATION ID | DRUG ID | RELATED DRUG | CLINICAL TRIAL/CLINICAL STUDY STATE | COUNTRY | PHASE | TARGET DISEASE | INSTITUTION | METADATA ABOUT GENE-PANEL-RELATED INFORMATION |
|---|---|---|---|---|---|---|---|---|
| ⋯ | | | | | | | | |
| #xx | #X | xyz | IN CLINICAL TRIAL | JAPAN | Phase 3 | DISEASE ID (LUNG CANCER) | abc PHARMACY | ⋯ |
| #yy | #Y | abc | None | — | — | | | ⋯ |

FIG. 34

```
        ┌──────────────────┐
        │      START        │
        └──────────────────┘
                 │
  ┌──────────────────────────────────────┐
  │ SEARCH DRUG DATABASE ON THE BASIS OF  │  ⌐S15c
  │   INFORMATION RELATED TO MUTATION     │
  └──────────────────────────────────────┘
                 │
  ┌──────────────────────────────────────┐
  │ GENERATE LIST INCLUDING INFORMATION   │  ⌐S16c
  │  RELATED TO CLINICAL TRIAL OF DRUG    │
  └──────────────────────────────────────┘
                 │
        ┌──────────────────┐
        │       END         │
        └──────────────────┘
```

FIG. 35

SEQUENCER — 2

READ SEQUENCE INFORMATION

GENE ANALYSIS APPARATUS — 1b

INPUT UNIT — 17

GENE PANEL INFORMATION

CONTROLLER — 11b

STORAGE UNIT — 12b

DISPLAY UNIT — 16

SEQUENCE DATA READING UNIT — 111

INFORMATION SELECTION UNIT — 112

GENE-PANEL-RELATED INFORMATION DATABASE — 121

DATA ADJUSTMENT UNIT — 113

REFERENCE SEQUENCE DATABASE — 122

MUTATION IDENTIFICATION UNIT — 114

MUTATION DATABASE — 123

REFERENCE SEARCH UNIT — 118

REFERENCE DATABASE — 125

OUTPUT UNIT — 13

REPORT CREATION UNIT — 115

ANALYSIS EXECUTION UNIT — 110b

MANAGEMENT SERVER — 3

COMMUNICATION UNIT — 14

MANAGEMENT UNIT — 116

GENE PANEL ID
GENE ID
ANALYSIS RECORD

ANALYSIS RECORD LOG — 151

EP 3 702 473 A1

FIG. 36

| MUTATION ID | CHROM | POS | REF | ALT | BIOLOGICAL BACKGROUND | MOLECULAR FUNCTION INFORMATION | CLINICAL INFORMATION | DOCUMENT |
|---|---|---|---|---|---|---|---|---|
| . . . | | | | | | | | |
| #aa | xx | yy | A | T | xxxxxxxxx | xxxxxxxxx | xxxxxxx | xxxxxx |
| . . . | | | | | | | | |

KRAS-G13D is an activating mutation. KRAS encodes the signaling protein K-Ras, a member of the Ras family; activating KRAS alterations may result in activation of downstream signaling pathways, including the Raf/MEK/ERK pathway (Pylayeva-Gupta et al., 2011: 21993244, Nakano et al., 1984; 6320174). Several MEK inhibitors are under clinical investigation, including the FDA approved therapies trametinib and cobimetinib, and may be relevant for tumors harboring K-Ras activation (Flaherty et al., 2012; 22663011, Larkin et all., 2014;25265494, Britten, 2013;23443307, Janne et al., 2013; 23200175). For colorectal carcinoma patients with metastatic disease and tumors harboring a KRAS or NRAS mutation, the NCCN guidelines (v.1.2016) recommend against the use of cetuximab and panitumumab.

FIG. 37

## GENE ANALYSIS RESULT REPORT

PATIENT ID: █████████

SEX: FEMALE

DISEASE: xxx CANCER, STAGE II

DOCTOR IN CHARGE: ████████

INSTITUTION: ██████

GENE PANEL: PANEL A

REQUEST DAY: MAY 20, 2017

MEASUREMENT DAY: MAY 30, 2017

REPORT DAY: JUNE 19, 2017

### DETECTED GENE MUTATION AND RELATED DRUG

| MARKER | CHANGE | JAPAN | | | USA | |
| --- | --- | --- | --- | --- | --- | --- |
| | | APPROVED | OFF LABEL | CLINICAL TRIAL | FDA APPROVAL | OFF LABEL |
| BRAF | p.V600E | – | DRUG A<br>DRUG B<br>DRUG C | – | – | DRUG A<br>DRUG B<br>DRUG D<br>DRUG E |
| BRCA1 | p.KI183R | – | – | DRUG F | DRUG F<br><br>DRUG G | – |

### CLINICAL STUDY LIST

| MARKER | TEST ID | PHASE | TARGET DISEASE | TEST NAME | DRUG NAME | INSTITUTION |
| --- | --- | --- | --- | --- | --- | --- |
| ALK;<br>ROS1 | Jxxxxxx-xxxxxxx | Phase 1, 2 | xxx CANCER | 1/2 PHASE TEST OF DRUG MX FOR xxx CANCER PATIENT | DRUG MX | COMPANY M |
| ROS1;<br>NTRK | Jxxxxxxx-xxxxxxx | Phase 1 | yyy CANCER | EVALUATION OF SAFETY AND PHARMACOKINETICS OF DRUG NY IN yyy CANCER PATIENT – | DRUG NY | COMPANY N |

FIG. 38

SEQUENCER ~2

READ SEQUENCE INFORMATION

GENE ANALYSIS APPARATUS ~1c

INPUT UNIT ~17

GENE PANEL INFORMATION

CONTROLLER ~11c

FIRST STORAGE UNIT ~12

SEQUENCE DATA READING UNIT ~111

INFORMATION SELECTION UNIT ~112c

GENE-PANEL-RELATED INFORMATION DATABASE ~121c

DISPLAY UNIT ~16

DATA ADJUSTMENT UNIT ~113c

INDEX SEQUENCE INFORMATION

REFERENCE SEQUENCE DATABASE ~122

MUTATION IDENTIFICATION UNIT ~114

OUTPUT UNIT ~13

REPORT CREATION UNIT ~115

MUTATION DATABASE ~123

ANALYSIS EXECUTION UNIT ~110c

GENE PANEL ID
GENE ID
ANALYSIS RECORD

MANAGEMENT SERVER ~3

COMMUNICATION UNIT ~14

MANAGEMENT UNIT ~116

ANALYSIS RECORD LOG ~151

EP 3 702 473 A1

FIG. 39

121D

| GENE PANEL NAME | GENE PANEL ID | INDEX SEQUENCE INFORMATION |
|---|---|---|
| ... | ... | ... |
| PANEL A | AAA | pppppppppp |
| PANEL B | BBB | qqqqqqqqqq |
| ... | ... | ... |

FIG. 40

| ☑ AKT1 | ☑ EGFR | ☐ JAK3 | ... |
|---|---|---|---|
| ☑ APC | ☑ ESR1 | ☑ KIT | ... |
| ☑ ALK | ☐ EML4 | ☑ MET | ... |
| ☑ BRAF | ☐ FBXW7 | ☑ PIK3CA | ... |
| ... | ... | ... | ... |

FIG. 41

○ LUNG CANCER PANEL

● COLON CANCER PANEL

○ BREAST CANCER PANELA

⋮

FIG. 42

RECEIVE INPUT OF
GENE PANEL INFORMATION ⌐S107

OBTAIN GENE PANEL INFORMATION ⌐S201

S202
REGISTERED GENE PANEL? — NO

YES

S203
DESIGNATED GENE PANEL? — NO

YES

⌐S206
DISPLAY MESSAGE
"THIS IS NOT THE DESIGNATED GENE
PANEL. DO YOU USE THIS GENE PANEL?"

⌐S207
INPUT FOR ASKING PERMISSION
ARECEIVED? — NO

YES

⌐S204
DISPLAY PERMISSION OF
USE OF GENE PANEL

⌐S205
DISPLAY THAT
GENE PANEL CANNOT BE USED

RETURN

FIG. 43

FIG. 44

```
                    ┌─────────────┐
                    │    START    │
                    └─────────────┘
                           │
        ┌──────────────────────────────────────┐
        │          PRETREATMENT                 │  ⌐S31
        └──────────────────────────────────────┘
                           │
        ┌──────────────────────────────────────┐
        │          SEQUENCING                   │  ⌐S32
        └──────────────────────────────────────┘
                           │
        ┌──────────────────────────────────────┐
        │   OBTAIN GENE-PANEL-RELATED           │  ⌐S33
        │         INFORMATION                   │
        └──────────────────────────────────────┘
                           │
        ┌──────────────────────────────────────┐
        │     ANALYZE GENE SEQUENCE             │  ⌐S34
        │  ACCORDING TO TYPE OF GENE PANEL      │
        └──────────────────────────────────────┘
                           │
        ┌──────────────────────────────────────┐
        │     QUALITY EVALUATION                │  ⌐S35
        │  ACCORDING TO TYPE OF GENE PANEL      │
        └──────────────────────────────────────┘
                           │
        ┌──────────────────────────────────────┐
        │          CREATE REPORT                │  ⌐S36
        └──────────────────────────────────────┘
                           │
                    ┌─────────────┐
                    │     END     │
                    └─────────────┘
```

FIG. 45

READ REGION: t1 BASE

TARGET GENE
(ENTIRE LENGTH: L BASE)

READ SEQUENCE

POSITION

0

t2 BASE          t3 BASE

100

DEPTH

EP 3 702 473 A1

FIG. 46

GENE ANALYSIS RESULT REPORT

PATIENT ID: ▮▮▮▮▮

SEX: FEMALE

DISEASE: xxx CANCER, STAGE II

DOCTOR IN CHARGE: ▮▮▮▮▮

INSTITUTION: ▮▮▮▮

GENE PANEL: PANEL A

QC INDEX: XXXXXXX

REQUEST DAY: MAY 20, 2017

MEASUREMENT DAY: MAY 30, 2017

REPORT DAY: JUNE 19, 2017

DETECTED GENE MUTATION AND RELATED DRUG

| MARKER | CHANGE | JAPAN | | | USA | |
|--------|--------|----------|-----------|---------------|--------------|-----------|
| | | APPROVED | OFF LABEL | CLINICAL TRIAL | FDA APPROVAL | OFF LABEL |
| BRAF | p.V600E | – | DRUG A<br>DRUG B<br>DRUG C | – | – | DRUG A<br>DRUG B<br>DRUG D<br>DRUG E |
| BRCA1 | p.K1183R | – | – | DRUG F | DRUG F<br><br>DRUG G | – |

CLINICAL STUDY LIST

| MARKER | TEST ID | PHASE | TARGET DISEASE | TEST NAME | DRUG NAME | INSTITUTION |
|--------|---------|-------|----------------|-----------|-----------|-------------|
| ALK;<br>ROS1 | Jxxxxxx-xxxxxxx | Phase 1, 2 | xxx CANCER | 1/2 PHASE TEST OF<br>DRUG MX FOR<br>xxx CANCER PATIENT | DRUG MX | COMPANY M |
| ROS1;<br>NTRK | Jxxxxxxx-xxxxxxx | Phase 1 | yyy CANCER | EVALUATION OF SAFETY<br>AND PHARMACOKINETICS<br>OF DRUG NY IN<br>yyy CANCER PATIENT<br>– | DRUG NY | COMPANY N |

# EP 3 702 473 A1

## INTERNATIONAL SEARCH REPORT

| International application No. |
|---|
| PCT/JP2018/039963 |

### A. CLASSIFICATION OF SUBJECT MATTER

Int.Cl. C12Q1/6869(2018.01)i, C12M1/00(2006.01)i, C12N15/09(2006.01)i, C12Q1/68(2018.01)i, G16B99/00(2019.01)i

According to International Patent Classification (IPC) or to both national classification and IPC

### B. FIELDS SEARCHED

Minimum documentation searched (classification system followed by classification symbols)
Int.Cl. C12Q1/6869, C12M1/00, C12N15/09, C12Q1/68, G16B99/00

Documentation searched other than minimum documentation to the extent that such documents are included in the fields searched

| | |
|---|---|
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)
JSTPlus/JMEDPlus/JST7580 (JDreamIII), CAplus/REGISTRY/MEDLINE/EMBASE/BIOSIS (STN), WPIDS/WPIX (STN)

### C. DOCUMENTS CONSIDERED TO BE RELEVANT

| Category* | Citation of document, with indication, where appropriate, of the relevant passages | Relevant to claim No. |
|---|---|---|
| X/<br>Y | 次世代シークエンサー等を用いた遺伝子パネル検査に基づくがん診療ガイダンス，第1.0版， 日本臨床腫瘍学会・日本癌治療学会・日本癌学会合同, 11 October 2017, i-viii, pp.1-5, non-official translation ("Cancer diagnosis guidance based on gene panel test using next generation sequencer", 1.0 edition, Joint of Japanese Society of Medical Oncology, Japan Society of Clinical Oncology, and Japanese Cancer Association Joint) | 1-5, 7-23, 29-49, 60-63/<br>6-44, 50-59 |
| Y/<br>A | JP 2017-67605 A (KODEN INDUSTRY CO., LTD.) 06 April 2017, paragraph [0028] (Family: none) | 6-44, 50-59/<br>1-5, 45-49, 60-63 |

☒ Further documents are listed in the continuation of Box C.　　☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 15 January 2019 (15.01.2019) | 29 January 2019 (29.01.2019) |

| Name and mailing address of the ISA/<br>Japan Patent Office<br>3-4-3, Kasumigaseki, Chiyoda-ku,<br>Tokyo 100-8915, Japan | Authorized officer |
|---|---|
| | Telephone No. |

Form PCT/ISA/210 (second sheet) (January 2015)

78

**INTERNATIONAL SEARCH REPORT**

International application No.

PCT/JP2018/039963

C (Continuation).   DOCUMENTS CONSIDERED TO BE RELEVANT

| Category* | Citation of document, with indication, where appropriate, of the relevant passages | Relevant to claim No. |
|---|---|---|
| Y/<br>A | JP 2010-60431 A (TOSHIBA CORP.) 18 March 2010, paragraphs [0050]-[0052] & US 2010/0054997 A1, paragraphs [0073]-[0075] | 6-44, 50-59/<br>1-5, 45-49, 60-<br>63 |
| Y/<br>A | JP 2011-40091 A (SYSMEX CORPORATION) 24 February 2011, claim 1, paragraphs [0001], [0012] & US 2002/0026292 A1, paragraphs [0002], [0011]-[0014] | 24-44, 50-59/1-<br>23, 45-49, 60-<br>63 |
| A | WO 2004/109551 A1 (HITACHI HIGH-TECHNOLOGIES CORP.) 16 December 2004, claims 1-25 (Family: none) | 1-63 |
| A | JP 2010-273681 A (MAYO FOUND FOR MEDICAL EDUCATION & RES) 09 December 2010, claims 1-47 & US 2008/0311563 A1, claims 1-22 | 1-63 |
| A | JP 2017-503258 A (AB-BIOTICS S.A.) 26 January 2017, claims 1-28 & US 2016/0314251 A1, claims 1-26 | 1-63 |
| A | JP 2001-243327 A (HITACHI, LTD.) 07 September 2001, claims 1-18 & EP 1111525 A2, claims 1-20 | 1-63 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015200678 PCT